(19) Europäisches Patentamt
European Patent Office
Office européen des brevets

(11) EP 1 945 798 B1

(12) # EUROPÄISCHE PATENTSCHRIFT

(45) Veröffentlichungstag und Bekanntmachung des Hinweises auf die Patenterteilung:
**22.05.2013 Patentblatt 2013/21**

(21) Anmeldenummer: **06818332.6**

(22) Anmeldetag: **31.10.2006**

(51) Int Cl.:
*C12Q 1/37* (2006.01) *C07K 17/00* (2006.01)

(86) Internationale Anmeldenummer:
**PCT/EP2006/010490**

(87) Internationale Veröffentlichungsnummer:
**WO 2007/051605 (10.05.2007 Gazette 2007/19)**

(54) **VERFAHREN ZUR BESTIMMUNG DER SPALTBARKEIT VON SUBSTRATEN**

METHOD FOR DETERMINING THE CLEAVABILITY OF SUBSTRATES

PROCEDE POUR DETERMINER L'APTITUDE AU CLIVAGE DE SUBSTRATS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **31.10.2005 DE 102005051978**

(43) Veröffentlichungstag der Anmeldung:
**23.07.2008 Patentblatt 2008/30**

(73) Patentinhaber: **Forschungszentrum Borstel
23845 Borstel (DE)**

(72) Erfinder:
- **GORRIS, Hans-Heiner
47551 Bedburg-Hau (DE)**
- **BADE, Steffen
23795 Klein Rönnau (DE)**
- **RÖCKENDORF, Niels
23863 Bargfeld-Stegen (DE)**
- **FREY, Andreas
23816 Groß Niendorf (DE)**

(74) Vertreter: **UEXKÜLL & STOLBERG
Patentanwälte
Beselerstrasse 4
22607 Hamburg (DE)**

(56) Entgegenhaltungen:
**WO-A-02/06306 WO-A-98/39471
WO-A-2005/007119 WO-A-2007/051603
WO-A-2007/051604 DE-A1- 19 745 668
DE-C1- 19 529 250**

- **MANETTA J V ET AL: "DESIGN AND IMPLEMENTATION OF A PARTICLE CONCENTRATION FLUORESCENCE METHOD FOR THE DETECTION OF HIV-1 PROTEASE INHIBITORS" ANALYTICAL BIOCHEMISTRY, ACADEMIC PRESS, SAN DIEGO, CA, US, Bd. 202, Nr. 1, 1992, Seiten 10-15, XP008062872 ISSN: 0003-2697**
- **CRAIG D B ET AL: "General protease assay method coupling solid-phase substrate extraction and capillary electrophoresis." ANALYTICAL CHEMISTRY 15 SEP 1998, Bd. 70, Nr. 18, 15. September 1998 (1998-09-15), Seiten 3824-3827, XP002464498 ISSN: 0003-2700**
- **BAECHLE D ET AL: "Biotinylated fluorescent peptide substrates for the sensitive and specific determination of cathepsin D activity." JOURNAL OF PEPTIDE SCIENCE : AN OFFICIAL PUBLICATION OF THE EUROPEAN PEPTIDE SOCIETY MAR 2005, Bd. 11, Nr. 3, März 2005 (2005-03), Seiten 166-174, XP002464499 ISSN: 1075-2617**
- **GORRIS HANS H ET AL: "Rapid profiling of peptide stability in proteolytic environments.", ANALYTICAL CHEMISTRY 15 FEB 2009 LNKD-PUBMED:19159331, vol. 81, no. 4, 15 February 2009 (2009-02-15), pages 1580-1586, ISSN: 1520-6882**
- **KOZLOV IGOR A ET AL: "A high-complexity, multiplexed solution-phase assay for profiling protease activity on microarrays.", COMBINATORIAL CHEMISTRY & HIGH THROUGHPUT SCREENING JAN 2008 LNKD-PUBMED:18220541, vol. 11, no. 1, January 2008 (2008-01), pages 24-35, ISSN: 1386-2073**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- Hans-Heiner Gorris: "Entwicklung eines Hochdurchsatz-Proteolysetests und Analyse der Proteolyse resistenz von Peptidepitopen; Inauguraldissertation.", 30 November 2005 (2005-11-30), Leibniz-Zentrum für Medizin und Biowissenschaften, Universität Lübeck., Lübeck, Deutschland.
- OAKELEY E J ET AL: "CHANGING FUNCTIONALITY OF SURFACES BY DIRECTED SELF-ASSEMBLY USING OLIGONUCLEOTIDES-THE OLIGO-TAG", BIOTECHNIQUES, INFORMA LIFE SCIENCES PUBLISHING, WESTBOROUGH, MA, US, vol. 27, no. 4, 1 October 1999 (1999-10-01), pages 752,754-756,758,7, XP001147133, ISSN: 0736-6205
- MATUSZCZYK GEORG ET AL: "Development of an ELISA for 2,4-D: Characterization of two polyclonal antisera", FRESENIUS JOURNAL OF ANALYTICAL CHEMISTRY, SPRINGER, BERLIN, DE, vol. 354, no. 1, 1 January 1996 (1996-01-01), pages 41-47, XP002463225, ISSN: 0937-0633, DOI: DOI:10.1007/S002169600007
- SKLADAL PETR ET AL: "Characterization of monoclonal antibodies to 2,4-dichlorophenoxyacetic acid using a piezoelectric quartz crystal microbalance in solution", JOURNAL OF IMMUNOLOGICAL METHODS, ELSEVIER SCIENCE PUBLISHERS B.V., AMSTERDAM, NL, vol. 176, no. 1, 1 January 1994 (1994-01-01), pages 117-125, XP002463226, ISSN: 0022-1759, DOI: DOI:10.1016/0022-1759(94) 90356-5
- JORACEK JIRI ET AL: "Improved direct piezoelectric biosensors operating in liquid solution for the competitive label-free immunoassay of 2,4-dichlorophenoxyacetic acid", ANALYTICA CHIMICA ACTA, ELSEVIER, AMSTERDAM, NL, vol. 347, no. 1-2, 1 January 1997 (1997-01-01), pages 43-50, XP002463227, ISSN: 0003-2670, DOI: DOI:10.1016/S0003-2670 (97)00125-6
- HATZIDAKIS G I ET AL: "USE OF L-LYSINE FLUORESCENCE DERIVATIVES AS TRACERS TO ENHANCE THE PERFORMANCE OF POLARIZATION FLUOROIMMUNOASSAYS. A STUDY USING TWO HERBICIDES AS MODEL ANTIGENS", ANALYTICAL CHEMISTRY, AMERICAN CHEMICAL SOCIETY, US, vol. 74, no. 11, 1 June 2002 (2002-06-01), pages 2513-2521, XP001132316, ISSN: 0003-2700, DOI: DOI: 10.1021/AC011051X
- DZGOEV A B ET AL: "HIGH-SENSITIVITY ASSAY FOR PESTICIDE USING A PEROXIDASE AS CHEMILUMINESCENT LABEL", ANALYTICAL CHEMISTRY, AMERICAN CHEMICAL SOCIETY, US, vol. 71, no. 22, 15 November 1999 (1999-11-15), pages 5258-5261, XP000926909, ISSN: 0003-2700, DOI: DOI:10.1021/AC990417R
- FRANEK MILAN ET AL: "Monoclonal ELISA for 2,4-dichlorophenoxyacetic acid: Characterization of antibodies and assay optimization", JOURNAL OF AGRICULTURAL AND FOOD CHEMISTRY, AMERICAN CHEMICAL SOCIETY, US, vol. 42, no. 6, 1 January 1994 (1994-01-01), pages 1369-1374, XP002463253, ISSN: 0021-8561, DOI: DOI:10.1021/JF00042A024
- "HANDBOOK OF IMMUNOLOGY 4TH EDITION : IMMUNOCHEMISTRY", HANDBOOK OF IMMUNOLOGY, XX, XX, 1 January 1986 (1986-01-01), pages 3.01-3.13, XP002913917,


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft Verfahren und die dazu erforderlichen Verbindungen zur Untersuchung der enzymatischen Spaltbarkeit von Substraten. wobei eine Eignung für Hochdurchsatzuntersuchungen von Peptidsubstratbibliotheken gegeben ist.

Enzymkatalysierte Abbauvorgänge spielen in allen Organismen eine zentrale Rolle. Enzyme ermöglichen und beschleunigen eine Vielzahl von biologischen Reaktionen und Stoffwechselprozessen. Diejenigen Stoffe, deren Umsetzung von den Enzymen katalysiert wird, werden allgemein als Substrate bezeichnet. Enzyme binden ihre Substrate hochspezifisch und ausschließlich an ihrem katalytischen Zentrum, entweder nach dem "Schlüssel-Schloß-" oder nach dem "Induced-Fit-Prinzip". Das bedeutet, daß ein bestimmtes Enzym nur eine eng begrenzte Anzahl von Substanzen als Substrat akzeptiert, welche den sterischen Anforderungen einer Bindung im katalytischen Zentrum entsprechen (Substratspezifität). Von jedem Enzym werden nur ganz bestimmte, definierte Reaktionen katalysiert (Wirkungsspezifität), wobei die Geschwindigkeit der Umsetzung von der sogenannten katalytischen Effizienz abhängt ($k_{cat}/K_M$). Die Wirkung von Enzymen kann von sogenannten Inhibitoren verringert oder aufgehoben werden.

Proteasen oder Peptidasen sind Enzyme, die den hydrolytischen Abbau von Proteinen oder8.5 Peptiden katalysieren. Sie werden nach ihrem katalytischen Mechanismus in verschiedene Klassen eingeteilt, von denen die Serinproteasen am besten untersucht sind. Zu ihnen zählen beispielsweise die Verdauungsenzyme des Dünndarms Trypsin, Chymotrypsin und Elastase. Ein Unterscheidungsmerkmal zwischen den Proteasen betrifft die Spaltungsstelle des Substrats. Exopeptidasen spalten Proteine oder Proteinbruchstücke an den freien Enden, indem sie fortschreitend Aminosäuren von den Enden freisetzen. Bei Endopeptidasen hingegen findet die Spaltung innerhalb der Peptidkette statt. Dadurch werden neue Spaltungsmöglichkeiten für die Exopeptidasen geschaffen. Aufgrund der zentralen Rolle, die Proteine in einem Lebewesen spielen, kommt auch den Proteasen eine große Bedeutung zu. Eine Proteaseunter- oder - überproduktion sowie Störungen bei der Kontrolle ihrer enzymatischen Wirkung können schwerwiegende Erkrankungen auslösen. Bei Unterproduktion gleicht man den Mangel durch Proteasesupplementierung aus, bei Enzymüberfunktion kommen Proteaseinhibitoren zum Einsatz. Die Verwendung von Proteaseinhibitoren bietet sich auch für die Therapie von Infektionskrankheiten an, bei denen Proteasen des Erregers zur Infektion und seiner Vermehrung im Wirt erforderlich sind. Zeigt der Erreger, wie z.B. das HI-Virus (AIDS) oder *Plasmodium falciparum* (Malaria), jedoch eine starke genetische Drift, bilden sich rasch Resistenzen gegen bestimmte Inhibitoren aus, und es müssen neue Wirkstoffe entwickelt werden.

Die Aktivität von Proteasen muß des weiteren bei der Entwicklung von Medikamenten auf Peptid- oder Proteinbasis berücksichtigt werden. Dies gilt insbesondere dann, wenn sie zur oralen Verabreichung vorgesehen sind. In diesem Fall müssen Wirkstoffe so gestaltet sein, daß sie von Proteasen des Verdauungstrakts nicht angegriffen werden. Sowohl für die Entwicklung von peptidischen Wirkstoffen zur oralen Verabreichung als auch für die Entwicklung von Proteaseinhibitoren sind geeignete Testsysteme erforderlich, mit denen die Stabilität - von Peptiden gegenüber Proteasen in Ab- bzw. Anwesenheit von Proteaseinhibitoren geprüft werden kann.

Es sind verschiedene Ansätze zur Untersuchung der Spaltbarkeit von Substraten bzw. von Enzymspezifitäten bekannt.

Obwohl für die Bindung eines Substrates in das katalytische Zentrum der Protease und für den mechanistischen Ablauf Protease-katalysierter Reaktionen seit langem Computersimulationen und theoretische Modelle existieren, mangelt es an geeigneten Verfahren zur praktischen Untersuchung der Protease-Substrat-Interaktion. Es wurden verschiedene Ansätze vorgeschlagen.

Größere Substrate und ganze Proteine wurden mit einer Proteasemischung in Kontakt gebracht und die Spaltungsfragmente anschließend elektrophoretisch oder chromatographisch getrennt. Nachteil dieser Verfahren ist jedoch, daß genaue Kenntnisse über die zu erwartenden Fragmente erforderlich sind, wenn der Nachweis mit Hilfe von Antikörpern erfolgen soll. Mit der Reverse Phase High Performance Liquid Chromatography (RP-HPLC) in Kombination mit massenspektrometrischen Verfahren konnte eine genaue Trennung und Identifizierung der Fragmente erfolgen (Coombs et al., 1996). Für die Messung von Massenspektren muß aber für jede einzelne Bestimmung einer Enzymkinetik ein Massenspektrometer verfügbar sein, was bei einfachen Laborausstattungen nicht gegeben ist.

Immunoverfahren wurden in verschiedenen Formaten für Enzymaktivitätsmessungen verwendet. Haber et al. (1969) entwickelten einen in abgewandelter Form bis heute in der klinischen Diagnostik verwendeten kompetitiven Radioimmunoassay (RIA) zur Bestimmung der Blutplasmaprotease Renin, eines Markers für bestimmte Formen des Bluthochdrucks. Dabei wird der Blutplasmaprobe eine definierte Menge des Reninsubstrates Angiotensinogen zugesetzt und das gebildete Angiotensin als Kompetitor für radioaktiv markiertes Angiotensin im Radioimmunoassay bestimmt. Durch Vergleich mit einer Renineichlösung läßt sich die absolute Plasmareninkonzentration ermitteln.

Bei neueren Verfahren wurde die radioaktive Markierung im allgemeinen durch ein enzymatisches Verstärkersystem ersetzt, welches ein Farbsubstrat umsetzt, das photometrisch vermessen wird.

Zur Bestimmung der HIV-1 Proteaseaktivität wurden biotinmarkierte Peptidsubstrate über Streptavidin an eine Mikrotitrationsplatte gebunden. Nach Beendigung der Spaltung durch die HIV-1 Protease wurden nur die ungeschnittenen, aber nicht die gespaltenen Peptide über einen Antikörper und einen enzymgekoppelten zweiten Antikörper nachgewiesen (O. Gutiérrez et al., 2002).

Solche Verfahren, bei denen ein Enzym in Lösung auf ein festphasengebundenes Substrat wirkt und die zum Beispiel in der deutschen Patentanmeldung DE 101 187 74 offenbart sind, werden als heterogene Enzymassays (HEA) bezeichnet. Diese einfachen und robusten Verfahren, mit denen auch nicht gereinigte Enzymfraktionen getestet werden können, haben allerdings den Nachteil, daß keine freie Zugänglichkeit des Enzyms zum festphasengebundenen Substrat gewährleistet ist. Rückschlüsse auf die Enzymkinetik sind nur mit Einschränkung möglich. Des weiteren können nur spezifische enzymatische Aktivitäten untersucht werden, bei denen die Schnittstelle genau definiert ist. Bei Enzymen mit breitem Substratspektrum, wie beispielsweise den Verdauungsenzymen des Dünndarms, kann nicht ausgeschlossen werden, daß das Protein zur Bindung des Substrats an die Festphase von der enzymatischen Aktivität abgebaut wird. Infolgedessen wären auch die ungeschnittenen Peptide nicht mehr nachweisbar. Schließlich muß für jedes zu untersuchende Substrat entweder ein neuer Nachweisantikörper hergestellt werden, oder die Erkennungssequenz für den Antikörper muß an das jeweilige Substrat angehängt werden.

Verfahren zur Untersuchung der Enzymkinetik erfordern eine exakte Erfassung des Substratumsatzes. Ein kritischer Punkt für die Untersuchung der enzymatischen Vorgänge liegt in der Verfügbarkeit von geeigneten Substraten. Nur wenn die Abnahme der Substratkonzentration oder die Zunahme der Produktkonzentration direkt verfolgt werden kann, sind Rückschlüsse auf die Enzymkinetik möglich. Versuchstechnisch ist es im Falle der Hydrolyse von Proteinen und Peptiden schwierig, Substrat und Produkt zu unterscheiden und Konzentrationsänderungen zwischen diesen beiden Molekülen festzustellen.

Bei indirekten Verfahren, wie z.B. der bio- oder immunchemischen Fragmentanalyse, dem homogenen Immunoproteasetest, dem heterogenen Immunoproteasetest sowie dem Proteasetest durch Enzymfragment-Komplementierung, wird die Position der Spaltung und das Ausmaß der Interaktion zwischen Protease und Substrat erst durch nachgeschaltete Maßnahmen erfaßbar.

Bei der bio- oder immunchemischen Fragmentanalyse werden die Spaltprodukte einer Proteolysereaktion chromatographisch isoliert oder elektrophoretisch separiert und danach einzeln charakterisiert, z.B. durch Sequenzierung, Massenspektrometrie oder Immunoblotting (z.B. Coombs et al., 1996). Das Verfahren kommt bei der Proteinsequenzierung zum Einsatz und bietet sich an, wenn die Wirkung einer gereinigten Protease auf ein Reinsubstrat untersucht werden soll oder wenn Antikörper gegen eine putative Proteaseschnittstelle des Substrates existieren. Spaltungskinetiken lassen sich nur in Sonderfällen und mit großem Aufwand durchführen, da für jeden Meßpunkt eine Produktreinigung oder -trennung durchgeführt werden muß und oft Fragmente ähnlicher Größe enstehen, die sich einer kompletten Trennung oder Reinigung entziehen. Darüber hinaus können komplexe Intermediatgemische entstehen, wenn das Substrat mehrere Schnittstellen für die Protease trägt. In solchen Fällen ist auch der immunologische Nachweis sehr aufwendig, da für jede Schnittstelle ein separater Antikörper zur Verfügung stehen muß. Diese Trennprobleme potenzieren sich, wenn die Wirkung von Proteasemischungen oder komplexen Protease-haltigen biologischen Proben auf ein Substrat bestimmt werden soll, da die Proteasen sich hier auch selbst angreifen oder irrelevante, in der Probe befindliche Substrate ebenfalls spalten. Hier kann das Produkt u. U. von Tausenden irrelevanter Fragmente ähnlicher Größe verdeckt werden.

Beim homogenen Immunoproteasetest bestimmt man die proteolytische Zerstörung oder Bildung eines definierten Substrates bzw. Produktes mit Hilfe eines Immunoassays, indem das Substrat zunächst in Lösung der Protease ausgesetzt und dann das im Reaktionsgemisch befindliche Produkt oder Substrat entweder direkt oder als Kompetitor in einem Immunoassay bestimmt wird (z.B. RIA oder ELISA). Das Substrat bzw. Produkt muß dazu nicht gereinigt werden, und es können rohe Enzympräparationen eingesetzt werden. Zur Durchführung des der Proteolyse nachgeschalteten RIA oder ELISA muß allerdings immer ein Produkt- oder Substrat-spezifischer Antikörper existieren. D.h. ein Immunoproteasetest ist immer spezifsch auf ein Substrat-Enzym-Paar ausgelegt. Haber et al. (1969) entwickelten z.B. einen in abgewandelter Form bis heute in der klinischen Diagnostik verwendeten kompetitiven Radioimmunoassay (RIA) zur Bestimmung der Blutplasmaprotease Renin, eines Markers für bestimmte Formen des Bluthochdrucks. Dabei wird der Blutplasmaprobe eine definierte Menge des Reninsubstrates Angiotensinogen zugesetzt und das gebildete Angiotensin als Kompetitor für radioaktiv markiertes Angiotensin im Radioimmunoassay bestimmt. Durch Vergleich mit einer Renineichlösung läßt sich die absolute Plasmareninkonzentration ermitteln.

Beim heterogenen Immunoproteasetest wird das Substrat bereits immobilisiert vorgelegt und das nach der Reaktion verbliebene Substrat durch einen Substrat-spezifischen Antikörper erfaßt. Dieses einfache und robuste Verfahren, mit dem auch nicht gereinigte Enzymfraktionen getestet werden können, hat allerdings den Nachteil, daß keine freie Zugänglichkeit des Enzyms zum festphasengebundenen Substrat gewährleistet ist. Rückschlüsse auf die Enzymkinetik sind nur mit Einschränkung möglich. Des weiteren können nur spezifische enzymatische Aktivitäten untersucht werden, bei denen die Schnittstelle genau definiert ist. Bei Enzymen mit breitem Substratspektrum, wie beispielsweise den Verdauungsenzymen des Dünndarms, kann nicht ausgeschlossen werden, daß auch die Verankerung des Substrats an die Festphase von der enzymatischen Aktivität abgebaut wird. Infolgedessen wären auch die ungeschnittenen Peptide nicht mehr nachweisbar. Schließlich muß für jedes zu untersuchende Substrat entweder ein neuer Nachweisantikörper

hergestellt werden, oder die Erkennungssequenz für den Antikörper muß an das jeweilige Substrat angehängt werden. D.h. dieses Testformat ist wie der homologe Immunoproteasetest auf ein Substrat-Enzym-Paar ausgelegt.

Ein Beispiel für dieses Testformat wurde in der deutschen Patentanmeldung DE 101 187 74 offenbart. Ein anderes wurde von Gutiérrez et al. (2002) zur Analyse der HIV-1 Proteaseaktivität beschrieben. Hier wurden biotinmarkierte Peptidsubstrate über Streptavidin an eine Mikrotitrationsplatte gebunden. Nach Beendigung der Spaltung durch die HIV-1 Protease wurden nur die ungeschnittenen, aber nicht die gespaltenen Peptide über einen Antikörper und einen enzymgekoppelten zweiten Antikörper nachgewiesen.

Direkte Verfahren, wie z.B. der chromogene Enzymtest oder Enzymtests auf Basis intramolekularer Fluoreszenzlöschung, erlauben die Analyse der Proteasereaktion in Echtzeit und sind daher für kinetische Untersuchungen gut geeignet.

Der chromogene Enzymtest liefert allerdings keine Aussagen über die Spaltbarkeit von Aminosäuresequenzmotiven sondern dient der Bestimmung von Enzymaktivitäten und Mengen, da sich in P1'-Position einer putative Schnittstelle immer eine chromogene Gruppe und keine Aminosäure befindet. Verfahren und Verbindungen zur Durchführung solcher chromogenen Enzymtests sind dem Fachmann seit langem bekannt (z.B. Bender et al., 1966).

Bei Enzymtests auf Basis intramolekularer Fluoreszenzlöschung umgeht man diesen Nachteil chromogener Enzymtests, da sich die zur Detektion erforderlichen Markierungen am proximalen und distalen Ende eines linearen synthetischen Peptidsubstrates befinden. Die von den Markierungen umschlossene Proteaseerkennungssequenz ist frei wählbar und darf somit eine komplette natürlich vorkommende Substratsequenz darstellen. Dieses Testformat basiert auf der sog. FRET-Technologie (Fluorescence Resonance Electron Transfer). FRET ist eine entfernungsabhängige Interaktion zwischen den elektronenangeregten Zuständen von zwei Farbstoffmolekülen, bei denen Energie von einem Donor- auf ein Akzeptormolekül übertragen wird, ohne daß Licht emittiert wird. Die Anwesenheit des Akzeptors löscht die Emission des Donors. Die ersten Anwendungen von FRET-basierten Enzymsubstraten gehen auf die 1970iger Jahre zurück (Latt et al., 1972, und Yaron et al., 1979). Die Farbstoffmoleküle sind an den beiden Enden der linearen Peptidkette befestigt. Solange das Peptid ungespalten ist, entsteht kein Fluoreszenzsignal des Donors. Bei der Spaltung des Peptides werden Donor und Akzeptor räumlich voneinander getrennt, das Löschen wird aufgehoben und der Donor emittiert ein Lichtsignal, das photometrisch bestimmt wird. Dieses Verfahren ermöglicht eine weitgehend freie Wahl der Spaltungsstelle mit den flankierenden Sequenzen, solange die Farbmoleküle die Spaltungsstelle einschließen.

Allerdings weist auch das FRET-Verfahren verschiedene Nachteile auf. So ist die Effektivität der strahlungslosen Energieübertragungen stark von der Entfernung von Donor und Akzeptor abhängig. Sie sinkt mit der sechsten Potenz des intramolekularen Abstands, so daß bislang nur Peptidsubstrate von maximal 11 Aminosäuren verwendet wurden (Wang et al., 1993). Als weiteres Problem kommt die schlechte Wasserlöslichkeit der meisten Fluoreszenzfarbstoffe und der Akzeptoren hinzu, so daß - abhängig von der Peptidsequenz - ein organisches Lösemittel wie Dimethylsulfoxid zum Assaypuffer zugesetzt werden muß. Dieses kann einen Einfluß auf die Enzymaktivität haben.

Des weiteren kann unter bestimmten Bedingungen die Anregung des Donors oder die Emission des Akzeptors gestört oder überlagert werden. Rohe Proteasepräparationen enthalten oft biogene Farbstoffe oder Fluorophore und auch die Substratsequenzen selbst können fluoreszierende Aminosäuren, wie z.B. Tryptophan enthalten. Letzteres ist gerade für Hochdurchsatzuntersuchungen an Peptidsubstratbibliotheken ein Problem, da der Ausschluß bestimmter Kandidaten a priori eine unerwünschte Wichtung in das Verfahren einführt. Um das zu umgehen, müssten solche Substratmoleküle gesondert untersucht werden, was einen erheblichen Mehraufwand nach sich zieht (J. George et al. 2003). WO 2005/007119 A nutzt FRET-Technologie zur Untersuchung der Aktivität einer Protease.

Die kombinatorische Chemie erlaubt es, eine Vielzahl unterschiedlicher Peptidsubstrate parallel zu synthetisieren. Ein gängiges Verfahren zur Herstellung solcher Bibliotheken ist die Spotsynthesetechnik (Frank, 1992). Die Peptidsubstrate sind auf einer Seite an einer Oberfläche gebunden, auf der anderen Seite können sie mit einer Markierung versehen werden, über die sich die Spaltung überprüfen läßt. Als Markierung dient ein radioaktives Isotop, ein Fluoreszenzfarbstoff oder eine andere signalgebende Gruppe.

Verfahren, die kombinatorische immobilisierte Peptidbibliotheken in Verbindung mit FRET einsetzen, wurden z.B. in DE 19840545 A1 offenbart. Für den hohen Durchsatz an verschiedenen Substraten werden allerdings die Nachteile von immobilisierten Peptiden - die schlechte Zugänglichkeit von membrangebundenen, dicht gepackten Substraten, die Verunreinigungen in Form von unvollständigen Syntheseprodukten, die den ohnehin schon hohen Hintergrund von FRET-Systemen weiter erhöhen, und die große Proteasemenge, die benötigt wird, um einen weitgehend vollständigen Substratabbau zu gewährleisten, - in Kauf genommen, ohne die Vorteile des FRET-Verfahrens - die genaue Analyse der Enzymkinetik - nutzen zu können.

Ein neuerer Ansatz, der im Gegensatz zu FRET-basierten Techniken für längere Substrate entwickelt wurde, bediente sich der sogenannten Enzymfragment-Komplementierung (Naqvi et al., 2004). Dazu wurde ein zyklisches Peptid synthetisiert, das einerseits eine Schnittstelle für das zu untersuchende Enzym enthält und andererseits ein Peptidfragment zur Komplementierung eines ansonsten inaktiven signalgebenden Enzyms. In der zyklischen Form ist die Komplementierung nicht möglich. Erst nach Spaltung der Schnittstelle entsteht das zur Komplementierung geeignete Fragment. Dabei muß allerdings gewährleistet sein, daß ein zyklisches Peptid ebenso gut gespalten wird wie ein lineares

und das komplementierende Fragment nicht angegriffen wird. Diese Einschränkungen sowie die relativ aufwendige Herstellung der zyklischen Peptide mit den entsprechenden Aufreinigungsschritten läßt es fraglich erscheinen, ob dieses Verfahren eine breite Verwendung finden kann.

Manetta et al. (1992), Anal. Biochem. 202, 10-15, offenbart ein Verfahren zur Identifikation von Peptiden, die auf die enzymatische Aktivität einer bestimmten Protease inhibitorisch wirken, wobei das Substratkonstrukt aus der N-terminal biotinylierten Peptidsequenz besteht, die nach der Festphasensynthese abgespalten und in Lösung am C-terminalen Lys mit FITC markiert wird. Craig et al. (1998), Anal. Chem. 70. 3824-3827, offenbart ein Verfahren zur Durchführung eines Proteaseassays, bei dem ein N-terminal biotinyliertes Peptid mit einer bekannten Proteaseschnittstelle, das eine C-terminale Fluoreszenzmarkierung enthält, verwendet wird. WO 1998/39471 A offenbart ein Verfahren zur Identifikation von Proteolysehemmenden Substanzen, wobei die peptidischen Substrate jeweils Liganden eines Ligandenbindungspaares am N- und C-Terminus aufweisen. Baechle et al. (2005), J. Pept. Sci. 11, 166-174, beschreibt ein Verfahren, bei dem selektiv die Aktivität von Cathepsin D in Gegenwart von Modulatoren mit Hilfe einers doppelt markierten Paptids gemessen werden kann, wobei das Substrat mit zwei chemisch detektierbaren Gruppen markiert ist, einem N-terminalen Fluorophorund einem C-terminalen Biotin. WO 2001/06306 A betrifft Verfahren zur Optimierung von Identifizierung von Modulatoren der Aktivität einer Protease, bei denen Peptidsubstrate zur Analyse der Proteaseaktivität optimiert werden.

Vor diesem Hintergrund liegt die Aufgabe der vorliegenden Erfindung darin, Verbindungen und Verfahren bereitzustellen, mit denen sich sowohl die Spaltbarkeit von Substraten als auch Enzymspezifitäten bestimmen lassen, wobei die im Stand der Technik vorhandenen Nachteile überwunden werden, und wobei eine Eignung für Hochdurchsatzuntersuchungen der Spaltbarkeit an Peptidsubstratbibliotheken gegeben ist.

Erfindungsgemäß wird diese Aufgabe insbesondere durch den Gegenstand der angefügten Ansprüche gelöst.

Die Erfindung betrifft insbesondere Verfahren zur Untersuchung der enzymatischen Spaltbarkeit von Substraten, welche für Hochdurchsatzuntersuchungen der Spaltbarkeit an Peptidsubstratbibliotheken geeignet sind, die dadurch gekennzeichnet sind, daß man

a) Verbindungen bereitstellt, die

- auf einer ersten Festphase synthetisiert werden;
- eine Komponente 1 aufweisen, die der ersten Festphase zugewandt ist und quantifiziert werden kann;
- einen auf enzymatische Spaltbarkeit zu analysierenden Abschnitt aufweisen, der aus der Gruppe umfassend Peptide und Polypeptide ausgewählt ist;
- eine Komponente 2 aufweisen, die von der ersten Festphase abgewandt ist und direkt oder über einen Bindungspartner an eine zweite Festphase binden kann;
- wobei zwischen dem zu spaltenden Abschnitt und den beiden Komponenten chemische Strukturen mit einer oder mehreren negativen Ladungen eingefügt sind, welche aus einer oder mehreren Aminosäuren bestehen, die Phosphat- oder Sulfatgruppen enthalten, oder aus der Gruppe der Aminodicarbonsäuren oder Aminopolycarbonsäuren ausgewählt sind:
- zwischen dem zu spaltenden Abschnitt und den chemischen Strukturen mit einer oder mehreren negativen Ladungen jeweils Abstandshalter eingefügt sind, wobei Polyethylenglycolsubstrukturen verwendet werden, wobei die Polyethylenglycolstruktur aus der Gruppe bestehend aus Aminopolyethylenglycoldiglycolsäure mit zwei Ethylenglycoleinheiten (PEG-2, MW 530.6) und Aminopolyethylenglycoldiglycolsäure mit neun Ethylenglycoleinheiten (PEG-9, MW 839.0) ausgewählt ist:

b) die Verbindungen nach Abspaltung von der ersten Festphase in Lösung mit einem Enzym oder Enzymgemisch in Kontakt bringt;
c) die gespaltenen und ungespaltenen Verbindungen anschließend an eine zweite Festphase bindet, die identisch mit der ersten Festphase sein kann, wobei die Bindung über Komponente 2 erfolgt, die direkt an die zweite Festphase oder an einen auf der zweiten Festphase aufgebrachten Bindungspartner bindet;
d) die nicht-immobilisierten Bestandteile von der zweiten Festphase abtrennt;
e) die Menge der ungespaltenen Verbindungen nachweist, indem man Komponente 1 quantifiziert; und
f) die Spaltbarkeit bestimmt, indem man die Menge der ungespaltenen Verbindungen vor und nach der Spaltungsreaktion vergleicht.

Die Verbindungen weisen einen polymeren, auf enzymatische Spaltbarkeit zu analysierenden Abschnitt auf, der auch als "zu analysierender Molekülbereich" bzw. "Substrat" bezeichnet wird. Der polymere Abschnitt kann aus der Gruppe, umfassend Peptide; <u>und</u> Polypeptide, ausgewählt werden. Bevorzugt handelt es sich bei dem zu spaltenden Abschnitt um Peptide.

Im Rahmen dieser Erfindung wird unter dem Begriff Peptid eine unverzweigte polymere Verbindung verstanden,

die aus einer Verknüpfung mehrerer Aminosäuren hervorgegangen ist. Eine Aminosäure ist jede Art von organischer Verbindung, die mindestens eine Amin- und eine Säurefunktion enthält. Der Begriff umfaßt somit nicht nur natürliche Aminosäuren wie sie in Lebewesen vorkommen, sondern auch jede andere synthetische Verbindung mit diesen Eigenschaften.

Unter dem Begriff Substrat (S) wird im Rahmen dieser Anmeldung der auf enzymatische Spaltbarkeit zu analysierende Abschnitt verstanden.

Die erfindungsgemäßen Verbindungen umfassen somit im wesentlichen ein Substrat und 2 Komponenten.

Die Erfindung betrifft des weiteren Verfahren, bei denen Komponente 2 quantifizierbar ist und Komponente 1 direkt oder über einen Bindungspartner an eine zweite Festphase binden kann, wobei die Bindung in Schritt c) über Komponente 1 erfolgt und man in Schritt e) die ungespaltenen Verbindungen nachweist, indem man Komponente 2 quantifiziert.

In einer weiteren Ausführungsform der Erfindung sind die erste und zweite Festphase identisch.

Unter Festphase wird jedes Material verstanden, das sich dazu eignet, die zu untersuchenden Verbindungen direkt oder indirekt zu binden. Auf Seiten der anorganischen Materialien eignen sich hierfür beispielsweise Keramik, Silikate, Glas, Silizium und Metalle. Von den organischen Substanzen können zum Beispiel Polysaccharide, wie Zellulose, oder Polyolefine, wie Polystyrol, Polypropylen oder halogenierte Polyolefine (PVC, PVDF usw.) verwendet werden. In einer bevorzugten Ausführungsform wird für die erste Festphase Zellulose und für die zweite Festphase Polystyrol verwendet. In einer weiteren bevorzugten Ausführungsform wird als zweite Festphase eine lichtdurchlässige Mikrotitrationsplatte aus Polystyrol verwendet.

Die auf der Festphase bereitgestellten Verbindungen umfassen zwei voneinander verschiedene Komponenten 1 und 2, wobei Komponente 1 der ersten Festphase zugewandt und Komponente 2 von der ersten Festphase abgewandt ist. Als Komponenten werden bevorzugt kleine Moleküle gewählt. Mindestens eine dieser Komponenten muß sich dazu eignen, die Verbindung an die zweite Festphase zu immobilisieren (insbes. binden). Diese Immobilisierung kann direkt an die Festphase erfolgen. Sie kann aber auch indirekt über einen Bindungspartner, beispielsweise einen Antikörper, der auf der Festphase aufgebracht ist, stattfinden. In der Regel erfolgt die spezifische Immobilisierung über ein Bindungspaar. Vorzugsweise ist der erste Bindungspartner ein im Vergleich zum zweiten Bindungspartner kleiner Ligand, der als eine der beiden Komponenten Bestandteil der Verbindung ist, und der zweite Bindungspartner ein Protein, das an der Festphase immobilisiert ist und den ersten Bindungspartner bindet. Die kovalente und nicht-kovalente Immobilisierung von Proteinen an die Oberfläche von Festphasen ist durch verschiedene bekannte Verfahren möglich. Eine kovalente Immobilisierung der Proteine kann beispielsweise durch die Bereitstellung von Carboxylatfunktionen als reaktive Gruppen auf der Festphase erfolgen. Die Erzeugung von Carboxylatgruppen auf Polyolefinoberflächen kann zum Beispiel durch Plasmaoxidation oder Oxidation mit Chromsäure, Permanganat oder Cersulfat erfolgen. Mit Carbodiimiden, wie zum Beispiel N-(3-Dimethylaminopropyl)-N'-ethylcarbodiimid EDC, aktivierte Carboxylatfunkionen bilden eine kovalente Bindung zu Proteinen aus. Üblicherweise erfolgt die Immobilisierung von Proteinen an die Oberfläche von Mikrotitrationsplatten aus Polystyrol über nicht-kovalente Adsorption.

Mindestens eine der Komponenten muß sich zur Quantifizierung der Verbindungen eignen, d.h., sie muß geeignet sein, die Menge an Verbindungen, die an die zweite Festphase gebunden ist, bestimmen zu können. In dem Fall, daß nur eine Komponente zur Quantifizierung geeignet ist, muß es die Komponente sein, die nicht zur Bindung an die zweite Festphase verwendet wird. Die zur Quantifizierung geeignete Komponente kann selbst signalerzeugend oder aber Bindungspartner für eine signalerzeugende Gruppe sein. Als signalerzeugende Gruppe kommen beispielsweise Verbindungen in Betracht, die zur Chemilumineszenz oder Fluoreszenz befähigt sind. In einer bevorzugten Ausführungsform wird ein hochaffines Bindungspaar verwendet, wobei der erste Bindungspartner ein im Vergleich zum zweiten Bindungspartner kleiner Ligand ist, der als eine der beiden Komponenten Bestandteil der Verbindung ist, und der zweite Bindungspartner ein Protein ist, das den ersten Bindungspartner hochaffin bindet und eine signalgebende Gruppe trägt. Besonders bevorzugt wird als erster Bindungspartner Biotin und als zweiter Bindungspartner Streptavidin gewählt, das mit einer signalgebenden Gruppe konjugiert ist. Als signalgebende Gruppe werden bevorzugt Enzyme verwendet, die luminogene, fluorogene oder chromogene Substrate umsetzen können. Besonders bevorzugt als signalgebende Gruppe wird Meerrettichperoxidase für die katalytische Umwandlung von farblosem Tetramethylbenzidin in die oxidierte farbige Form verwendet.

In einer bevorzugten Ausführungsform werden die Verbindungen direkt auf der ersten Festphase synthetisiert, wobei viele verschiedene Verbindungen mit unterschiedlichen zu spaltenden Abschnitten parallel synthetisiert werden können, d.h. auf verschiedenen Abschnitten derselben Festphase. Die Synthese der Substanzbibliothek von peptidischen Analyten erfolgt mit Peptidsyntheseverfahren, die dem Fachmann bekannt sind. In einer bevorzugten Ausführungsform wird das FMOC-Syntheseverfahren für Zellulosefilter-immobilisierte Peptidbibliotheken verwendet (Frank, 1992). Der Einsatz kombinatorischer Synthesetechniken ermöglicht, gleichzeitig eine Vielzahl von Analyten ortsabhängig parallel aus Aminosäuren herzustellen. Im Gegensatz zum FRET-Verfahren gibt es keine versuchstechnische Begrenzung der Länge des Analyten. Die Länge ist allein von der Effizienz des gewählten Syntheseverfahrens abhängig. Mit der SPOT-Synthese lassen sich Analyten mit einem frei wählbaren Sequenzmotiv von mindestens 16 Aminosäuren

herstellen.

In einer bevorzugten Ausführungsform wird ein unter definierten Bedingungen abspaltbarer Anker auf die Zellulosemembran aufgebracht, auf den die gewünschten Verbindungen synthetisiert werden. Dies hat den Vorteil, daß unerwünschte, carboxyterminal verkürzte Kettenneustartprodukte beim Abspalten der Verbindungen auf der Membran verbleiben. Der erfindungsgemäß verwendete Anker kann aus den Aminosäurebausteinen Prolin und Lysin bestehen (Bray et al., 1990).

Im Anschluß an die Synthese des Ankers wird dann Komponente 1 eingebaut, die die Verbindung an einem Ende begrenzt. Nach der sequentiellen Synthese des zu spaltenden Abschnitts wird an dessen anderem Ende Komponente 2 eingebaut. Dabei werden die Komponenten bevorzugt so gewählt, daß sie eine möglichst geringe sterische Hinderung beim Kontakt des zu spaltenden Abschnitts mit einem Enzym bewirken und daß sie dem zu spaltenden Abschnitt eine bessere Löslichkeit in wäßrigen Puffern verschaffen.

Um eine bessere Löslichkeit des zu spaltenden Abschnitts und eine bessere Zugänglichkeit für das Nachweissystem zu erreichen, werden im Lauf der Synthese zwischen dem zu analysierenden Molekülbereich und den beiden Komponenten Abstandhalter eingefügt, vorzugsweise ein Abstandhalter, der zu einem Abstand der beiden detektierbaren Gruppen voneinander von mehr als 100 Å führt. Es werden wasserlösliche Abstandhalter wie Polyethylenglycol- (PEG) verwendet. Unter Polyethylenglykol-Substrukturen werden folgende Strukturen verstanden: verzweigte oder unverzweigte, an einem oder mehreren Enden substituierte Ethylenglycol-Homopolymere oder Propylenglycol-Homopolymere, sowie gemischte Ethylenglycol-/Propylenglycol-Copolymere mit durchschnittlichen Molekulargewichten zwischen 100 und 5000 g/mol. Unter Polyol-Substrukturen werden lineare oder verzweigte Polyole, die 3 bis 15 Hydroxylgruppen enthalten können, verstanden. Die erfindungsgemäß verwendeten Polyethylenglycolsubstrukturen sind Amino-Polyethylenglycol(PEG)-diglycolsäure mit ie 2 Ethylenglycoleinheiten (PEG-2, MW 530,6) und Amino-Polyethylenglycol (PEG)-diglycolsäure mit je 9 Ethylenglycoleinheiten (PEG-9), MW 839.0).

Es werden im Laufe der Synthese zwischen dem zu spaltenden Abschnitt und den beiden Komponenten jeweils chemische Strukturen mit einer oder mehreren negativen Ladungen eingefügt. Dadurch kann eine unspezifische Bindung der Verbindungen an die Festphasen weitgehend verhindert werden. Unspezifische Bindung der Verbindungen ist eine nicht über die Komponente 1 oder 2 vermittelte Adhäsion an die Festphase. Ladungsträger im Sinne der vorliegenden Erfindung sind Aminosäuren, wie sie im Rahmen dieser Erfindung definiert sind, die mindestens eine negative Ladung tragen, welche von Phosphat-, Sulfat- oder Carboxylatgruppen bereitgestellt werden kann. Geeignete Aminosäuren sind beispielsweise Asparaginsäure, Glutaminsäure, Aminoadipinsäure, Carboxyasparaginsäure, Carboxyglutaminsäure, Carboxymethylcystein, Phosphoserin, Phosphothreonin, Phosphotyrosin, Phosphonomethylphenylalanin, Sulfoserin, Sulfothreonin und Sulfotyrosin, jeweils in ihrer L- oder D-Konfiguration. Besonders bevorzugt werden auf beiden Seiten jeweils zwei D-Glutaminsäurebausteine verwendet, da alpha-Peptidbindungen von D-Aminosäuren durch Proteasen nicht gespalten werden.

Um einen Einfluß der negativen Ladungen auf die Spaltungsreaktion zu vermeiden, werden zwischen dem zu spaltenden Abschnitt und den negativen Ladungen auf beiden Seiten Abstandhalter eingefügt. In der besonders bevorzugten Form werden Amino-Polyethylenglycol(PEG)-diglycolsäurebausteine mit je 2 Ethylenglycoleinheiten als Abstandhalter verwendet.

Alle genannten Bausteine, die nicht Teil des zu analysierenden Abschnitts sind, haben die Eigenschaft, daß sie beim Inkontaktbringen der gelösten Verbindung mit einer Substanz oder einem Substanzgemisch, deren Einfluß auf die Stabilität des zu analysierenden Molekülbereichs festgestellt werden sollen, nicht gespalten werden.

In einer Ausführungsform der Erfindung werden die Verbindungen nach Abschluß der Synthese von der ersten Festphase abgespalten, herausgewaschen und im Vakuum getrocknet. Danach werden sie in einem geeigneten inerten Lösungsmittel aufgenommen, wobei bevorzugt ein wäßriger, tensidhaltiger Puffer verwendet wird.

Die Verbindungen werden nach Abspaltung von der ersten Festphase mit einem Enzym oder Enzymgemisch in Kontakt gebracht, um die Auswirkungen des Enzyms, bzw. Enzymgemischs auf den zu analysierenden Molekülbereich zu testen. In einer bevorzugten Ausführungsform wird das Enzym, bzw. Enzymgemisch aus der Gruppe der Proteasen ausgewählt. Als Enzymgemische können gereinigte Enzyme, aber auch biologische Proben, wie z.B. Rohextrakte verschiedenster Ausgangsmaterialien verwendet werden. Mögliche Ausgangsmaterialien sind Intestinalflüssigkeiten, Stuhl, Blut, Urin, Speichel, Sputum, Lymphflüssigkeiten, andere Körperflüssigkeiten, Zellysate, Gewebelysate und Organlysate.

Die Spaltungsreaktion kann auf verschiedene Arten terminiert werden. Zur Bestimmung von Enzymkinetiken erfolgt die Terminierung nach einem definierten Zeitintervall. In einer bevorzugten Ausführungsform wird die Spaltungsreaktion durch Zugabe eines Inhibitors beendet. In einer weiteren bevorzugten Ausführungsform erfolgt die Terminierung der Spaltungsreaktion durch die Zerstörung des Enzyms, bzw. der im Enzymgemisch enthaltenen Enzyme. Dies kann beispielsweise durch Erhitzen erfolgen. Das Ausschalten der enzymatischen Aktivität kann auch durch Änderung der Reaktionsbedingungen, wie beispielsweise die Änderung der Protonenkonzentration und/oder den Entzug von Co-Faktoren der Enzyme erfolgen. Durch all diese Terminierungsmethoden soll die Verbindung nicht gespalten werden.

Die Bindung der gespaltenen und ungespaltenen Verbindungen an die zweite Festphase kann über die Komponente 1 erfolgen. In einer bevorzugten Ausführungsform erfolgt sie über die Komponente 2. Dies hat den Vorteil eines

zusätzlichen Reinigungsschritts. Es können nur solche ungespaltenen Analyten und Spaltprodukte gebunden werden, bei denen auf der vom Syntheseanker abgewandten Seite Komponente 2 vollständig vorhanden ist. Analyten, bei denen die Synthese nicht vollständig verlaufen ist, sowie deren Spaltprodukten fehlt auf der vom Syntheseanker abgewandten Seite die Komponente 2, so daß sie infolgedessen nicht an die Festphase gebunden und nachgewiesen werden. Das ist von Vorteil für die Sensitivität des Verfahrens.

Die für die Bindung an die Festphase verwendete Komponente kann Partner eines hochaffinen Bindungspaares sein. Ein Ligand bildet zusammen mit einem Rezeptor ein solches Bindepaar, wobei der Ligand meistens ein niedermolekulares Molekül ist, an dessen stereochemische Eigenschaften der Rezeptor angepaßt ist. Bei einem Zusammentreffen von Ligand und Rezeptor kommt es aufgrund der stereochemischen Eigenschaften von Ligand und Rezeptor zu einer spezifischen Bindung. In der Gruppe der Rezeptoren bilden Antikörper eine Untergruppe. Ihre Liganden werden ais Antigene bezeichnet. Kleine Antigene, die alleine nicht in der Lage sind, die Bildung von Antikörpern zu bewirken, werden als Haptene bezeichnet. In einer bevorzugten Ausführungsform wird die Komponente ausgewählt aus der Gruppe der Liganden oder Haptene. Als zweiter Partner des Bindungspaares wird ein gegen diese Komponente gerichteter festphasengebundener Rezeptor beziehungsweise Antikörper verwendet. In einer besonders bevorzugten Ausführungsform wird das Hapten 2,4-Dichlorphenoxyessigsäure (2,4-D) als Komponente verwendet. In diesem Fall erfolgt die Bindung an die zweite Festphase in einer bevorzugten Ausführungsform über einen monoklonalen anti-2,4-D Antikörper (Franek, 1994), mit dem die zweite Festphase beschichtet ist. Bevorzugt wird in diesem Fall eine Mikrotitrationsplatte aus Polystyrol als Festphase verwendet.

In einer besonders bevorzugten Ausführungsform wird direkt an der 2,4-D-Carboxylatfunktion eine verzweigte oder unverzweigte aliphatische Kette gebunden, wodurch die Affinität der Bindung zwischen 2,4-D und Antikörper um ein Mehrfaches erhöht wird. In der bevorzugten Form werden unverzweigte Alkan-Reste mit einer $(CH_2)_n$-Kettenlänge von $n \geq 6$ verwendet, wobei die Kettenlänge vorzugsweise maximal n = 11 beträgt. Der Alkan-Rest (n = 11) wird durch die Verwendung von Aminoundecansäure bereitgestellt. In einer anderen bevorzugten Ausführungsform (n=6) wird der Alkan-Rest durch Aminohexansäure bereitgestellt.

Die Abtrennung der nicht-immobilisierten Bestandteile von der zweiten Festphase erfolgt in einer bevorzugten Ausführungsform durch einen Waschschritt. Dabei kann als Waschlösung beispielsweise ein wäßriger tensidhaltiger Puffer wie D-PBS mit Tween20 eingesetzt werden.

Die Menge der ungespaltenen Verbindung wird bestimmt, indem man die Komponente, die nach der Immobilisierung an die zweite Festphase auf der von dieser abgewandten Seite liegt, quantifiziert. In einer Ausführungsform ist dies die Komponente 2 und in einer bevorzugten Ausführungsform die Komponente 1. Wie bereits beschrieben, kann die zur Quantifizierung geeignete Komponente selbst signalerzeugend oder aber Bindungspartner für eine signalerzeugende Gruppe sein. In einer bevorzugten Ausführungsform wird die für die Quantifizierung eingesetzte Komponente ausgewählt aus der Gruppe der Liganden oder Haptene. Als zweiter Partner des Bindungspaares wird ein gegen diese Komponente gerichteter Rezeptor beziehungsweise Antikörper verwendet, der mit einer signalgebenden Gruppe gekoppelt ist. Bevorzugt wird der Ligand Biotin als Komponente und eine an den Rezeptor Streptavidin gekoppelte signalgebende Gruppe verwendet.

Der Antikörper, mit dem die Festphase beschichtet ist, bindet das Hapten, unabhängig davon, wie das Gesamtmolekül, von dem das Hapten einen Teil bildet, aufgebaut ist. D. h. unabhängig davon ob es sich um eine gespaltene oder um eine nicht gespaltene Verbindung handelt. Nach der Spaltungsreaktion liegt in der Lösung eine Mischung aus gespaltener und nicht gespaltener Verbindung vor, die beide mit gleicher Affinität vom Antikörper gebunden werden. Aufgrund des fehlenden Biotins der gespaltenen Verbindungen reduzieren die gespaltenen Verbindungen das maximale Signal, das dann erhalten wird, wenn ausschließlich die ungespaltene Verbindung zur Bindung durch den Antikörper zur Verfügung steht. Da der Antikörper ungespaltene und gespaltene Verbindungen mit gleicher Affinität bindet, sinkt das maximale Signal proportional zum Anteil der gespaltenen Verbindung. Das maximale Signal wird bestimmt, indem die Verbindung, die nicht mit Enzym in Kontakt gebracht wurde und demzufolge ungespalten ist, in einem getrennten Ansatz ebenfalls auf die beschichtete Festphase gegeben wird. Auf diese Weise ist der prozentuale Anteil der gespaltenen Verbindung bestimmbar: 100 * (1 - Signal nach Enzymspaltung / Signal ohne Enzymspaltung). Wenn beispielsweise das Signal nach einer Spaltungsreaktion 50 % des maximalen Signals erreicht, so wurde die halbe Menge der eingesetzten Verbindung gespalten. Die Stärke des maximalen Signals wird durch die Menge des Antikörpers auf der Festphase festgelegt.

Die Erfindung betrifft außerdem Verfahren zur Bestimmung der Enzymkinetik, die dadurch gekennzeichnet sind, daß man die erfindungsgemäßen Verfahren zur Untersuchung der enzymatischen Spaltbarkeit wiederholt durchführt, wobei das Inkontaktbringen mit dem Enzym bzw. Enzymgemisch für verschiedene Zeitintervalle oder mit verschiedenen Enzymkonzentrationen erfolgt, und man die Halbwertszeit der Substrate bestimmt.

Zur Bestimmung von Enzymkinetiken wird in einer Ausführungsform der Erfindung die Substratkonzentration [S] im allgemeinen viel höher gewählt als die Konzentration des Enzyms [E], so daß bei der Auswertung die Michaelis-Menten-Kinetik angewendet werden kann. In einer möglichen Ausführungsform können mit reinen Enzymlösungen enzymatische Messungen unter den Bedingungen einer nullten Ordnung ($[S] > K_M$) durchgeführt werden, die eine

genaue Analyse des enzymatischen Katalysemechanismus erlauben. In dieser Ausführungsform können für eine bestimmte Enzymmenge die Michaelis-Menten Konstante ($K_M$) = die Substratkonzentration, bei der die halbmaximale Enzymgeschwindigkeit erreicht wird - und die maximale Enzymgeschwindigkeit ($V_{max}$) bestimmt werden. Je nach Enzym und Substrat sind exakt definierte Substratkonzentrationen von bis zu 1 mM erforderlich. Wenn dies die Kapazitätsgrenze von parallelen Peptidsyntheseverfahren wie der SPOT-Synthese überschreitet, kann die Synthese der erfindungsgemäßen Verbindungen auch nach klassischen Verfahren, wie z.B. der Synthese auf Harzträgern, die dem Fachmann bekannt ist, erfolgen. Im Gegensatz zu FRET-Verfahren sind Interferenzen der Substratmoleküle untereinander ausgeschlossen, was eine nach oben hin offene Wahl der Substratkonzentration ermöglicht.

In der bevorzugten Ausführungsform der enzymatischen Spaltung werden Bedingungen pseudo-erster Ordnung ($[E] < [S] < K_M$) gewählt. In einer möglichen Variation dieser Ausführungsform werden mehrere gleiche Mischungen von Enzym und Verbindung bzw. Substrat für unterschiedlich lange Zeiten miteinander in Kontakt gebracht. Das unterschiedlich lange Inkontaktbringen von Substrat und Enzym erlaubt eine Auswertung der Enzymkinetik analog zu einer kontinuierlichen Messung des Substratumsatzes durch chromogene und FRET-Substrate. Ein weiterer Vorteil ist die Tatsache, daß für die Auswertung keine exakte Quantifizierung der eingesetzten Substratmenge erforderlich ist. Der Nachweis des gespaltenen bzw. ungespaltenen Substrates kann bei niedrigeren Konzentrationen erfolgen als bei FRET-Verfahren, da hier ein erheblich niedrigeres Hintergrundsignal aus dem Substrat und der eingesetzten Proteasepräparation beobachtet wird.

Es ist allgemein bekannt, daß verschiedene Substrate in ihrem Abbauverhalten stark variieren können. Da eine Kinetik pseudo-erster Ordnung exponentiell vom Produkt aus Meßzeit und Enzymkonzentration abhängt, können bei gegebener Enzymkonzentration extrem kurze oder sehr lange Meßzeiten erforderlich werden, um die ganze Variationsbreite des Abbauverhaltens abzudecken. Da die Enzymaktivität nicht beliebig lange konstant bleibt und einer beliebigen Verringerung der Meßzeit technische Grenzen gesetzt sind, läßt sich die gesamte Bandbreite der Substratstabilität als Funktion der Zeit bei bestimmten Substraten nicht komplett erfassen. In einer besonders bevorzugten Ausführungsform wird daher unter den Bedingungen pseudo-erster Ordnung bei gegebener Meßzeit die Enzymkonzentration logarithmisch variiert und das Abbauverhalten als Funktion der Enzymkonzentration bestimmt. Die jeweiligen Reaktionsgeschwindigkeiten lassen sich dann mittels Normierung auf eine definierte Enzymkonzentration ermitteln.

In einer möglichen Variante dieser Ausführungsformen unter pseudo-erster Ordnung können Substanzen identifiziert werden, die einen Einfluß auf die Spaltungsreaktion haben. Dazu werden die erfindungsgemäßen Verfahren wie beschrieben durchgeführt, wobei eine oder mehrere zusätzliche Messungen durchgeführt werden, die sich von der ersten durch den Zusatz der zu testenden Substanz zum Reaktionsgemisch unterscheiden. In einer bevorzugten Ausführungsform werden die Substanzen ausgewählt aus der Gruppe der Inhibitoren, bestehend aus kompetitiven, tight-binding und nicht-kompetitiven Inhibitoren. Besonders bevorzugt werden kompetitive Inhibitoren, wie beispielsweise der Serinproteaseinhibitor Aprotinin untersucht. Durch Vergleich der Effizienz der Spaltungsreaktion mit und ohne Inhibitor kann dessen Wirksamkeit ermittelt werden.

Die Erfindung betrifft des weiteren Verbindungen, die sich zum Einsatz in den erfindungsgemäßen Verfahren eignen. Solche Verbindungen sind somit geeignet, die enzymatische Spaltbarkeit von Substraten zu untersuchen und die Enzymspezifität zu analysieren.

Die erfindungsgemäßen Verbindungen haben die allgemeine Struktur

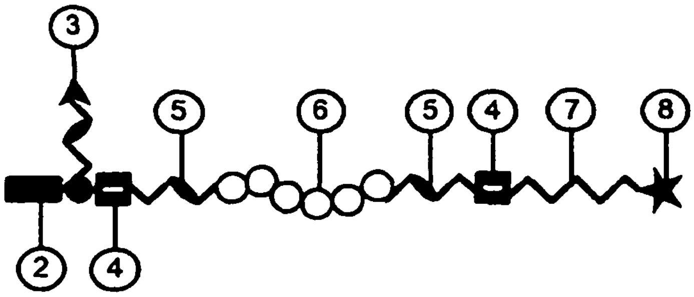


[anders ausgedrückt: (2)-(3)-(4)-(5)-(6)-(5)-(4)-(7)-(8)], die eine die Bausteine (2) und (3) und die Bausteine (4) und (5) umfassende Komponente A aufweist, die über Baustein (2) an eine erste Festphase gebunden und über Baustein (3) detektiert und/oder quantifiziert werden kann und gegebenenfalls an eine zweite Festphase gebunden werden kann, wobei das von der Festphase wegweisende Ende der Komponente A über Baustein (3) oder, wenn die Komponente A den Baustein (5) umfaßt, über Baustein (5) mit einem auf enzymatische Spaltbarkeit zu analysierenden Abschnitt (6) verknüpft ist, der aus der Gruppe umfassend Peptide und Polypeptide ausgewählt ist, und der an seinem der Komponenten A gegenüberliegenden Ende mit einer Komponente B verknüpft ist, die den Baustein (8) und optional die Bausteine (4), (5) und/oder (7) umfaßt, wobei die Komponente B entweder direkt oder über Baustein (7) oder, wenn die Komponente

B den Baustein (5) umfaßt, über Baustein (5) mit dem auf enzymatische Spaltbarkeit zu analysierenden Abschnitt (6) verknüpft ist und über Baustein (8) detektiert und/oder quantifiziert und gegebenenfalls an eine zweite Festphase gebunden werden kann, wobei
der Baustein (2) ein Syntheseanker ist,
der Baustein (3) eine detektierbare Gruppe ist,
der Baustein (4) eine chemische Struktur mit einer oder mehreren negativen Ladungen ist, welche aus einer oder mehreren Aminosäuren besteht, die Phosphat- oder Sulfatgruppen enthalten, oder aus der Gruppe der Aminodicarbonsäuren oder Aminopolycarbonsäuren ausgewählt sind,
der Baustein (5) ein Abstandhalter ist, der jeweils eine Polyethylenglycolstruktur mit einer Molmasse von 100 bis 5000 g/mol umfasst, wobei die Polyethylenglycolstruktur aus der Gruppe bestehend aus Aminopolyethylenglycoldiglycolsäure mit zwei Ethylenglycoleinheiten (PEG-2. MW 530,6) und Aminopolyethylenglycoldiglycolsäure mit neun Ethylenglycoleinheiten (PEG-9, MW 839.0) ausgewählt ist,
der Baustein (7) ein Abstandhalter ist,
der Baustein (8) eine detektierbare Gruppe ist,
wobei die Bausteine (4) und (5) der Komponenten A und B untereinander jeweils gleich oder verschieden sein können.

Der Baustein (2) ist vorzugsweise ein spaltbarer Anker, über den die Verbindung an die Festphase gebunden ist und durch dessen selektive Spaltung sie von der Festphase gelöst werden kann. Insbesondere ist der Baustein (2) aus epsilon-Lysyl-Prolin (Lys-Pro), p-[Amino-(2,4-dimethoxybenzyl)]-phenoxyacetyl (Rink-Linker), p-Benzyloxybenzylalkohol (Wang-Linker) oder 4-Hydroxymethylphenoxyacetyl (HMP) ausgewählt. Gemäß einer Ausführungsform ist der Baustein (2) epsilon-Lysyl-Prolin (Lys-Pro), wobei die Verbindung über den Prolin-Rest an die Festphase gebunden vorliegt und gespalten werden kann. Wenn es sich bei der Festphase um Zellulose handelt, kann Baustein (2) vorzugsweise eine Aminosäure sein. Die Spaltung der resultierenden Esterbindung kann dann durch eine Behandlung mit Ammoniak erfolgen.

Der Baustein (3) ist vorzugsweise aus der Gruppe der biotinylierten Aminosäuren ausgewählt. Bei der biotinylierten Aminosäure handelt es sich insbesondere um Biocytin oder N-gamma-(N-biotinyl-3-(2-(2-(3-aminopropyloxy)-ethoxy)-ethoxy)-propyl)-L-Glutamat.

Baustein (4) besteht aus einer oder mehreren Aminosäuren, die Phosphat- oder Sulfatgruppen enthalten, oder die aus der Gruppe der Aminodicarbonsäuren oder Aminopolycarbonsäuren ausgewählt sind. Bei den Aminodicarbonsäuren handelt es sich z.B. um Asparaginsäure, Glutaminsäure, Aminoadipinsäure oder Carboxymethylcystein, bei den Polycarbonsäuren z.B. um Carboxyasparaginsäure oder Carboxylutaminsäure, bei den Phosphatgruppen enthaltenden Aminosäuren z.B. um Phosphoserin, Phosphothreonin, Phosphotyrosin oder Phosphonomethylphenylalanin, bei den Sulfatgruppen enthaltenden Aminosäuren z.B. um Sulfoserin, Sulfothreonin oder Sulfotyrosin, jeweils in ihrer L- oder D-Konfiguration. Bevorzugt enthält der Baustein (4) zwei D-Glutaminsäuren.

Der Baustein (7) ist vorzugsweise aus der Gruppe der aliphatischen Aminocarbonsäuren ausgewählt, wobei Aminoundecansäure oder Aminohexansäure bevorzugt sind.

Der Baustein (8) ist vorzugsweise aus der Gruppe bestehend aus 2,4-Dichlorphenoxyessigsäure und Dinitrophenylverbindungen wie z. B. 2,4-Dinitrophenylglycin, 2,4-Dinitrophenylaminobuttersäure, 2,4-Dinitrophenylaminocapronsäure oder 2,4-Dinitrophenylaminoundecansäure ausgewählt.

Gemäß einer besonderen Ausführungsform der Erfindung sind die Bausteine (7) und (8) in einem Baustein zusammengefaßt, der ein 2,4-Dichlophenoxyessigsäurederivat der allgemeinen Formel (I), die in den gleichzeitig, d.h. am selben Tag, eingereichten internationalen Anmeldungen "Neue 2,4-Dichlorphenoxyessigsäurederivate und deren Verwendung in diagnostischen und analytischen Nachweisverfahren" (WO 2007/051604) und "Kit für hoch-sensitive Nachweis-Assays" (WO 2007/051603) (Anmelder: jeweils Forschungszentrum Borstel et al.) sowie in den entsprechenden deutschen Prioritätsanmeldungen (DE 10 2005 051 977.6 und DE 10 2005 051 976.8) offenbart sind und auf die hiermit ausdrücklich Bezug genommen wird, umfasst.

Bei diesen Verbindungen handelt es sich um Verbindungen der Formel (I)

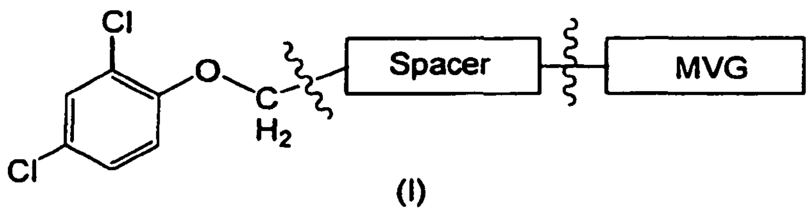

(I)

,

wobei der Spacer einen Abstandshalter und MVG eine markierungsvermittelnde Gruppe darstellt.

Diese Verbindungen sind in Wasser stabil und löslich, wobei der Spacer 1 bis 25 gleiche oder verschiedene geschützte oder ungeschützte Aminosäuren oder Nukleotide umfaßt. Der Spacer kann auch aus einer linearen oder

verzweigten Kette aus 1 bis 10 Monosacchariden bestehen, zum Beispiel aus Glucose, Mannose, Galactose, Ribose, Arabinose, N-Acetylglucosamin oder Fructose oder aus 1 bis 5 Disaccharidbausteinen wie Cellobiose, Lactose, Chitobiose, Lactosamin aufgebaut sein, die vorzugsweise aber nicht zwangsläufig beta-1,4-glycosidisch verknüpft sind oder aus Kombinationen oder Derivaten der genannten Strukturen bestehen. Au-βerdem kann der Spacer aus O-glycosylierten Serin-, Threonin- oder N-glycosylierten Asparagin- oder Glutaminsäure-Untereinheiten aufgebaut sein oder diese enthalten. Der Spacer kann darüber hinaus aus linearen oder verzweigten Polyolen mit 3 bis 15 Hydroxylgruppen aufgebaut sein, die ganz oder teilweise mit den oben genannten Spacer-Komponenten, wie z.B. Saccharid- oder Polyolstrukturen, verknüpft sein können.

Der Spacer kann außerdem aus der folgenden Gruppe ausgewählte Reste umfassen:

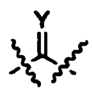
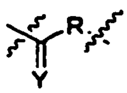
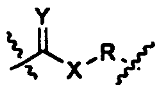
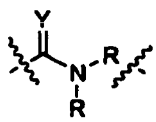

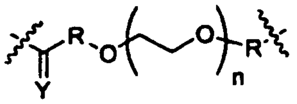
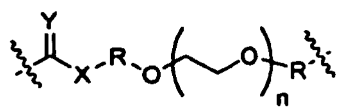
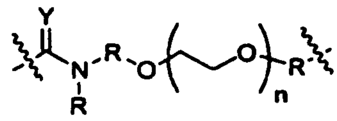

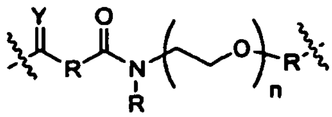
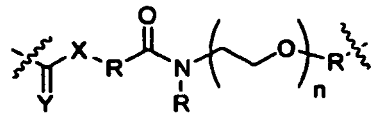
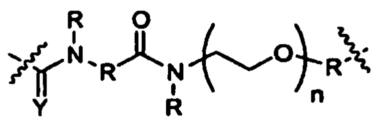

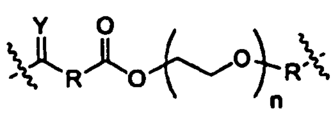
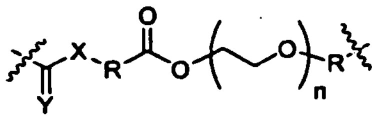
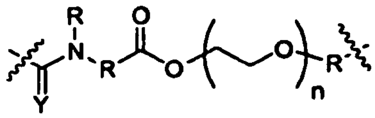

wobei X und Y unabhängig voneinander -0- oder -S- sind und n eine ganze Zahl im Bereich zwischen 1 und 15 ist, wobei MVG aus der folgenden Gruppe ausgewählt ist:

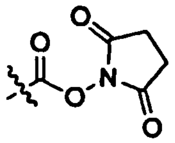
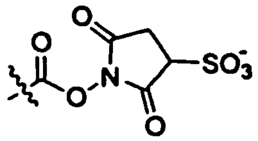
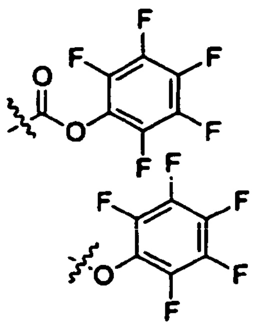
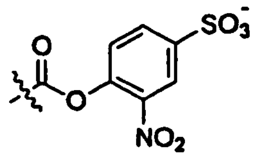

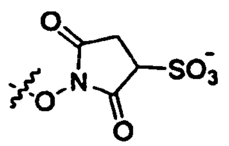
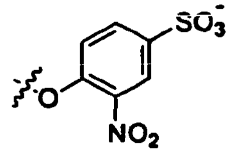
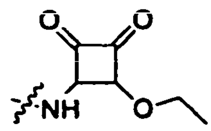

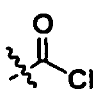
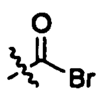
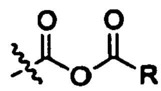
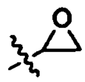
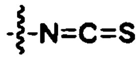
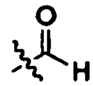
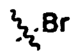

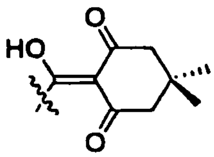
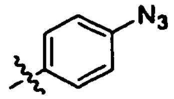
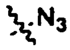
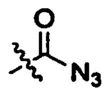
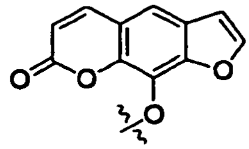

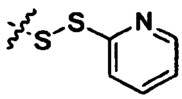
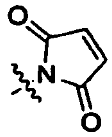
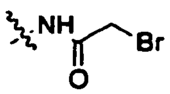
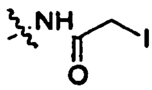
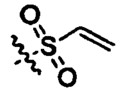

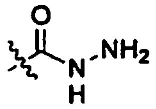
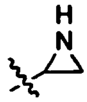
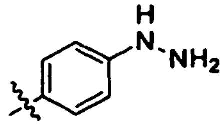
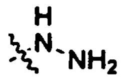

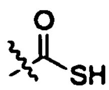
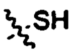
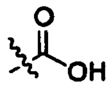
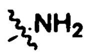

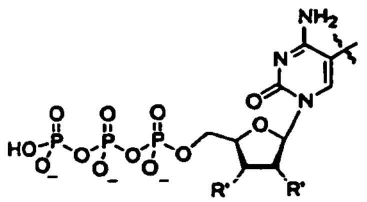
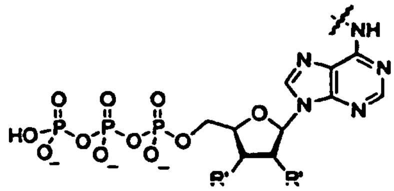

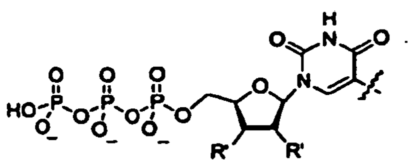
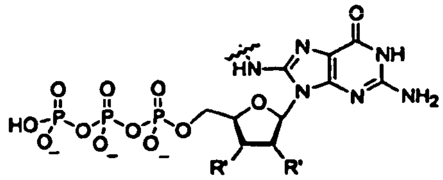

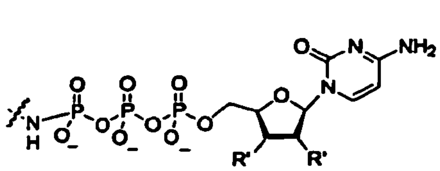
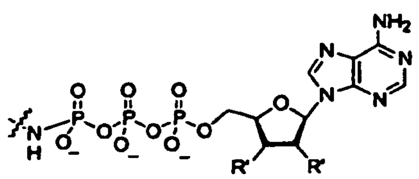

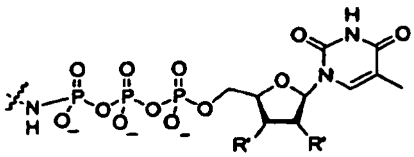
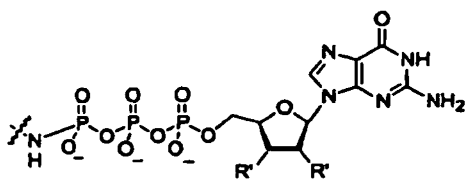

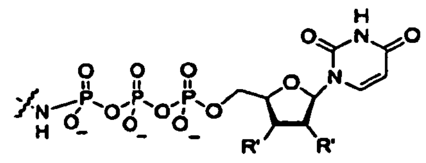

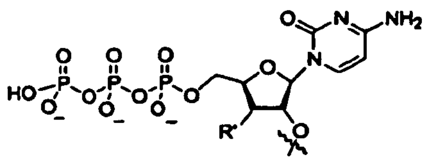

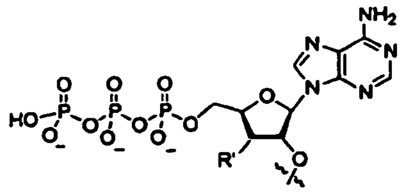

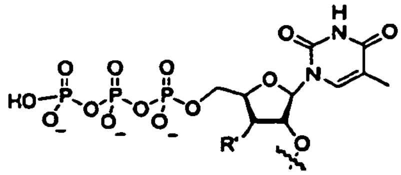

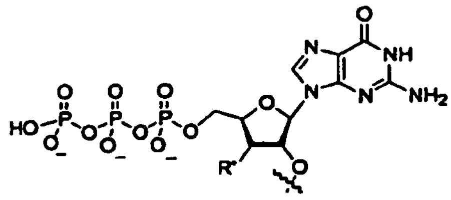

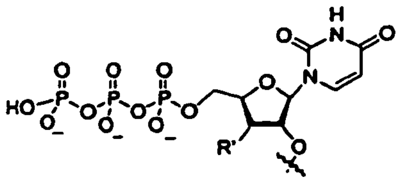

R' = H, OH

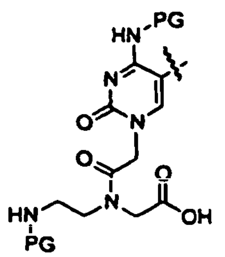

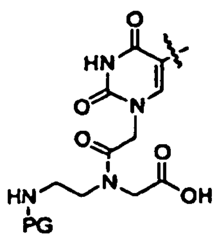

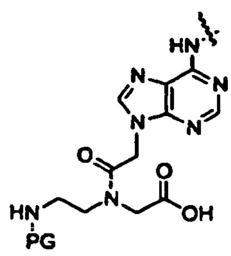

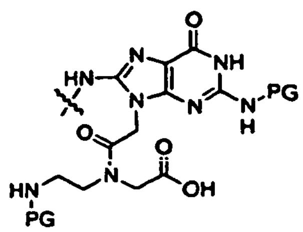

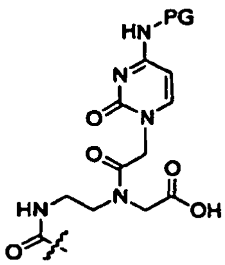

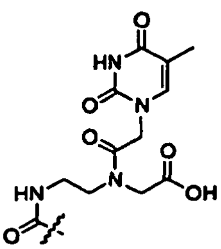

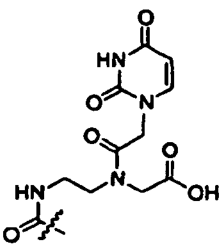

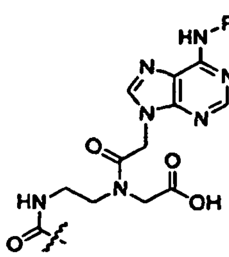

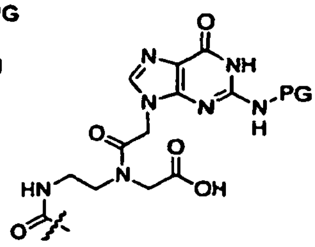

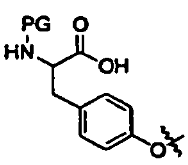

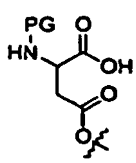

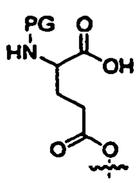

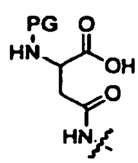

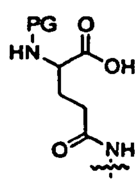

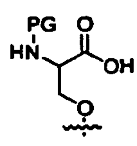

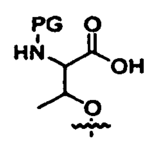

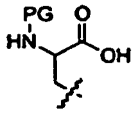 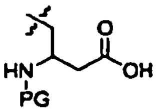 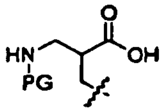 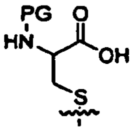 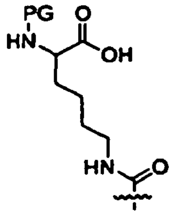 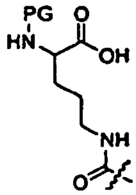 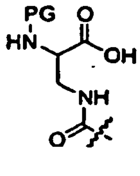

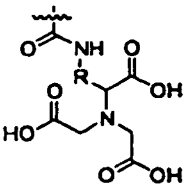 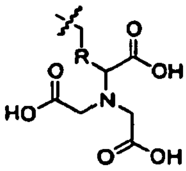

PG = Fmoc, Boc, Bsmoc, Bhoc

wobei R jeweils gleiche oder verschiedene Reste aus der folgenden Gruppe sind:

Wasserstoff, linearer, verzweigter oder cyclischer Alkyl- oder Alkoxyrest mit 1 bis 15 Kohlenstoffatomen, linearer oder verzweigter Alkenylrest mit 2 bis 15 Kohlenstoffatomen, geschütztes oder ungeschütztes Amin.

Durch MVG kann diese Verbindung an einen auf enzymatische Spaltbarkeit zu analysierenden Abschnitt (6) gekuppelt werden. Die Kupplung kann entweder direkt erfolgen oder indirekt, indem MVG an Baustein (4) oder (5) gekuppelt wird.

Gemäß einer besonderen Ausführungsform der Erfindung sind die Bausteine (4) und (5) der Komponente A aus der Gruppe bestehend aus PEG-2/D-Glutamat, PEG-2/Carboxyglutamat, PEG-9/D-Glutamat und PEG-9/Carboxyglutamat ausgewählt, und unabhängig davon sind die Bausteine (4) und (5) der Komponente B aus der Gruppe bestehend aus PEG-2/D-Glutamat, PEG-2/Carboxyglutamat, PEG-9/D-Glutamat und PEG-9/Carboxyglutamat ausgewählt.

Die Erfindung betrifft außerdem Kits zur Durchführung der erfindungsgemäßen Verfahren. Die erfindungsgemäßen Kits enthalten die erfindungsgemäßen Verbindungen oder die Komponenten A und/oder B der erfindungsgemäßen Verbindungen, Festphasen, z.B. in Form von Mikrotitrationsplatten, wobei diese mit entsprechenden Antikörpern beschichtet sein können, Puffer und Lösungen sowie optional verschiedene Enzyme oder auch Inhibitorsubstanzen.

In einer Ausführungsform der Erfindung enthält das Kit statt der vollständigen, erfindungsgemäßen Verbindungen sogenannte Rahmenkonstrukte.

Unter einem Rahmenkonstrukt wird in der vorliegenden Erfindung ein Konstrukt verstanden, das die beiden Komponenten A und B umfaßt, welche optional die Abstandhalter und negativen Ladungsträger enthalten. Dem Rahmenkonstrukt fehlt somit der in der Mitte lokalisierte zu analysierende Abschnitt, so daß das Rahmenkonstrukt aus zwei Teilen besteht, die mit einem zu analysierenden Abschnitt gekuppelt werden müssen, um die erfindungsgemäßen Verbindungen zu erhalten und das erfindungsgemäße Verfahren durchführen zu können.

Die Enzymkinetiken können mit Hilfe der Michaelis-Menten Gleichung ermittelt werden. Mit einem Programm zur Kurvenanpassung, z. B. GraphPad Prism 4 (GraphPad Software, San Diego, CA, USA), wird die Funktion der photometrischen Meßwerte zum Zeitpunkt t bzw. die Enzymkonzentration [E] durch nichtlineare Regression an eine Reaktionskinetik pseudo-erster Ordnung angepaßt. Die zugrundeliegende Formel der Exponentialfunktion für die Regression läßt sich aus der Michaelis-Menten Gleichung herleiten, und es gilt für den Fall, daß für die enzymatische Reaktion [S] >> [E] gewählt wird:

$$v = k_{cat}\,[E]_{gesamt}\,[S] / ([S] + K_M) \qquad (1)$$

| | |
|---|---|
| v | = Reaktionsgeschwindigkeit |
| $k_{cat}$ | = katalytische Konstante bzw. Wechselzahl |
| [S] | = Substratkonzentration |
| $[E]_{gesamt}$ | = Gesamtenzymkonzentration |
| $K_M$ | = Michaelis-Menten Konstante |

Unter den Reaktionsbedingungen pseudo-erster Ordnung ist $[S] < K_M$. Die Gleichung (1) vereinfacht sich zu:

$$v = k_{cat}\,[E]_{gesamt}\,[S] / K_M \qquad (2)$$

Die Reaktionsgeschwindigkeit v kann als Änderung der Substratkonzentration nach der Zeit betrachtet werden. Daher läßt sich (2) als Differentialgleichung schreiben:

$$\delta[S]_t / \delta t = - [S]_t [E]_{gesamt} k_{cat} / K_M \qquad (3)$$

$$\Leftrightarrow [S]_t = [S]_0 \exp(- t [E]_{gesamt} k_{cat} / K_M) \qquad (4)$$

$[S]_t$ = Substratkonzentration nach der Zeit t
$[S]o$ = Substratkonzentration ohne Inkontaktbringen mit dem Enzym
t = Zeit

$[E]_{gesamt}$ $k_{cat}$ / $K_M$ entspricht der Geschwindigkeitskonstante pseudo-erster Ordnung k, wobei für $[E]_{gesamt}$ in der bevorzugten Ausführungsform die Enzymkonzentration im Dünndarm einzusetzen ist.

Die photometrischen Messungen ergeben Werte als optische Dichte (OD), die proportional zum nicht umgesetzten Substrat [S] sind. Die Hintergrundsignale der Messungen werden bei der Regression an die Exponentialfunktion berücksichtigt. Es gilt:

$$OD_t = OD_{fin} + (OD_0 - OD_{fin}) \exp(- t [E]_{gesamt} k_{cat} / K_M) \qquad (5)$$

$OD_t$ = optische Dichte nach der Zeit t
$OD_{fin}$ = minimal erreichbare optische Dichte (entspricht dem Grenzwert der optischen Dichte für unendliche Reaktionszeit)
$OD_0$ = optische Dichte ohne Inkontaktbringen mit dem Enzym

Da die OD eine Funktion von t ist, können durch Bestimmung der OD nach verschiedenen Reaktionszeiten die 3 Parameter $OD_0$, $OD_{fin}$ und die Geschwindigkeitskonstante k (= $[E]_{gesamt}$ $k_{cat}$ / $K_M$) durch Kurvenanpassung ermittelt werden. $[E]_{gesamt}$ wird dabei in allen Messungen konstant gehalten.

In der bevorzugten Ausführungsfonn wird allerdings nicht die Zeit, sondern die Enzymkonzentration variiert. Dazu werden auf einer Mikrotitrationsplatte mehrere Enzymverdünnungen mit jeweils der gleichen Menge an Substrat in Kontakt gebracht und die Reaktionen über eine definierte Zeit t, in der bevorzugten Ausführungsform 90 Minuten, ablaufen lassen. Es gilt:

$$OD_{[E]} = OD_{[E]max} + (OD_0 - OD_{[E]max}) \exp(- t [E]_{gesamt} k_{cat} / K_M) \qquad (6)$$

$OD_{[E]}$ = optische Dichte bei einer bestimmten Enzymkonzentration
$OD_{[E]max}$ = minimal erreichbare optische Dichte (entspricht dem Grenzwert der optischen Dichte bei unendlicher Enzymkonzentration)
$OD_0$ = optische Dichte ohne Enzym

Analog zur Zeitabhängigkeit der OD bei gegebener Enzymkonzentration lassen sich durch die Enzymabhängigkeit der OD bei gegebener Zeit mittels Kurvenanpassung die 3 Parameter $OD_0$, $OD_{[E]max}$ und die Konstante (t $k_{cat}$ / $K_M$) bestimmen.

Für die Messung der Stabilität unterschiedlicher Substrate gegenüber einer Protease oder Proteasemischung wird in der bevorzugten Form die Halbwertszeit ($t_{1/2}$) eines Substrates bestimmt, denn der Beitrag einzelner Enzyme zum Substratabbau kann nicht genau aufgelöst werden. Es gilt:

$$½ [S]_0 = [S]_0 \exp(- t_{1/2} [E]_{gesamt} k_{cat} / K_M) \qquad (7)$$

$$\Leftrightarrow \ln 0{,}5 = - t_{1/2} [E]_{gesamt} k_{cat} / K_M \qquad (8)$$

$$\Leftrightarrow t_{1/2} = \ln 2 \, K_M / ([E]_{gesamt} k_{cat}) \qquad (9)$$

Setzt man Gleichung (9) in Gleichung (5) ein, so erhält man:

$$OD_t = OD_{fin} + (OD_0 - OD_{fin}) \exp(- t \ln(2) / t_{1/2}) \qquad (10)$$

$$\Leftrightarrow \quad OD_t = OD_{fin} + (OD_0 - OD_{fin})\ 0{,}5\hat{\ }(t / t_{1/2}) \qquad (11)$$

In Gleichung (11) kommt weder die katalytische Effizienz ($k_{cat}/K_M$) noch die genaue Enzymkonzentration [E] vor. Die mit Gleichung (11) bestimmte Halbwertszeit bezieht sich auf die verwendete Enzympräparation. Da die Substratumsätze, die bei Variation der Enzymmenge oder der Inkubationszeit (t) gemessen werden, proportional zueinander sind, solange die Bedingungen einer Reaktion erster Ordnung eingehalten werden, ist es möglich die Enzympräparation zu verdünnen, statt die Inkubationszeit zu variieren, und daraus folgt:

$$OD_{Vf} = OD_{max} + (OD_0 - OD_{max})\ 0{,}5\hat{\ }(t * Vf / t_{1/2}) \qquad (12)$$

Vf = Verdünnungsfaktor der Enzympräparation

Um die Effektivität eines Inhibitors und die Inhibitionskonstante $K_i$ zu bestimmen, wird der Substratumsatz sowohl in Anwesenheit wie auch in Abwesenheit des Inhibitors gemessen. Ist die zur Erzielung einer Inhibition eingesetzte Inhibitorkonzentration viel größer als die Enzymkonzentration, spricht man von einem "klassischen" Inhibitionsmechanismus. In diesem Fall darf bei der Auswertung nach der Michaelis-Menten-Kinetik die im Enzym-Inhibitor-Komplex gebundene Inhibitormenge vernachlässigt und $[I]_{frei} = [I]_{gesamt}$ gesetzt werden. Bei einem kompetitiven Inhibitor ergibt sich die Geschwindigkeitsgleichung (13):

$$v_i = k_{cat} [E]_{gesamt} [S] / ([S] + K_M (1 + [I]_{gesamt} / K_{i,app})) \qquad (13)$$

$v_i$ = Reaktionsgeschwindigkeit der inhibierten Reaktion
$[I]_{gesamt}$ = Gesamtinhibitorkonzentration
$K_{i,app}$ = scheinbare Inhibitionskonstante des Enzym-Inhibitor-Komplexes bei einer gegebenen Substratkonzentration; sie wird durch Gleichung (14) definiert:

$$K_{i,app} = K_i (1 + [S]/K_M) \qquad (14)$$

$K_i$ = Inhibitionskonstante

Unter den Reaktionsbedingungen pseudo-erster Ordnung ($[S] < K_M$) ergibt sich aus den Gleichungen (13) und (14) für die Reaktionsgeschwindigkeit der inhibierten Reaktion die Gleichung (15):

$$v_i = k_{cat} [E]_{gesamt} [S] / K_M (1 + [I]_{gesamt} / K_i) \qquad (15)$$

Gleichung (15) unterscheidet sich von Gleichung (2) dadurch, daß in Gegenwart eines Inhibitors die Michaelis Menten Konstante $K_M$ durch den erweiterten Term $K_M (1 + [I]_{gesamt} / K_i)$ ersetzt wird. Wird bei der inhibierten Reaktion $K_M (1 + [I]_{gesamt} / K_i)$ für $K_M$ in Gleichung (9) eingesetzt, gilt analog für die Halbwertszeit:

$$t_{1/2,i} = \ln 2\ K_M (1 + [I]_{gesamt} / K_i) / ([E]_{gesamt}\ k_{cat}) \qquad (16)$$

$t_{1/2,i}$ = Halbwertszeit der inhibierten Reaktion

Zur Bestimmung der Inhibitionskonstanten werden die Reaktionsgeschwindigkeiten oder Halbwertszeiten der inhibierten und der nicht inhibierten Reaktion ins Verhältnis gesetzt, und man erhält:

$$v_i/v_0 = t_{1/2,i} / t_{1/2,0} = 1 + [I]_{gesamt} / K_i \qquad (17)$$

$v_0$ = Reaktionsgeschwindigkeit der nicht inhibierten Reaktion
$t_{1/2,0}$ = Halbwertszeit der nicht inhibierten Reaktion

Wird eine enzymkatalysierte Reaktion beim Einsetzen equimolarer Mengen von Enzym und Inhibitor inhibiert, folgt sie dem "tight-binding"-Inhibitionsmechanismus. Dieser tritt ein, wenn es sich um einen hochaffinen Inhibitor handelt oder die Enzymkonzentration sehr hoch ist. In beiden Fällen wird die freie Inhibitorkonzentration durch die Ausbildung des Enzym-Inhibitor-Komplexes beträchtlich reduziert. Unter Berücksichtigung dieser Randbedingung läßt sich Reaktionsgeschwindigkeitsgleichung (18) ableiten (Morrison 1969, Bieth 1995).

$$v_i/v_0 = 1 - \{[E]_{gesamt} + [I]_{gesamt} + K_{i,app} - \{([E]_{gesamt} + [I]_{gesamt} + K_{i,app})^2 - 4[E]_{gesamt}\,[I]_{gesamt}\}^{0,5}\}/2\,[E]_{gesamt} \qquad (18)$$

Mit Gleichung (18) läßt sich unter Verwendung eines Iterationsprogramms, z.B. EnzFitter (Biosoft, Cambridge, UK), aus den gemessenen Reaktionsgeschwindigkeiten der inhibierten und nicht inhibierten Reaktion $K_{i,app}$ bestimmen und $K_i$ daraus ableiten.

Die Erfindung wird nachfolgend anhand von Beispielen und Figuren beschrieben, wobei es sich um bevorzugte Ausführungsformen der erfindungsgemäßen Verfahren handelt, die die Erfindung jedoch nicht einschränken.

Beschreibung der Figuren:

Figur 1 zeigt eine schematische Darstellung einer bevorzugten Ausführungsform des erfindungsgemäßen Verfahrens:

(Bezugszeichen: 1. Zellulosemembran; 2. Syntheseanker; 3. Biotin; 4. Negative Ladungen; 5. PEG-Abstandhalter; 6. Aminosäuresequenz des zu analysierenden Molekülbereichs; 7. Aminoundecansäure-Abstandhalter; 8. 2,4-Dichlorphenoxyessigsäure; 9. Protease; 10. Mikrotitrationsplatte; 11. Anti-2,4-D-Antikörper; 12. Streptavidin; 13. Meerrettichperoxidase; 14. TMB-Farbsubstrat)

(A) Oligopeptide werden in der folgenden Reihenfolge auf einer Zellulosemembran (1) synthetisiert: Syntheseanker (2), Biotin als detektierbare Gruppe bzw. Komponente 1 (3), Träger einer negativen Ladung (4), PEG-Abstandhalter (5), Sequenzmotiv des zu analysierenden Molekülbereichs aus n Aminosäuren (6), PEG-Abstandhalter (5), Träger einer negativen Ladung (4), Aminoundecansäure-Abstandhalter (7) und 2,4-Dichlorphenoxyessigsäure als detektierbare Gruppe bzw. Komponente 2 (8). Die Peptide werden nach Beendigung der Synthese von der Zellulosemembran abgespalten und
(B) mit einer Lösung von Proteasen (9) in Kontakt gebracht.
(C) Der Nachweis von gespaltenen und ungespaltenen Peptiden erfolgt an einer Mikrotitrationsplatte (10) über Anti-2,4-D-Antikörper (11), die das Peptid über 2,4-D (8) einfangen. Auf der anderen Seite des ungespaltenen Analyten bindet Biotin (3) Streptavidin (12), das mit dem Enzym Meerrettichperoxidase (13) gekoppelt ist. Die Meerrettichperoxidase wandelt farbloses TMB in die farbige, oxidierte Form (14) um.

**(Figur 2** zeigt den Einfluss unterschiedlicher flankierender Sequenzen auf die unspezifische Bindung von Peptiden an Mikrotitrationsplatten.
Die neun Sequenzmotive KIKVYLPRMK, VFKGLWEKAF, PVQMMYQIGL, VFKGLWEKAFKDE, KIKVYLPRMKMEE, FSLASRLYAEERY, ERKIKVYLPRMKMEEK, VQHFKRELMNLPQQCN, GLFRVASMASEKMKIL zeichnen sich durch eine starke Tendenz, unspezifisch an Mikrotitrationsplatten aus Polystyrol zu binden, aus. Es wurde die Möglichkeit untersucht, diese Plattenbindung zu unterdrücken, indem die Sequenzmotive mit Flankierungen bestehend aus hydrophilen ungeladenenen und/oder negativ geladenen Bausteinen versehen wurden. Mikrotitrationsplatten wurden mit einem 2,4-D nicht erkennenden Antikörper beschichtet und verbleibende Bindestellen auf der Platte danach mit 1% (w/v) Casein in DPBS abgesättigt. Die oben beschriebenen Peptide mit den unterschiedlichen Flankierungen wurden aufgetragen und ihre unspezifische Bindung an die Mikrotitrationsplatte mit Hilfe von Streptavidin-gekoppelter Meerretichperoxidase und einem chromogenen Substrat nachgewiesen. Das Diagramm zeigt die Absorptionswerte bei 450 nm, die bei Verwendung von 0,2% der Peptidmenge eines Synthese-SPOTs erzielt wurden; Werte ≥ 3 OD wurden durch Verwendung von 0,02% der Peptidmenge eines Synthese-SPOTs bestimmt und mit 10 multipliziert. Offene Balken zeigen die Werte des am stärksten unspezifisch an die Mikrotitrationsplatte

bindenden Peptids, geschlossene Balken geben den Mittelwert für die neun verschiedenen, stark unspezifisch bindenden Peptide wieder. Die unterschiedlichen flankierenden Bereiche der Peptide sind durch folgende Symbole gekennzeichnet: P9: Amino-Polyethylenglycol(PEG)-diglycolsäure mit 9 Ethylenglycoleinheiten; P2: Amino-Polyethylenglycol(PEG)-diglycolsäure mit 2 Ethylenglycoleinheiten; -: D-Glutamat (einfache negative Ladung); =: Carboxyglutamat (zweifach negative Ladung). Die carboxyterminale Markierung der mit einem * gekennzeichneten Flankierungsvariante war Amino-Polyethylenglycoi(PEG)-diglycolsäure mit 9 Ethylenglycoleinheiten und Biocytin. Alle anderen Flankierungsvarianten trugen N-y-(N-biotinyl-3-(2-(2-(3-aminopropytoxy)-ethoxy)-ethoxy)-propyl)-L-Glutamat als carboxyterminale Markierung. Aminoterminal waren alle Peptide mit dem Abstandhalter Aminoundecansäure und der Markierung 2,4-D versehen.
Die Einführung negativer Ladungen in den flankierenden Bereichen senkt die unspezifische Plattenbindung stärker als die Einführung ungeladener, hydrophiler Bausteine. Dabei ist die Stärke der Reduzierung der Plattenbindung abhängig von der Zahl der eingeführten negativen Ladungen.

**Figur 3** zeigt die Auswertung von spaltungsexperimenten bei Kinetik pseudo-erster Ordnung. Der Abbau des Substrats GPARLA durch Trypsin und von GVPFGP durch Chymotrypsin ist gezeigt. (A) In einer Variation der Ausführungsform erfolgte die Bestimmung der Halbwertszeit durch Variation der Inkubationszeit und (B) in der bevorzugten Ausführungsform durch die Variation der Enzymkonzentration. In (A) wird [E] konstant gehalten (Trypsinkonzentration: 5 ng/ml bzw. 0,2 nM; Chymotrypsinkonzentration: 500 ng/ml bzw. 20 nM) und t variiert, während in (B) t konstant gehalten (jeweils 90 min) und [E] variiert wird. $OD_0$, $OD_{max}$ und $t_{1/2}$ werden jeweils als Parameter an der Regressionskurve optimiert. Aus $t_{1/2}$ läßt sich die katalytische Effizienz ($k_{cat}/K_M$) berechnen. Die nach beiden Verfahren bestimmten Werte für $k_{cat}/K_M$ unterscheiden sich nicht signifikant voneinander (5-fach Messung; ungepaarter t-Test: $p > 0{,}05$). In beiden Fällen ist der exponentielle Verlauf einer Reaktion pseudo-erster Ordnung deutlich zu erkennen.

**Figur 4** zeigt den Einfluss unterschiedlicher flankierender Sequenzen auf die katalytische Effizienz <$k_{cat}/K_M$) bei der enzymatischen Spaltung von Peptiden.
Je drei verschiedene Peptide, deren Aminosäuresequenzmotiv flankiert war von unterschiedlichen negativ geladenen und/oder hydrophilen Bausteinen, wurden mit unterschiedlichen Mengen von Trypsin (Figur 4 oben) bzw. Chymotrypsin (Figur 4 unten) inkubiert und der Grad der Hydrolyse durch einen nachgeschalteten Enzymimmunassay bestimmt. Durch nichtlineare Anpassung des Kurvenverlaufs der Absorptionswerte in Abhängigkeit von der Enzymmenge konnten die $k_{cat}/K_M$-Werte bestimmt werden. Die Bezeichnung der Flankierungsvarianten ist analog zu Figur 2.

## Beispiele

### Beispiel 1

### Herstellung von Oligopeptiden mittels SPOT-Synthese

Die Synthese der Oligopeptide mit einer Sequenzlänge von bis zu 16 Aminosäuren erfolgte mit dem FMOC-Syntheseverfahren für Zellulosemembran-immobilisierte Peptidbibliotheken (Frank, 1992). Alle Arbeitsschritte wurden bei RT durchgeführt. Zellulosemembranen wurden mit 0,02 ml/cm$^2$ der Fluorenylmethoxycarbonyl (Fmoc)-geschützten Aminosäure Prolin (0,2 M Fmoc-Prolin, 0,46 M 1-Methylimidazol und 0,26 M N,N'-Diisopropylcarbodiimid (DICD) in trockenem N,N'-Dimethylformamid (DMF)) verestert. Nach der Kupplungsreaktion wurden nicht umgesetzte reaktive Gruppen für 24 h mit 0,13 ml/cm$^2$ 2 % (v/v) Essigsäureanhydrid in DMF abgesättigt. Anschließend wurde dreimal mit DMF gewaschen. Die Abspaltung der Fmoc-Schutzgruppe erfolgte für 5 min durch 0,09 ml/cm$^2$ 20 % (v/v) Piperidin in DMF. Danach wurde viermal mit DMF gewaschen. Anschließend wurde dreimal mit 100 % Ethanol gewaschen, und die Membranen wurden im Kaltluftstrom getrocknet.
Die folgenden Syntheseschritte wurden mit einer automatischen Pipettiermaschine (z.B. ASP 222, Intavis, Köln) durchgeführt. Zunächst wurde eine Lösung aus 0,2 M tert-Butyloxycarbonyl-(Boc)-lysin-(Fmoc)-OH und 0,35 M 1-Hydroxy-benzotriazol (HoBt) sowie 0,25 M N,N'-Diisopropylcarbodiimid (DICD) in trockenem, entsalztem 1-Methyl-2-pyrrolidon (NMP) 30 min vor ihrer Verwendung bereitet und bei RT inkubiert. Nach 30 min Reaktionszeit wurde die Lösung zentrifugiert, um aufgetretene Präzipitate zu entfernen, und jeweils 0,1 µl dieser Lösung wurden mit Hilfe der automatischen Pipettiermaschine auf definierte Areale der Zellulosemembran pipettiert. Areale, auf welche die Kupplungslösungen pipettiert werden, werden als SPOTs bezeichnet.
Nach der Kupplungsreaktion wurden nicht umgesetzte reaktive Gruppen für 24 h mit 0,13 ml/cm$^2$ 2 % (v/v). Essigsäureanhydrid in DMF abgesättigt. Anschließend wurde dreimal mit DMF gewaschen. Die Abspaltung der Fmoc-Schutzgruppe erfolgte für 5 min durch 0,09 ml/cm$^2$ 20 % (v/v) Piperidin in DMF. Danach wurde fünfmal mit DMF

gewaschen. Als Nachweis für die Kupplungsreaktionen wurden die freien Aminogruppen auf der Zellulosemembran für 10 min mit 0,13 ml/$cm^2$ einer Bromphenolblaulösung (0,01 % (w/v) in DMF) angefärbt. Anschließend wurde dreimal mit 100 % Ethanol gewaschen, und die Membranen wurden im Kaltluftstrom getrocknet.

Dieser Inkubationszyklus, bestehend aus 1x Essigsäureanhydrid, 3x DMF, 1x Piperidin, 5x DMF, 1x Bromphenolblau und 3x Ethanol wurde auch zwischen allen nachfolgenden Syntheseschritten durchgeführt. Dabei wurde jedoch die Inkubationszeit mit der Essigsäureanhydridlösung, die dann nur noch zum Absättigen der nicht reagierten Aminofunktionen dient, auf 20 min verkürzt.

Zur Verlängerung der Peptidkette wurden für jedes Peptid jeweils 0,2 µl einer 0,2 M Aminosäureaktivesterlösung appliziert. Zur Herstellung der Aminosäureaktivesterlösungen wurde eine Lösung von 0,2 M Fmoc-Aminosäure, deren Seitenkette, falls erforderlich, mit geeigneten Gruppen (tert.-Butyl (tBu) für Serin, Threonin, Tyrosin, Glutaminsäure und Asparaginsäure; Trityl für Asparagin, Glutamin, Histidin; t-Butyl-oxycarbonyl (Boc) für Lysin und Tryptophan; 2,2,4,6,7-Pentamethyldihydrobenzofuran-5-sulfonyl (Pbf) für Arginin und Acetamidomethyl (Acm) für Cystein) geschützt war, und 0,35 M 1-Hydroxybenzotriazol (HoBt) in trockenem, entsalztem N-Methylpyrrolidinon mit 1,25 Mol DICD pro Mol Aminosäure versetzt und 30 min bei RT reagieren lassen. Anschliessend wurden entstandene Präzipitate durch Zentrifugation entfernt. Für jeden Syntheseschritt wurde die Aminosäureaktivesterlösung dreimal aufgebracht und jeweils mindestens 40 min bei RT reagieren lassen.

Die Seitenkettenschutzgruppen (außer Acm) wurden durch zwei einstündige Inkubationen mit 0,09 ml/$cm^2$ einer Lösung aus 50 % (v/v) Trifluoressigsäure, 2 % (v/v) destilliertem Wasser und 3 % (v/v) Triisobutylsilan in Dichlormethan abgespalten. Anschließend wurden die zellulosegebundenen Peptide viermal mit Dichlormethan, dann viermal mit 0,1 % (v/v) HCl, 50 % (v/v) Methanol in zweifach destilliertem Wasser und schließlich viermal mit 1 M Essigsäure, pH 1,9 gewaschen. Die Membran wurde über Nacht im Vakuum getrocknet. Die SPOTs wurden ausgestanzt und in 2 ml-Mikroreaktionsgefäße transferiert. Um die Peptide von den Membranschnipseln abzuspalten, wurden 500 µl einer Lösung aus 0,1 M Triethylammoniumacetat (TEAA), 20 % (v/v) Ethanol, pH 7,5 in zweifach destilliertem Wasser zu jedem Membranschnipsel gegeben. In dieser Form wurde über Nacht inkubiert, die Überstände wurden anschließend in ein frisches 2 ml-Mikroreaktionsgefäß gegeben und die Abspaltungsreaktion wurde für 2 h wiederholt. Beide Peptidlösungen wurden vereinigt und das Lösungsmittel im Vakuum entfernt. Die so getrockneten Peptide wurden in 1,5 ml 10 mM Natriumphosphatpuffer, pH 7,0, 10 mM NaCl (L-PBS) x 0,005 % (w/v) Tween 20 gelöst, in flüssigem Stickstoff schockgefroren und bei -80° C gelagert.

Die nachfolgende Liste gibt eine Übersicht über die synthetisierten Peptide.

Sequenzen sind von aminoterminaler in carboxyterminale Richtung angegeben. Die Synthesebausteine sind wie folgt definiert: 2,4D = 2,4-Dichlorphenoxyessigsäure; Aun = Aminoundecansäure; PEG9 = Amino-Polyethylenglycol (PEG)-diglycolsäure mit 9 Ethylenglycoleinheiten; PEG2 = Amino-Polyethylenglycol(PEG)-diglycolsäure mit 2 Ethylenglycoleinheiten; LysBio = Biocytin; Ank = Syntheseanker, bestehend aus Lysin und Prolin; glu = D-Glutamat; GluPEGBio = N-γ-(N-biotinyl-3-(2-(2-(3-aminopropyloxy)-ethoxy)-ethoxy)-propyl)-L-Glutamat; Gla = L-Carboxyglutamat

1. 2,4D-Aun-XXX-PEG9-LysBio-Ank
2. 2,4D-Aun-XXX-GluPEGBio-Ank
3. 2,4D-Aun-XXX-glu-GluPEGBio-Ank
4. 2,4D-Aun-glu-XXX-GluPEGBio-Ank
5. 2,4D-Aun-glu-XXX-glu-GluPEGBio-Ank
6. 2,4D-Aun-XXX-glu-glu-GluPEGBio-Ank
7. 2,4D-Aun-glu-XXX-glu-glu-GluPEGBio-Ank
8. 2,4D-Aun-glu-glu-XXX-glu-GluPEGBio-Ank
9. 2,4D-Aun-glu-glu-XXX-GluPEGBio-Ank
10. 2,4D-Aun-glu-glu-XXX-glu-glu-GluPEGBio-Ank
11. 2,4D-Aun-XXX-Gla-GluPEGBio-Ank
12. 2,4D-Aun-Gla-XXX-GluPEGBio-Ank
13. 2,4D-Aun-Gla-XXX-Gla-GluPEGBio-Ank
14. 2,4D-Aun-XXX-Gla-Gla-GluPEGBio-Ank
15. 2,4D-Aun-Gla-XXX-Gla-Gla-GluPEGBio-Ank
16. 2,4D-Aun-Gla-Gla-XXX-Gla-GluPEGBio-Ank
17. 2,4D-Aun-Gla-Gla-XXX-GluPEGBio-Ank
18. 2,4D-Aun-Gla-Gla-XXX-Gla-Gla-GluPEGBio-Ank
19. 2,4D-Aun-XXX-PEG2-GluPEGBio-Ank
20. 2,4D-Aun-PEG2-XXX-GluPEGBio-Ank
21. 2,4D-Aun-PEG2-XXX-PEG2-GluPEGBio-Ank
22. 2,4D-Aun-XXX-PEG9-GluPEGBio-Ank
23. 2,4D-Aun-PEG9-XXX-GluPEGBio-Ank

24. 2,4D-Aun-PEG9-XXX-PEG9-GluPEGBio-Ank
25. 2,4D-Aun-PEG2-XXX-PEG2-glu-GluPEGBio-Ank
26. 2,4D-Aun-PEG2-XXX-PEG2-Gla-GluPEGBio-Ank
27. 2,4D-Aun-glu-PEG2-XXX-PEG2-GluPEGBio-Ank
28. 2,4D-Aun-Gla-PEG2-XXX-PEG2-GluPEGBio-Ank
29.2,4D-Aun-glu-PEG2-XXX-PEG2-glu-GluPEGBio-Ank
30. 2,4D-Aun-Gla-PEG2-X3GC-PEG2-Gla-GluPEGBio-Ank
31. 2,4D-Aun-PEG2-XXX-glu-GluPEGBio-Ank
32. 2,4D-Aun-PEG2-XXX-glu-glu-GluPEGBio-Ank
33. 2,4D-Aun-PEG2-XXX-Gla-GluPEGBio-Ank
34. 2,4D-Aun-PEG2-XXX-Gla-Gla-GluPEGBio-Ank
35. 2,4D-Aun-glu-PEG2-XXX-GluPEGBio-Ank
36. 2,4D-Aun-glu-PEG2-XXX-glu-GluPEGBio-Ank
37.2,4D-Aun-glu-PEG2-XXX-glu-glu-GluPEGBio-Ank
38. 2,4D-Aun-Gla-PEG2-XXX-GluPEGBio-Ank
39. 2,4D-Aun-Gla-PEG2-XXX-Gla-GluPEGBio-Ank
40. 2,4D-Aun-Gla-PEG2-XXX-Gla-Gla-GluPEGBio-Ank
41. 2,4D-Aun-glu-glu-PEG2-XXX-PEG2-glu-glu-GluPEGBio-Ank

wobei XXX stellvertretend für folgende Sequenzmotive steht (Aminosäuren sind entsprechend dem Standard-Ein-Buchstabencode angegeben):

a) KIKVYLPRMK
b) VFKGLWEKAF
c) PVQMMYQIGL
d) AADQARELIN
e) GSIGAASMEF
f) VFKGLWEKAFKDE
g) KIKVYLPRMKMEE
h) FSLASRLYAEERY
i) VDAASVSEEFRAD
j) RIMGEQEQYDSYN
k) ERKIKVYLPRMKMEEK
l) VQHFKRELMNLPQQCN
m) GLFRVASMASEKMKIL
n) AEAGVDAASVSEEFRA
o) MLVLLPDEVSGLEQLE

42. 2,4D-Aun-GPARLA-PEG9-LysHio-Ank
43. 2,4D-Aun-GVPFGP-PEG9-LysBio-Ank
44. 2,4D-Aun-GGSGPFGRSALVPEE-PEG9-LysBio-Ank
45. 2,4D-Aun-GGSGPDGRSALVPEE-PEG9-LysBio-Ank
46. 2,4D-Aun-GGSGPFGRSDLVPEE-PEG9-LysBio-Ank
47. 2,4D-Aun-PAPFAAA-PEG9-LysBio-Ank
48. 2,4D-Aun-GPARLAIG-PEG9-LysBio-Ank

sowie alle 185 bzw. 188 Sequenzmotive, die sich ergeben, wenn das Modellantigen Ovalbumin stückweise als Peptide mit einer Länge von jeweils 16 bzw. 10 Aminosäuren und einem Vorschub von 2 Aminosäuren synthetisiert wird. Diese aus Ovalbumin abgeleiteten Peptide wurden mit den flankierenden Bereichen No. 41 synthetisiert.

Ovalbumin-Aminosäuresequenz:

GSIGAASMEFCFDVFKELKVHHANENIFYCPIAIMSALAMVYLGAKDSTRTQINKVVRFDKL
PGFGDSIEAQCGTSVNVHSSLRDILNQTKPNDVYSFSLASRLYAEERYPILPEYLQCVKELY
RGGLEPINFQTAADQARELINSWVESQTNGIIRNVLQPSSVDSQTAMVLVNAIVFKGLWEKA
FKDEDTQAMPFRVTEQESKPVQMMYQIGLFRVASMASEKMKILELPFASGTMSMLVLLPDEV
SGLEQLESIINFEKLTEWTSSNVMEERKIKVYLPRMKMEEKYNLTSVLMAMGITDVFSSSAN
LSGISSAESLKISQAVHAAHAEINEAGREVVGSAEAGVDAASVSEEFRADHPFLFCIKHIAT
NAVLFFGRCVSP

Beispiel 2

Gewinnen einer intestinalen Enzymlösung aus dem Dünndarm von Mäusen mittels Darmlavage

Narkotisierte weibliche Balb/c Mäuse wurden durch zervikale Dislokation getötet und ihre Bauchdecke geöffnet. Der Dünndarm wurde hinter dem Magenausgang (proximales Ende) mit einem Hämostaten abgeklemmt und zum Magen hin durchtrennt. Der Dünndarm wurde freigelegt und mit einem zweiten Hämostaten nahe dem hinteren (distalen) Ende abgeklemmt. Hinter dieser Stelle wurde der Darm ein zweites Mal durchtrennt.

Die anschließenden Präparationsschritte zur Gewinnung der Verdauungssekrete wurden auf einer eisgekühlten Oberfläche durchgeführt. Der beidseitig verschlossene Dünndarm wurde in eisgekühlter simulierter Intestinalflüssigkeit (SIF; 8 mM Phosphat, pH 7,2, mit 4,6 mM $K^+$, 111,3 mM $Na^+$, 101,5 mM Cl) nach Lockwood und Randall (1949), welche die physiologischen Bedingungen des Dünndarms hinsichtlich der Ionenkonzentrationen möglichst genau imitieren sollte, geschwenkt, um äußere Verunreinigungen zu entfernen und ihn feucht zu halten. Zur Gewinnung der im Dünndarm enthaltenen Verdauungssekrete wurde das distale Ende des Darms in ein Reaktionsgefäß gehängt und der an dieser Seite befestigte Hämostat entfernt. Anschließend wurde am proximalen Darmende ein seitlicher Schnitt gesetzt, so daß eine mit einer Knopfkanüle versehene Spritze in den spannungsfrei gehaltenen Darm eingeführt werden konnte. Das Auswaschen des Darminhalts erfolgte in drei Schritten. Die Waschlösungen wurden nacheinander langsam in den Darm injiziert und an der unteren Darmseite getrennt in drei Reaktionsgefäßen aufgesammelt. Zunächst wurde der Darm mit einem Volumen von 6 ml Perfluorhexan, einer mit Wasser nicht mischbaren, inerten Flüssigkeit gespült und das Perfluorhexan mit 4 ml Luft ausgetrieben. Dann wurde mit einer Mischung von 2,25 ml Perfluorhexan und 0,25 ml SIF gewaschen, die die nicht-physiologischen Ionen $Li^+$ und $Cs^+$ enthielt, um nachfolgend über spektroskopische Verfahren die Verdünnung bestimmen zu können. Beide Ionen haben keinen Einfluß auf die Proteaseaktivität von Darmlavage. In einem dritten Waschschritt wurden 5 ml SIF ohne Tracer injiziert. Durch Zentrifugation wurden feste Bestandteile des Darminhalts und das Perfluorhexan von der wäßrigen Phase, in der sich die Verdauungsenzyme befanden, abgetrennt. Mit Hilfe einer Inductively Coupled Plasma - Mass Spectrometry (ICP-MS) wurden die Konzentrationen von $Li^+$ und $Cs^+$ in der intestinalen Enzymlösung bestimmt und unter Berücksichtigung des Volumens des Waschpuffers der Verdünnungsfaktor berechnet. Die Verdünnung der dritten Lavage wurde über endogene Marker des Intestinalsekrets (z.B. Enzymaktivitäten, Immunglobuline) im Vergleich mit den ersten beiden intestinalen Enzymlösungen ermittelt. Die Lagerung der Enzymlösungen erfolgte nach Einfrieren mit flüssigem Stickstoff bei -70° C.

Beispiel 3

Untersuchung des Einflusses von hydrophilen Gruppen und Ladungen auf das unspezifische Bindeverhalten von Peptiden an Mikrotitrationsplatten

Zur Analyse der unspezifischen Bindung an die Mikrotitrationsplatten wurden die folgenden in Beispiel 1 aufgeführten Peptide verwendet: Rahmenkonstrukt 1 bis 41 in Kombination mit den Sequenzmotiven a-c, f-h, k-m. Für das Enzymimmunoverfahren wurden high-bind Mikrotitrationsplatten mit 96 (8 x 12) Vertiefungen (z. B. von Costar/Corning, Wiesbaden) verwendet. Zur Beschichtung der Platten wurden 75 µl einer frisch bereiteten Lösung von 50 ng/ml polyklonales Mäuse-IgG, das keine Spezifität für 2,4-D aufwies, in L-PBS in jede einzelne Vertiefung pipettiert. Nach Inkubation über Nacht bei 4° C wurde die Beschichtungslösung abgesaugt, und die Vertiefungen der Mikrotitrationsplatte wurden mit einem tensidhaltigen Puffer (DPBST) (Dulbecco's PBS (DPBS): 2,7 mM KCl, 1,5 mM $KH_2PO_4$, 136 mM NaCl, 8,1 mM $Na_2HPO_4$, pH 7,3; DBST: DPBS x 0,05 % (w/v) Tween 20) mit Hilfe eines automatischen Plattenwaschers (z.B. Columbus Washer, Tecan, Crailsheim) dreimal gewaschen und anschließend leergesaugt. Um unspezifische Anlagerungen an die Mikrotitrationsplatte so weit wie möglich zu unterdrücken, wurden deren Vertiefungen mit einer Absättigungslösung (1% (w/v) Casein in DPBS) gefüllt. Nach einer erneuten mindestens dreistündigen Inkubation bei

RT wurde die Absättigungslösung wie zuvor abgesaugt, die Mikrotitrationsplatte viermal mit DPBST gewaschen. Die leergesaugten Vertiefungen wurden mit Peptidlösung gefüllt. Dazu wurden die nach der Peptidsynthese in Beispiel 1 erhaltenen Peptidlösungen 1:10 in L-PBS × 0,005 % (w/v) Tween 20 verdünnt. Je Peptid wurde eine Vertiefung einer Mikrotitrationsplatte mit 45 µl, eine weitere mit 72 µl SIF x 0,005 % (w/v) Tween 20 gefüllt. In die erste Vertiefung wurden sodann 30 µl, in die zweite 3 µl der jeweiligen verdünnten Peptidlösung gegeben. In 6 Vertiefungen jeder Mikrotitrationsplatte wurde nur 75 µl SIF x 0,005 % (w/v) Tween 20 für die Bestimmung eines Hintergrundsignals gegeben. Nach einer Inkubationszeit von 2,5 h bei RT wurden die nicht gebundenen Bestandteile des Gemisches durch Absaugen und viermaliges Waschen mit DPBST entfernt und die Vertiefungen leergesaugt. Durch Zugabe von 75 µl 1 µg/ml peroxidasemarkiertem Streptavidin in DPBS mit 1% (w/v) Casein wurden an die Platte gebundene Peptide mit Peroxidase als Reporterenzym versehen. Nach einer Stunde wurde die Streptavidin-Lösung abgesaugt und die Mikrotitrationsplatte sechsmal mit DPBST gewaschen und leergesaugt. Das Entwickeln des Farbsignals erfolgte nach Zugabe von jeweils 75 µl eines Tetramethylbenzidin-Farbsubstrats in jede Vertiefung der Mikrotitrationsplatte im Dunklen und wurde nach 30 min durch Zugabe von je 125 µl 1 M Schwefelsäure abgebrochen. Das Farbsignal wurde mit einem Mikrotitrationsplattenlesegerät (z.B. VersaMax, Molecular Devices, Ismaning) bei einer Wellenlänge von 450 nm photometrisch bestimmt.

Figur 2 zeigt die unterschiedlich starke unspezifische Plattenbindung, die mit verschiedenen Peptiden in Abhängigkeit von den unterschiedlichen flankierenden Bereichen erhalten wurde.

**Beispiel 4**

**Beschichtung und Absättigen der Vertiefungen von Mikrotitrationsplatten zur nachfolgenden Beladung mit 2,4-D-markierten Peptiden**

Für das Enzymimmunoverfahren wurden high-bind Mikrotitrationsplatten mit 96 (8 x 12) Vertiefungen (z. B. von Costar/Corning, Wiesbaden) verwendet. Zur Beschichtung der Platten wurden 75 µl einer frisch bereiteten Lösung von 50 ng/ml Anti-2,4-D-Antikörper (Franek et al., 1994) in L-PBS in jede einzelne Vertiefung pipettiert. Nach Inkubation über Nacht bei 4° C wurde die Beschichtungslösung abgesaugt, und die Vertiefungen der Mikrotitrationsplatte wurden mit einem tensidhaltigen Puffer (DPBST) (Dulbecco's PBS (DPBS): 2,7 mM KCl, 1,5 mM $KH_2PO_4$, 136 mM NaCl, 8,1 mM $Na_2HPO_4$, pH 7,3; DBST: DPBS x 0,05 % (w/v) Tween 20) mit Hilfe eines automatischen Plattenwaschers (z.B. Columbus Washer, Tecan, Crailsheim) dreimal gewaschen und anschließend leergesaugt. Um unspezifische Anlagerungen an die Mikrotitrationsplatte zu verhindern, wurden deren Vertiefungen mit einer Absättigungslösung (1% (w/v) Casein in DPBS) gefüllt. Nach einer erneuten mindestens dreistündigen Inkubation bei RT wurde die Absättigungslösung wie zuvor abgesaugt, die Mikrotitrationsplatte viermal mit DPBST gewaschen und die Vertiefungen leergesaugt.

**Beispiel 5**

**Enzymatische Umsetzung von Substraten mit einer definierten Enzymlösung von Trypsin und Chymotrypsin**

Auf einer Mikrotitrationsplatte aus Polypropylen, die das gleiche Format wie die beschichtete Mikrotitrationsplatte aus Polystyrol besaß, wurden in der ersten Vertiefung einer Reihe 7,5 µl Peptidlösung und in den nächsten 10 Vertiefungen jeweils 5 µl der Peptidlösung vorgelegt. Für das Enzym Trypsin wurde ein Peptid mit der Aminosäuresequenz GPARLA (Nummer 42 aus Beispiel 1) und für das Enzym Chymotrypsin ein Peptid mit der Sequenz GVPFGP (Nummer 43 aus Beispiel 1) gewählt. Die letzte Vertiefung, die als Hintergrundkorrektur dienen sollte, blieb leer. Anschließend wurde in alle Vertiefungen simulierte Intestinalflüssigkeit (SIF) mit einem Zusatz von 0,005 % Tween 20 und 1 mM CaCl (SIFT-CaCl) gegeben. Dabei erhielt die Vertiefung mit 7,5 µl Peptidlösung 60 µl SIFT-CaCl, die Vertiefungen mit 5 µl Peptidlösung je 45 µl SIFT-CaCl. Die leere Vertiefung für die Hintergrundkorrektur erhielt 50 µl SIFT-CaCl. Anschließend wurde die enzymatische Reaktion durch die Zugabe von 7,5 µl **Enzymlösung** (10 ug/ml Trypsin- bzw. Chymotrypsinlösung) auf die Vertiefung mit 7,5 µl Peptidlösung und dem entsprechenden Volumen SIFT-CaCl gestartet. Von dieser Mischung wurden sofort 25 µl in die nachfolgende Vertiefung pipettiert, gemischt, und dieser Schritt so oft wiederholt, daß eine serielle 1:3-Verdünnung der Enzymlösung über insgesamt 10 Vertiefungen entstand. In die letzte Peptid-enthaltende Vertiefung wurde keine Enzymlösung gegeben. Somit lag in jeder Vertiefung nach der Verdünnungsreihe ein Volumen von 50 µl vor. Die Polypropylenplatte wurde mit einem Noppendeckel verschlossen und 90 min bei 37° C inkubiert. Anschließend wurde die enzymatische Reaktion durch die Zugabe von 50 µl einer Proteaseinhibitorlösung (308 nM Aprotinin, 20 µM Leupeptin, 400 µM 4-(2-Aminoethyl)benzolsulfonylfluorid (AEBSF)-Hydrochlorid in L-PBS) in jede Vertiefung beendet, und die Mikrotitrationsplatte mit einem Noppendeckel verschlossen. Nach 10 min bei 4° C wurde die Mikrotitrationsplatte für 10 min auf 90° C erhitzt und vor der weiteren Verwendung für mindestens 5 min auf 0° C gekühlt.

**Beispiel 6**

**Inhibition einer enzymatischen Umsetzung**

Zur Messung von Inhibitionskonstanten wurden jeweils zwei parallele Enzymverdünnungen durchgeführt. Eine Enzymverdünnung wurde in Anwesenheit und die andere Enzymverdünnung in Abwesenheit des Proteaseinhibitors Aprotinin durchgeführt. Auf einer Mikrotitrationsplatte aus Polypropylen, die das gleiche Format wie die beschichtete Mikrotitrationsplatte aus Polystyrol besaß, wurden in der ersten Vertiefung einer Reihe 7,5 µl Peptidlösung und in den nächsten 10 Vertiefungen jeweils 5 µl der Peptidlösung vorgelegt. Für das Enzym Trypsin wurde ein Peptid mit der Aminosäuresequenz GPARLA (Nummer 42 aus Beispiel. 1) und für das Enzym Chymotrypsin ein Peptid mit der Sequenz GVPFGP (Nummer 43 aus Beispiel 1) gewählt. Die letzte Vertiefung, die als Hintergrundkorrektur dienen sollte, blieb leer. Anschließend wurde für die Durchführung der proteolytischen Spaltung in Anwesenheit eines Inhibitors in die erste Vertiefung 7,5 µl einer Aprotininlösung (1 µg/ml für Trypsin und 1 mg/ml für Chymotrypsin) und in die nächsten 10 Vertiefungen jeweils 5 µl der Aprotininlösung (1 µg/ml für Trypsin und 1 mg/ml für Chymotrypsin) gegeben. Die letzte Vertiefung, die als Hintergrundkorrektur dienen sollte, blieb leer. Anschließend wurde in alle Vertiefungen simulierte Intestinalflüssigkeit (SIF) mit einem Zusatz von 0,005 % (w/v) Tween 20 und 1 mM CaCl (SIFT-CaCl) gegeben. Dabei erhielt die Vertiefung mit 7,5 µl Peptidlösung ohne Aprotininlösung 60 µl SIFT-CaCl und die Vertiefungen mit 5 µl Peptidlösung ohne Aprotininlösung je 45 µl SIFT-CaCl. Die Vertiefung mit 7,5 µl Peptidlösung und 7,5 µl Aprotininlösung erhielt 52,5 µl SIFT-CaCl und die Vertiefungen mit 5 µl Peptidlösung und 5 µl Aprotininlösung je 40 µl SIFT-CaCl. Die leere Vertiefung für die Hintergrundkorrektur erhielt 50 µl SIFT-CaCl. Anschließend wurde die enzymatische Reaktion durch die Zugabe von 7,5 µl Enzymlösung (10 µg/ml Trypsin- bzw. 1 mg/ml Chymotrypsinlösung) auf die Vertiefung mit 7,5 µl Peptidlösung und 60 µl SIF-CaCl bzw. 52,5 µl SIF-CaCl und 7,5 µl Aprotininlösung gestartet. Von dieser Mischung wurden sofort 25 µl in die nachfolgende Vertiefung pipettiert, gemischt, und dieser Schritt so oft wiederholt, daß eine serielle 1:3-Verdünnung der Enzymlösung über insgesamt 10 Vertiefungen entstand. In die letzte Peptid-enthaltende Vertiefung wurde keine Enzymlösung gegeben. Somit lag in jeder Vertiefung nach der Verdünnungsreihe ein Volumen von 50 µl vor. Die Polypropylenplatte wurde mit einem Noppendeckel verschlossen und 90 min bei 37° C inkubiert. Anschließend wurde die enzymatische Reaktion durch die Zugabe von 50 µl einer Proteaseinhibitorlösung (308 nM Aprotinin, 20 µM Leupeptin, 400 µM 4-(2-Aminoethyl)benzolsulfonylfluorid (AEBSF)-Hydrochlorid in L-PBS) in jede Vertiefung beendet, und die Mikrotitrationsplatte mit einem Noppendeckel verschlossen. Nach 10 min bei 4° C wurde die Mikrotitrationsplatte für 10 min auf 90° C erhitzt und vor der weiteren Verwendung für mindestens 5 min auf 0° C gekühlt.

**Beispiel 7**

**Untersuchung des Einflusses von hydrophilen Gruppen in den flankierenden Sequenzen auf die enzymatische Spaltbarkeit von Peptiden**

Auf einer Mikrotitrationsplatte aus Polypropylen, die das gleiche Format wie die beschichtete Mikrotitrationsplatte aus Polystyrol besaß, wurden in der ersten Vertiefung einer Reihe 7,5 µl Peptidlösung und in den nächsten 10 Vertiefungen jeweils 5 µl der Peptidlösung vorgelegt. Für die Untersuchungen mit dem Enzym Trypsin wurden die folgenden, in Beispiel 1 aufgeführten Peptide verwendet: Rahmenkonstrukt 1 bis 41 in Kombination mit den Sequenzmotiven d, i und n. Für die Untersuchungen mit dem Enzym Chymotrypsin wurden die folgenden, in Beispiel 1 aufgeführten Peptide verwendet: Rahmenkonstrukt 1 bis 41 in Kombination mit den Sequenzmotiven e und o und Rahmenkonstrukt 1 bis 40 in Kombination mit dem Sequenzmotiv j. Die letzte Vertiefung, die als Hintergrundkorrektur dienen sollte, blieb leer. Anschließend wurde in alle Vertiefungen simulierte Intestinalflüssigkeit (SIF) mit einem Zusatz von 0,005 % Tween 20 und 1 mM CaCl (SIFT-CaCl) gegeben. Dabei erhielt die Vertiefung mit 7,5 µl Peptidlösung 60 µl SIFT-CaCl, die Vertiefungen mit 5 µl Peptidlösung je 45 µl SIFT-CaCl. Die leere Vertiefung für die Hintergrundkorrektur erhielt 50 µl SIFT-CaCl. Anschließend wurde die enzymatische Reaktion durch die Zugabe von 7,5 µl Enzymlösung (500 µg/ml Trypsin- bzw. Chymotrypsinlösung) auf die Vertiefung mit 7,5 µl Peptidlösung und 60 µl SIFT-CaCl gestartet. Von dieser Mischung wurden sofort 25 µl in die nachfolgende Vertiefung pipettiert, gemischt, und dieser Schritt so oft wiederholt, daß eine serielle 1:3-Verdünnung der Enzymlösung über insgesamt 10 Vertiefungen entstand. In die letzte Peptid-enthaltende Vertiefung wurde keine Enzymlösung gegeben. Somit lag in jeder Vertiefung nach der Verdünnungsreihe ein Volumen von 50 µl vor. Die Polypropylenplatte wurde mit einem Noppendeckel verschlossen und 90 min bei 37° C inkubiert. Anschließend wurde die enzymatische Reaktion durch die Zugabe von 50 µl einer Proteaseinhibitorlösung (308 nM Aprotinin, 20 µM Leupeptin, 400 µM 4-(2-Aminoethyl)benzolsulfonylfluorid (AEBSF)-Hydrochlorid in L-PBS) in jede Vertiefung beendet, und die Mikrotitrationsplatte mit einem Noppendeckel verschlossen. Nach 10 min bei 4° C wurde die Mikrotitrationsplatte für 10 min auf 90° C erhitzt und vor der weiteren Verwendung für mindestens 5 min auf 0° C gekühlt.

**Beispiel 8**

**Enzymatische Umsetzung von Substraten mit einer intestinalen Enzymlösung**

Auf einer Mikrotitrationsplatte aus Polypropylen, die das gleiche Format wie die beschichtete Mikrotitrationsplatte aus Polystyrol (siehe Beispiel 4) besaß, wurden in der ersten Vertiefung einer Reihe 7,5 μl Peptidlösung und in den nächsten 10 Vertiefungen jeweils 5 μl der Peptidlösung vorgelegt. Die letzte Vertiefung, die als Hintergrundkorrektur dienen sollte, blieb leer. Anschließend wurde in alle Vertiefungen simulierte Intestinalflüssigkeit (SIF) mit einem Zusatz von 0,005 % Tween 20 und 1 mM CaCl (SIFT-CaCl) gegeben. Dabei erhielt die Vertiefung mit 7,5 μl Peptidlösung 60 μl SIFT-CaCl, die Vertiefungen mit 5 μl Peptidlösung je 45 μl SIFT-CaCl. Die leere Vertiefung für die Hintergrundkorrektur erhielt 50 μl SIFT-CaCl. Anschließend wurde die enzymatische Reaktion durch die Zugabe von 7,5 μl einer Verdünnung von einer intestinalen Enzymlösung (Gewinnung siehe Beispiel 2) auf die Vertiefung mit 7,5 μl Peptidlösung und 60 μl SIFT-CaCl gestartet. Von dieser Mischung wurden sofort 25 μl in die nachfolgende Vertiefung pipettiert, gemischt, und dieser Schritt so oft wiederholt, daß eine serielle 1:3-Verdünnung der intestinalen Enzymlösung über insgesamt 10 Vertiefungen entstand. In die letzte Peptid-enthaltende Vertiefung wurde keine Enzymlösung gegeben. Somit lag in jeder Vertiefung nach der Verdünnungsreihe ein Volumen von 50 μl vor. Die Polypropylenplatte wurde mit einem Noppendeckel verschlossen und 90 min bei 37° C inkubiert. Anschließend wurde die enzymatische Reaktion durch die Zugabe von 50 μl einer Proteaseinhibitorlösung (308 nM Aprotinin, 20 μM Leupeptin, 400 μM 4-(2-Aminoethyl)benzolsulfonylfluorid (AEBSF)-Hydrochlorid in L-PBS) in jede Vertiefung beendet, und die Mikrotitrationsplatte mit einem Noppendeckel verschlossen. Nach 10 min bei 4° C wurde die Mikrotitrationsplatte für 10 min auf 90° C erhitzt und vor der weiteren Verwendung für mindestens 5 min auf 0° C gekühlt.

**Beispiel 9**

**Nachweis der ungespaltener Peptide nach Bindung an eine feste Phase**

Mit einer Mehrkanalpipette wurden jeweils 75 μl des Reaktionsgemisches aus Beispiel 5, 6, 7 oder 8 auf die mit anti-2,4-D-Antikörper beschichtete und abgesättigte Mikrotitrationsplatte gegeben (zur Beschichtung siehe Beispiel 4). Nach einer Inkubationszeit von 2,5 h bei RT wurden die nicht gebundenen Bestandteile des Reaktionsgemisches durch Absaugen und viermaliges Waschen mit DPBST entfernt und die Vertiefungen leergesaugt. Durch Zugabe von 75 μl 1 μg/ml peroxidase-markiertem Streptavidin in DPBS mit 1% (w/v) Casein wurden die ungespaltenen Verbindungen mit Peroxidase als Reporterenzym versehen. Nach einer Stunde wurde die Lösung abgesaugt und die Mikrotitrationsplatte sechsmal mit DPBST gewaschen und leergesaugt. Das Entwickeln des Farbsignals erfolgte durch die Zugabe von jeweils 75 μl eines Tetramethylbenzidin-Farbsubstrats in jede Vertiefung der Mikrotitrationsplatte. Die Entwicklung erfolgte im Dunklen und wurde nach 30 min durch Zugabe von je 125 μl 1 M Schwefelsäure abgebrochen. Das Farbsignal wurde mit einem Mikrotitrationsplattenlesegerät (z.B. VersaMax, Molecular Devices, Ismaning) bei einer Wellenlänge von 450 nm photometrisch bestimmt.

Die photometrischen Meßwerte eines seriell verdünnten Reaktionsansatzes mit Protease und des Reaktionsansatzes ohne Enzym wurden für die nichtlinear Kurvenanpassung verwendet, indem für Experimente mit den definierten Trypsin- und Chymotrypsinlösungen und den entsprechenden Substraten die vorher beschriebene Gleichung (6):

$$OD_{[E]} = OD_{max} + (OD_0 - OD_{max}) \exp(- t\, [E]_{gesamt}\, k_{cat} / K_M)$$

zur Bestimmung der drei Parameter $OD_0$, $OD_{max}$ und katalytische Effizienz ($k_{cat}/K_M$) verwendet wurde. Die katalytische Effizienz ist ein Maß für die Wirksamkeit der Spaltung eines Substates durch ein Enzym.

GPRARLA (Nummer 42 aus Beispiel 1) wurde von Trypsin mit einer katalytischen Effizienz von $2{,}0 \cdot 10^6 \pm 0{,}9 \cdot 10^6$ $M^{-1}$ $S^{-1}$ gespalten und GVPFGP (Nummer 43 aus Beispiel 1) von Chymotrypsin mit einer katalytischen Effizienz von $2{,}3 \cdot 10^4 \pm 0{,}9 \cdot 10^4$ $M^{-1}$ $s^{-1}$ (Jeweils geometrischer Mittelwert und Standardabweichung einer 5-fach Messung). Zum Vergleich, mit FRET basierten Verfahren war die Variante GPARLAIG des Substrates von Trypsin mit einer katalytischen Effizienz von $1{,}2 \cdot 10^6$ $M^{-1}$ $s^{-1}$ gespalten worden (Grahn et al., 1998).

Die Inhibitionskonstante von Aprotinin kann durch den Vergleich der katalytischen Effizienz ($k_{cat}/K_M$) in Gegenwart und in Abwesenheit von Aprotinin berechnet werden. $k_{cat}/K_M$-Werte werden nach Gleichung (6) berechnet und ins Verhältnis gesetzt (Gleichung (17)):

$$(k_{cat}/K_M)_0 / (k_{cat}/K_M)_i = 1 + [I]_{gesamt} / K_i$$

Der enzymatische Abbau des Substrates GPARLA (Nummer 42 aus Beispiel 1) durch Trypsin wurde durch Aprotinin mit einer Inhibitionskonstanten von $1{,}0*10^{-9} \pm 9{,}1*10^{-10}$ M inhibiert und der Abbau von GVPFGP (Nummer 43 aus Beispiel 1) durch Chymotrypsin durch Aprotinin mit einer Inhibitionskonstanten von $4{,}8*10^{-7} \pm 8{,}1*10^{-8}$ M (Jeweils geometrischer Mittelwert und Standardabweichung einer 4-fach Messung).

Die Analyse des Einflusses von hydrophilen und negativ geladenen Bausteinen in den flankierenden Bereichen auf die enzymatische Hydrolyse ergab, daß die Anwesenheit negativer Ladungen unmittelbar anschließend an die Substratsequenz in den meisten Fällen zu einer Verschlechterung der katalytisches Effizienz im Vergleich zu Substraten ohne benachbarte negative Ladungen führte. Dieser Störeffekt der negativen Ladungen ließ sich durch Einbau eines hydrophilen PEG-Abstandhalters zwischen Substratsequenz und negativer Ladung in fast allen Fällen wieder ausschalten. Dies ist in Figur 4 dargestellt.

**Beispiel 10**

**Bestimmung der Halbwertszeiten von Peptidepitopen aus einem Modellantigen in Intestinalflüssigkeit**

Zur Verifizierung der Anwendbarkeit des Verfahrens auf eine Vielzahl unterschiedlichster Peptide wurden die Halbwertszeiten ($t_{1/2}$) von 186 16mer und von 188 10mer Peptiden, welche jeweils die gesamte Aminosäuresequenz des Modellantigens Ovalbumin überlappend abdecken, in einer intestinalen Enzymlösung bestimmt. Die Peptidsynthese erfolgte wie in Beispiel 1 beschrieben. Dabei wurden die Peptide aminoterminal mit der flankierenden Sequenz 2,4D-Aun-glu-glu-PEG2- und carboxyterminal mit der flankierenden Sequenz -PEG2-glu-glu-GluPEGBio (flankierende Sequenzen 41 in Beispiel 1) versehen. Die protoeolytische Spaltung der Peptide wurde wie in Beispiel 8 beschrieben in verdünnter muriner intestinaler Enzymlösung (Gewinnung siehe Beispiel 2) durchgeführt und die Restmenge an ungespaltenen Peptiden anschließend wie in Beispiel 9 beschrieben bestimmt.

Die Halbwertszeit der einzelnen Peptide in unverdünnter Intestinalflüssigkeit wurde berechnet, indem die photometrischen Meßwerte ($OD_V$) bei verschiedenen Verdünnungen der intestinalen Enzymlösung (Vf) für eine nichtlineare Kurvenanpassung an die Gleichung (12):

$$OD_V = OD_{max} + (OD_0 - OD_{max})\ 0{,}5\text{^}(5400\ sec * Vf / t_{1/2})$$

verwendet wurden. Die so ermittelten Halbwertszeiten sind in Tabelle 1 wiedergegeben. In diesem Beispiel wurde ein Bereich der Halbwertszeiten in unverdünnter Intestinalflüssigkeit von 0,00084 sec bis 40,13 sec abgedeckt.

**Tabelle 1: Halbwertszeiten von 10mer und 16mer Peptiden aus dem Modellantigen Ovalbumin in muriner Intestinalflüssigkeit.**

| 10-mer Peptide | | | 16-mer Peptide | |
|---|---|---|---|---|
| Sequenz | $t_{1/2}$ [sec] | | Sequenz | $t_{1/2}$ [sec] |
| GSIGAASMEF | 2.7013 | | GSIGAASMEFCFDVFK | 0.0097 |
| IGAASMEFCF | 0.0629 | | IGAASMEFCFDVFKEL | 0.0084 |
| AASMEFCFDV | 0.0556 | | AASMEFCFDVFKELKV | 0.0068 |
| SMEFCFDVFK | 0.0103 | | SMEFCFDVFKELKVHH | 0.0084 |
| EFCFDVFKEL | 0.0088 | | EFCFDVFKELKVHHAN | 0.0098 |
| CFDVFKELKV | 0.0122 | | CFDVFKELKVHHANEN | 0.0103 |
| DVFKELKVHH | 0.0101 | | DVFKELKVHHANENIF | 0.0201 |
| FKELKVHHAN | 0.0094 | | FKELKVHHANENIFYC | 0.0074 |
| ELKVHHANEN | 0.1969 | | ELKVHHANENIFYCPI | 0.0851 |
| KVHHANENIF | 1.2897 | | KVHHANENIFYCPIAI | 0.0892 |
| HHANENIFYC | 0.0485 | | HHANENIFYCPLAIMS | 0.0575 |
| ANENIFYCPI | 0.1557 | | ANENIFYCPIAIMSAL | 0.0425 |
| ENIFYCPIAI | 0.0890 | | ENIFYCPIAIMSALAM | 0.0327 |

(fortgesetzt)

| 10-mer Peptide | | | 16-mer Peptide | |
|---|---|---|---|---|
| Sequenz | $t_{1/2}$ [sec] | | Sequenz | $t_{1/2}$ [sec] |
| IFYCPIAIMS | 0.0469 | | IFYCPIAIMSALAMVY | 0.0167 |
| YCPIAIMSAL | 0.2911 | | YCPIAIMSALAMVYLG | 0.0125 |
| PIAIMSALAM | 0.1596 | | PIAIMSALAMVYLGAK | 0.0077 |
| AIMSALAMVY | 0.0512 | | AIMSALAMVYLGAKDS | 0.0088 |
| MSALAMVYLG | 0.0132 | | MSALAMVYLGAKDSTR | 0.0063 |
| ALAMVYLGAK | 0.0077 | | ALAMVYLGAKDSTRTQ | 0.0045 |
| AMVYLGAKDS | 0.0111 | | AMVYLGAKDSTRTQIN | 0.0037 |
| VYLGAKDSTR | 0.0135 | | VYLGAKDSTRTQINKV | 0.0040 |
| LGAKDSTRTQ | 0.0122 | | LGAKDSTRTQINKVVR | 0.0033 |
| AKDSTRTQIN | 0.0092 | | AKDSTRTQINKVVRFD | 0.0038 |
| DSTRTQINKV | 0.0210 | | DSTRTQINKVVRFDKL | 0.0044 |
| TRTQINKVVR | 0.0027 | | TRTQINKVVRFDKLPG | 0.0045 |
| TQINKVVRFD | 0.0094 | | TQFNKVVRFDKLPGFG | 0.0085 |
| INKVVRFDKL | 0.0069 | | INKVVRFDKLPGFGDS | 0.0111 |
| KVVRFDKLPG | 0.0119 | | KVVRFDKLPGFGDSIE | 0.0131 |
| VRFDKLPGFG | 0.1526 | | VRFDKLPGFGDSIEAQ | 0.1209 |
| FDKLPGFGDS | 25.2450 | | FDKLPGFGDSIEAQCG | 5.6930 |
| KLPGFGDSIE | 40.1300 | | KLPGFGDSIEAQCGTS | 6.4211 |
| PGFGDSIEAQ | 17.8110 | | PGFGDSIEAQCGTSVN | 5.0348 |
| FGDSIEAQCG | 7.1220 | | FGDSIEAQCGTSVNVH | 1.3951 |
| DSIEAQCGTS | 13.4820 | | DSIEAQCGTSVNVHSS | 0.4688 |
| IEAQCGTSVN | 6.4814 | | IEAQCGTSVNVHSSLR | 0.1930 |
| AQCGTSVNVH | 4.3435 | | AQCGTSVNVHSSLRDI | 0.0267 |
| CGTSVNVHSS | 1.1378 | | CGTSVNVHSSLRDILN | 0.0499 |
| TSVNVHSSLR | 0.1177 | | TSVNVHSSLRDILNQI | 0.1029 |
| VNVHSSLRDI | 0.0148 | | VNVHSSLRDILNQITK | 0.0614 |
| VHSSLRDILN | 0.0527 | | VHSSLRDILNQITKPN | 0.1104 |
| SSLRDILNQI | 0.1504 | | SSLRDILNQITKPNDV | 0.1963 |
| LRDILNQITK | 0.0939 | | LRDILNQITKPNDVYS | 0.0514 |
| DILNQITKPN | 4.1593 | | DILNQITKPNDVYSFS | 0.0223 |
| LNQITKPNDV | 20.7000 | | LNQITKPNDVYSFSLA | 0.0054 |
| QITKPNDVYS | 0.2963 | | QITKPNDVYSFSLASR | 0.0025 |
| TKPNDVYSFS | 0.0120 | | TKPNDVYSFSLASRLY | 0.0011 |
| PNDVYSFSLA | 0.0026 | | PNDVYSFSLASRLYAE | 0.0014 |
| DVYSFSLASR | 0.0037 | | DVYSFSLASRLYAEER | 0.0011 |
| YSFSLASRLY | 0.0019 | | YSFSLASRLYAEERYP | 0.0032 |
| FSLASRLYAE | 0.0035 | | FSLASRLYAEERYPIL | 0.0031 |

(fortgesetzt)

| 10-mer Peptide | | | 16-mer Peptide | |
|---|---|---|---|---|
| Sequenz | $t_{1/2}$ [sec] | | Sequenz | $t_{1/2}$ [sec] |
| LASRLYAEER | 0.0031 | | LASRLYAEERYPILPE | 0.0023 |
| SRLYAEERYP | 0.0052 | | SRLYAEERYPILPEYL | 0.0028 |
| LYAEERYPIL | 0.1052 | | LYAEERYPILPEYLQC | 0.0281 |
| AEERYPILPE | 11.5000 | | AEERYPILPEYLQCVK | 0.2682 |
| ERYPILPEYL | 1.466 | | ERYPILPEYLQCVKEL | 0.1018 |
| YPILPEYLQC | 0.1016 | | YPILPEYLQCVKELYR | 0.0298 |
| ILPEYLQCVK | 0.3817 | | ILPEYLQCVKELYRGG | 0.0143 |
| PEYLQCVKEL | 0.1101 | | PEYLQCVKELYRGGLE | 0.0152 |
| YLQCVKELYR | 0.0263 | | YLQCVKELYRGGLEPI | 0.0093 |
| QCVKELYRGG | 0.0128 | | QCVKELYRGGLEPINF | 0.0085 |
| VKELYRGGLE | 0.0225 | | VKELYRGGLEPINFQT | 0.0052 |
| ELYRGGLEPI | 0.0244 | | ELYRGGLEPINFQTAA | 0.0085 |
| YRGGLEPINF | 0.0238 | | YRGGLEPINFQTAADQ | 0.0110 |
| GGLEPINFQT | 0.0336 | | GGLEPINFQTAADQAR | 0.0207 |
| LEPINFQTAA | 0.0242 | | LEPINFQTAADQAREL | 0.0155 |
| PINFQTAADQ | 0.0812 | | PINFQTAADQARELIN | 0.0183 |
| NFQTAADQAR | 0.1293 | | NFQTAADQARELINSW | 0.0247 |
| QTAADQAREL | 0.0514 | | OTAADQARELINSWVE | 0.0297 |
| AADQARELIN | 0.0325 | | AADQRELINSWVESQ | 0.0322 |
| DQARELINSW | 0.0224 | | DQARELINSWVESQTN | 0.0223 |
| ARELINSWVE | 0.0156 | | ARELINSWVESQTNGI | 0.0078 |
| ELINSWVESQ | 1.2344 | | ELINSWVESQTNGIIR | 0.1455 |
| INSWVESQTN | 3.3348 | | INSWVESQTNGIIRNV | 0.0095 |
| SWVESQTNGI | 0.7564 | | SWVESQTNGIIRNVLQ | 0.0099 |
| VESQTNGIIR | 0.3309 | | VESQTNGIIRNVLQPS | 0.0085 |
| SQTNGIIRNV | 0.0066 | | SQTNGIIRNVLQPSSV | 0.0032 |
| TNGIIRNVLQ | 0.0072 | | TNGIIRNVLQPSSVDS | 0.0033 |
| GIIRNVLQPS | 0.0038 | | GIIRNVLQPSSVDSQT | 0.0050 |
| IRNVLQPSSV | 0.0049 | | IRNVLQPSSVDSQTAM | 0.0069 |
| NVLQPSSVDS | 0.3384 | | NVLQPSSVDSQTAMVL | 0.1618 |
| LQPSSVDSQT | 0.1866 | | LQPSSVDSQTAMVLVN | 0.0332 |
| PSSVDSQTAM | 4.5647 | | PSSVDSQTAMVLVNAI | 0.0371 |
| SVDSQTAMVL | 0.2013 | | SVDSQTAMVLVNAIVF | 0.0236 |
| DSQTAMVLVN | 0.0195 | | DSQTAMVLVNAIVFKG | 0.0080 |
| QTAMVLVNAI | 0.0264 | | QTAMVLVNAIVFKGLW | 0.0051 |
| AMVLVNAIVF | 0.0165 | | AMVLVNAIVFKGLWEK | 0.0061 |
| VLVNAIVFKG | 0.0087 | | VLVNAIVFKGLWEKAF | 0.0030 |

(fortgesetzt)

| 10-mer Peptide | | | 16-mer Peptide | |
|---|---|---|---|---|
| Sequenz | $t_{1/2}$ [sec] | | Sequenz | $t_{1/2}$ [sec] |
| VNAIVFKGLW | 0.0051 | | VNAIVFKGLWEKAFKD | 0.0028 |
| AIVFKGLWEK | 0.0054 | | AIVFKGLWEKAFKDED | 0.0037 |
| VFKGLWEKAF | 0.0039 | | VFKGLWEKAFKDEDTQ | 0.0057 |
| KGLWEKAFKD | 0.0053 | | KGLWEKAFKDEDTQAM | 0.0084 |
| LWEKAFKDED | 0.0131 | | LWEKAFKDEDTQAMPF | 0.0101 |
| EKAFKDEDTQ | 0.0329 | | EKAFKDEDTQAMPFRV | 0.0037 |
| AFKDEDTQAM | 7.6573 | | AFKDEDTQAMPFRVTE | 0.0047 |
| KDEDTQAMPF | 0.6468 | | KDEDTQAMPFRVTEQE | 0.0049 |
| EDTQAMPFRV | 0.0033 | | EDTQAMPFRVTEQESK | 0.0041 |
| TQAMPFRVTE | 0.0047 | | TQAMPFRVTEQESKPV | 0.0076 |
| AMPFRVTEQE | 0.0063 | | AMPFRVTEQESKPVQM | 0.0050 |
| PFRVTEQESK | 0.1310 | | PFRVTEQESKPVQMMY | 0.1097 |
| RVTEQESKPV | 2.1948 | | RVTEQESKPVQMMYQI | 0.1086 |
| TEQESKPVQM | 19.4780 | | TEQESKPVQMMYQIGL | 0.0696 |
| QESKPVQMMY | 3.8079 | | QESKPVQMMYQIGLFR | 0.0055 |
| SKPVQMMYQI | 0.1240 | | SKPVQMMYQIGLFRVA | 0.0020 |
| PVQMMYQIGL | 0.0880 | | PVQMMYQIGLFRVASM | 0.0022 |
| QMMYQIGLFR | 0.0060 | | QMMYQIGLFRVASMAS | 0.0021 |
| MYOIGLFRVA | 0.0025 | | MYQIGLFRVASMASEK | 0.0025 |
| QIGLFRVASM | 0.0015 | | QIGLFRVASMASEKMK | 0.0021 |
| GLFRVASMAS | 0.0060 | | GLFRVASMASEKMKIL | 0.0047 |
| FRVASMASEK | 0.0082 | | FRVASMASEKMKILEL | 0.0026 |
| VASMASEKMK | 0.1745 | | VASMASEKMKILELPF | 0.0075 |
| SMASEKMKIL | 0.0072 | | SMASEKMKILELPFAS | 0.0073 |
| ASEKMKILEL | 0.0103 | | ASEKMKILELPFASGT | 0.0034 |
| EKMKILELPF | 0.0135 | | EKMKILELPFASGTMS | 0.0134 |
| MKILELPFAS | 0.0183 | | MKILELPFASGTMSML | 0.0220 |
| ILELPFASGT | 0.0767 | | ILELPFASGTMSMLVL | 0.0507 |
| ELPFASGTMS | 0.0648 | | ELPFASGTMSMLVLLP | 0.0410 |
| PFASGTMSML | 3.8440 | | PFASGTMSMLVLLPDE | 0.0444 |
| ASGTMSMLVL | 0.1692 | | ASGTMSMLVLLPDEVS | 0.0325 |
| GTMSMLVLLP | 0.0949 | | GTMSMLVLLPDEVSGL | 0.0306 |
| MSMLVLLPDE | 0.0317 | | MSMLVLLPDEVSGLEQ | 0.0139 |
| MLVLLPDEVS | 0.3230 | | MLVLLPDEVSGLEQLE | 0.1049 |
| VLLPDEVSGL | 8.3587 | | VLLPDEVSGLEQLESI | 0.2344 |
| LPDEVSGLEQ | 0.3722 | | LPDEVSGLEQLESIIN | 0.1597 |
| DEVSGLEQLE | 0.1719 | | DEVSGLEQLESIINFE | 0.0265 |

(fortgesetzt)

| 10-mer Peptide | | | 16-mer Peptide | |
|---|---|---|---|---|
| Sequenz | $t_{1/2}$ [sec] | | Sequenz | $t_{1/2}$ [sec] |
| VSGLEQLESI | 1.0014 | | VSGLEQLESIINFEKL | 0.0393 |
| GLEQLESIIN | 0.5162 | | GLEQLESIINFEKLTE | 0.0228 |
| EQLESIINFE | 0.0686 | | EQLESIINFEKLTEWT | 0.0345 |
| LESIINFEKL | 0.0663 | | LESIINFEKLTEWTSS | 0.0424 |
| SIINFEKLTE | 0.0667 | | SIINFEKLTEWTSSNV | 0.0653 |
| INFEKLTEWT | 0.0541 | | INFEKLTEWTSSNVME | 0.0497 |
| FEKLTEWTSS | 0.0563 | | FEKLTEWTSSNVMEER | 0.0286 |
| KLTEWTSSNV | 0.4176 | | KLTEWTSSNVMEERKI | 0.0414 |
| TEWTSSNVME | 0.5842 | | TEWTSSNVMEERKIKV | 0.0139 |
| WTSSNVMEER | 0.9286 | | WTSSNVMEERKIKVYL | 0.0060 |
| SSNVMEERKI | 0.0399 | | SSNVMEERKIKVYLPR | 0.0021 |
| NVMEERKIKV | 0.0072 | | NVMEERKIKVYLPRMK | 0.0021 |
| MEERKIKVYL | 0.0037 | | MEERKIKVYLPRMKME | 0.0014 |
| ERKIKVYLPR | 0.0008 | | ERKIKVYLPRMKNEEK | 0.0012 |
| KIKVYLPRMK | 0.0017 | | KIKVYLPRMKMEEKYN | 0.0016 |
| KVYLPRMKME | 0.0044 | | KVYLPRMKMEEKYNLT | 0.0030 |
| YLPRMKMEEK | 0.0088 | | YLPRMKMEEKYNLTSV | 0.0054 |
| PRMKMEEKYN | 0.0056 | | PRMKMEEKYNLTSVLM | 0.0051 |
| MKMEEKYNLT | 0.0367 | | MKMEEKYNLTSVLMAM | 0.0321 |
| MEEKYNLTSV | 0.0093 | | MEEKYNLTSVLMAMGI | 0.0225 |
| EKYNLTSVLM | 0.0124 | | EKYNLTSVLMAMGITD | 0.02111 |
| YNLTSVLMAM | 0.0294 | | YNLTSVLMAMGITDVF | 0.0298 |
| LTSVLMAMGI | 0.0787 | | LTSVLMAMGITDVFSS | 0.0170 |
| SVLMAMGITD | 0.1194 | | SVLMAMGITDVFSSSA | 0.0276 |
| LMAMGITDVF | 0.8333 | | LMAMGITDVFSSSANL | 0.0241 |
| AMGITDVFSS | 0.0154 | | AMGITDVFSSSANLSG | 0.0262 |
| GITDVFSSSA | 0.0178 | | GITDVFSSSANLSGIS | 0.0240 |
| TDVFSSSANL | 0.0190 | | TDVFSSSANLSGISSA | 0.0361 |
| VFSSSANLSG | 0.1849 | | VFSSSANLSGISSAES | 0.2471 |
| SSSANLSGIS | 0.7012 | | SSSANLSGISSAESLK | 0.3682 |
| SANLSGISSA | 0.6149 | | SANLSGISSAESLKIS | 0.0601 |
| NLSGISSAES | 1.5216 | | NLSGISSAESLKISQA | 0.0601 |
| SGISSAESLK | 0.5455 | | SGISSAESLKISQAVH | 0.0526 |
| ISSAESLKIS | 0.0401 | | ISSAESLKISQAVHAA | 0.0502 |
| SAESLKISQA | 0.0451 | | SAESLKISQAVHAAHA | 0.0475 |
| ESLKISQAVH | 0.0465 | | ESLKISQAVHAAHAEI | 0.0463 |
| LKISQAVHAA | 0.0192 | | LKISQAVHAAHAEINE | 0.0154 |

(fortgesetzt)

| 10-mer Peptide | | | 16-mer Peptide | |
|---|---|---|---|---|
| Sequenz | $t_{1/2}$ [sec] | | Sequenz | $t_{1/2}$ [sec] |
| ISQAVHAAHA | 0.1691 | | ISQAVHAAHAEINEAG | 0.1789 |
| QAVHAAHAEI | 0.1777 | | QAVHAAHAEINEAGRE | 0.0901 |
| VHAAHAEINE | 0.1356 | | VHAAHAEINEAGREVV | 0.0783 |
| AAHAEINEAG | 0.7772 | | AAHAEINEAGREVVGS | 0.1269 |
| HAEINEAGRE | 0.1388 | | HAEINEAGREVVGSAE | 0.1531 |
| EINEAGREVV | 0.1025 | | EINEAGREVVGSAEAG | 0.1950 |
| NEAGREVVGS | 0.1284 | | NEAGREVVGSAEAGVD | 0.1676 |
| AGREVVGSAE | 0.0967 | | AGREVVGSAEAGVDAA | 0.1212 |
| REVVGSAEAG | 1.7169 | | REVVGSAEAGVDAASV | 0.0694 |
| VVGSAEAGVD | 4.0618 | | VVGSAEAGVDAASVSE | 0.0475 |
| GSAEAGVDAA | 3.1271 | | GSAEAGVDAASVSEEF | 0.0261 |
| AEAGVDAASV | 0.0711 | | AEAGVDAASVSEEFRA | 0.0214 |
| AGVDAASVSE | 0.0548 | | AGVDAASVSEEFRADH | 0.0322 |
| VDAASVSEEF | 0.0426 | | VDAASVSEEFRADHPF | 0.0389 |
| AASVSEEFRA | 0.0246 | | AASVSEEFRADHPFLF | 0.0233 |
| SVSEEFRADH | 0.0788 | | SVSEEFRADHPFLFCI | 0.0029 |
| SEEFRADHPF | 0.0759 | | SEEFRADHPFLFCIKH | 0.0038 |
| EFRADHPFLF | 0.0569 | | EFRADHPFLFCIKHIA | 0.0031 |
| RADHPFLFCI | 0.0056 | | RADHPFLFCIKHIATN | 0.0038 |
| DHPFLFCIKH | 0.0042 | | DHPFLFCIKHIATNAV | 0.0046 |
| PFLFCIKHIA | 0.0046 | | PFLFCIKHIATNAVLF | 0.0055 |
| LFCIKHIATN | 0.0185 | | LFCIKHIATNAVLFFG | 0.0038 |
| CIKHIATNAV | 0.0929 | | CIKHIATNAVLFFGRC | 0.0025 |
| KHIATNAVLF | 0.0719 | | KHIATNAVLFFGRCVS | 0.0008 |
| IATNAVLFFG | 0.0048 | | HIATNAVLFFGRCVSP | 0.0009 |
| TNAVLFFGRC | 0.0015 | | | |
| AVLFFGRCVS | 0.0012 | | | |
| VLFFGRCVSP | 0.0014 | | | |

Das Modellantigen Ovalbumin wurde in Form von 188 überlappenden 10mer Peptiden bzw. 186 überlappenden 16mer Peptiden (jeweils 2 Aminosäuren Vorschub) auf Zellulosemembran synthetisiert und während der Synthese aminoterminal mit der flankierenden Sequenz 2,4D-Aun-glu-glu-PEG2- und carboxyterminal mit der flankierenden Sequenz -PEG2-glu-glu-GluPEGBio versehen. Nach Abspaltung von der Membran wurden die Peptide für 90 min in verschiedenen Verdünnungen einer aus murinem Dünndarm isolierten Enzymlösung inkubiert. Die Peptide wurden anschließend an mit anti-2,4-D-Antikörpern beschichteten Polystyrolplatten immobilisiert und die Restmenge an nicht proteolytisch gespaltenen Peptiden wurde durch Nachweis des carboxyterminalen Biotin mit Hilfe von Peroxidase-gekoppeltem Streptavidin und einem colorogenen Substrat bestimmt. Die photometrischen Meßwerte bei verschiedenen Verdünnungen der intestinalen Enzymlösung ($OD_v$) wurden in die Gleichung $OD_v = OD_{max} + (OD_0 - OD_{max})$ O,5^(5400 sec * Vf / $t_{1/2}$) eingesetzt, wobei Vf den jeweiligen Verdünnungsfaktor der Enzymlösung darstellt, und durch nichtlineare Kurvenanpassung daraus die Halbwertszeiten $t_{1/2}$ abgeleitet. Die angegebenen Halbwertszeiten beziehen sich auf unverdünnte Intestinalflüssigkeit.

Literatur:

Bender M et al. The Determination of the Concentration of Hydrolytic Enzyme Solutions: α-Chymotrypsin, Trypsin, Papain, Elastase, Subtilisin, and Acetylcholinesterase. 1966; J. Am. Chem. Soc. 88:5890-5913.
Bieth J. Theoretical and Practical Aspects of Proteinase Inhibition Kinetics. 1995; Methods Enzymol. 248:59-84.
Bray et al. The simultaneous multiple production of solution phase peptides: assessment of the Geysen method of simultaneous peptide synthesis. 1990; Tetrahedron Letters 31(40):5811-5814.
Coombs G et al. Distinct Mechanisms Contribute to Stringent Substrate Specificity of Tissuetype Plasminogen Activator. 1996; J. Biol. Chem. 271:4461-4467.
Franek M et al. Monoclonal ELISA for 2,4-Dichlorphenoxyacetic Acid: Characterization of Antibodies and Assay Optimization. 1994; J. Agric. Food Chem. 42:1369-1374.
Frank R. Spot synthesis: an easy technique for the positionally addressable, parallel chemical synthesis on a membrane support. 1992; Tetrahedron 48:9217-9232.
George J et al. Evaluation of an Imaging Platform during the Development of a FRET Protease Assay. 2003; J. Biomol. Screen. 8:72-80.
Grahn, S et al. Design and synthesis of fluorogenic trypsin peptide substrates based on resonance energy transfer. 1998; Anal. Biochem. 265: 225-231.
Haber E et al. Application of a radioimmunoassay for angiotensin I to the physiologic measurements of plasma renin activity in normal human subjects. 1969; J. Clin. Endocrin. Metab. 29:1349-1355.
Latt S et al. Fluorescence determination of carboxypeptidase A activity based on electronic energy transfer. 1972; Anal. Biochem. 50:56-62.
Lockwood J, Randall HAT. The place of electrolyte studies in surgical patients. 1949 Bull. N.Y. Acad. Med. 24:228
Morrison J. Kinetics of the reversible inhibition of enzyme catalysed reactions of tight-binding inhibitors. 1969; Biochim. Biophys. Acta 185:269-265.
Naqvi et al. ß-Galaktosidase Enzyme Fragment Complementation as a High-Throughput Screening Protease Technology. 2004; J. Biomol. Screen. 9:398-408.
O. Gutiérrez et al. An immunoenzymatic solid-phase assay for quantitative determination of HIV-1 protease acitivity. 2002; Anal. Biochem. 307:18-24
Wang et al. A Continuous Fluorescence Assay of Renin Activity. 1993; Anal. Biochem. 210:351-359.
Yaron A et al. Intramolecularly Quenched Fluorogenic Substrates for Hydrolytic Enzymes. 1979; Anal. Biochem. 95:228-235.

**Patentansprüche**

1. Verfahren zur Untersuchung der enzymatischen Spaltbarkeit von Substraten, welches für Hochdurchsatzuntersuchungen an Peptidsubstratbibliotheken geeignet ist, **dadurch gekennzeichnet, dass** man

    a) Verbindungen bereitstellt, die

    - auf einer ersten Festphase synthetisiert werden;
    - eine Komponente 1 aufweisen, die der ersten Festphase zugewandt ist und quantifiziert werden kann;
    - einen auf enzymatische Spaltbarkeit zu analysierenden Abschnitt aufweisen, der aus der Gruppe umfassend Peptide und Polypeptide ausgewählt ist;
    - eine Komponente 2 aufweisen, die von der ersten Festphase abgewandt ist und direkt oder über einen Bindungspartner an eine zweite Festphase binden kann;
    - wobei zwischen dem zu spaltenden Abschnitt und den beiden Komponenten chemische Strukturen mit einer oder mehreren negativen Ladungen eingefügt sind, welche aus einer oder mehreren Aminosäuren bestehen, die Phosphat- oder Sulfatgruppen enthalten, oder aus der Gruppe der Aminodicarbonsäuren oder Aminopolycarbonsäuren ausgewählt sind;
    - zwischen dem zu spaltenden Abschnitt und den chemischen Strukturen mit einer oder mehreren negativen Ladungen jeweils Abstandshalter eingefügt sind, wobei Polyethylenglycolsubstrukturen verwendet werden, wobei die Polyethylenglycolstruktur aus der Gruppe bestehend aus Aminopolyethylenglycoldiglycolsäure mit zwei Ethylenglycoleinheiten (PEG-2, MW 530,6) und Aminopolyethylenglycoldiglycolsäure mit neun Ethylenglycoleinheiten (PEG-9, MW 839,0) ausgewählt ist;

b) die Verbindungen nach Abspaltung von der ersten Festphase in Lösung mit einem Enzym oder Enzymgemisch in Kontakt bringt;
c) die gespaltenen und ungespaltenen Verbindungen anschließend an eine zweite Festphase bindet, die identisch mit der ersten Festphase sein kann, wobei die Bindung über Komponente 2 erfolgt, die direkt an die zweite Festphase oder an einen auf der zweiten Festphase aufgebrachten Bindungspartner bindet;
d) die nicht-immobilisierten Bestandteile von der zweiten Festphase abtrennt;
e) die Menge der ungespaltenen Verbindungen nachweist, indem man Komponente 1 quantifiziert; und
f) die Spaltbarkeit bestimmt, indem man die Menge der ungespaltenen Verbindungen vor und nach der Spaltungsreaktion vergleicht.

**2.** Verfahren zur Bestimmung der Enzymkinetik, **dadurch gekennzeichnet, dass** man das Verfahren nach Anspruch 1 wiederholt durchführt, wobei das Inkontaktbringen in Schritt b) für verschiedene Zeitintervalle oder mit verschiedenen Enzymkonzentrationen erfolgt und man die Halbwertszeit der Substrate bestimmt.

**3.** Verfahren nach Anspruch 1 oder 2, **dadurch gekennzeichnet, dass** man in Schritt d) die nicht-immobilisierten Bestandteile durch einen Waschschritt von der zweiten Festphase entfernt.

**4.** Verfahren nach einem der Ansprüche 1 bis 3, **dadurch gekennzeichnet, dass** der zu analysierende polymere Abschnitt ein Peptid ist.

**5.** Verfahren nach einem der Ansprüche 1 bis 4, **dadurch gekennzeichnet, dass** Komponente 1 und 2 verschieden sind und jeweils aus der Gruppe umfassend Liganden, Haptene und Biotin ausgewählt sind.

**6.** Verfahren nach Anspruch 5, **dadurch gekennzeichnet, dass** das Hapten 2,4-Dichlorphenoxyessigsäure (2,4-D) ist.

**7.** Verfahren nach einem der Ansprüche 1 bis 6, **dadurch gekennzeichnet, dass** die erste und die zweite Festphase aus einem Material bestehen, das aus der Gruppe umfassend Silikate, Keramik, Glas, Metalle, organische Substanzen, ausgewählt wird.

**8.** Verfahren nach einem der Ansprüche 1 bis 7, **dadurch gekennzeichnet, dass** man Substrate mit verschiedenen peptidischen Abschnitten parallel aus Aminosäuren herstellt.

**9.** Verfahren nach einem der Ansprüche 1 bis 8, **dadurch gekennzeichnet, dass** das Enzym oder Enzymgemisch aus der Gruppe der Proteasen ausgewählt wird.

**10.** Verfahren nach einem der Ansprüche 1 bis 9, **dadurch gekennzeichnet, dass** in Schritt b) zusätzlich ein Inhibitor zugegeben wird, dessen Einfluß auf die Spaltungsreaktion analysiert werden soll.

**11.** Verbindung der allgemeinen Struktur

(2)-(3)-(4)-(5)-(6)-(5)-(4)-(7)-(8),

die eine die Bausteine (2) und (3) und die Bausteine (4) und (5) umfassende Komponente A aufweist, die über Baustein (2) an eine erste Festphase gebunden und über Baustein (3) detektiert und/oder quantifiziert werden kann und gegebenenfalls an eine zweite Festphase gebunden werden kann, wobei das von der Festphase wegweisende Ende der Komponente A über Baustein (5) mit einem auf enzymatische Spaltbarkeit zu analysierenden Abschnitt (6) verknüpft ist, der aus der Gruppe umfassend Peptide und Polypeptide ausgewählt ist, und der an seinem der Komponente A gegenüberliegenden Ende mit einer Komponente B verknüpft ist, die den Baustein (8) und die Bausteine (4), (5) und optional (7) umfasst, wobei die Komponente B über Baustein (5) mit dem auf enzymatische Spaltbarkeit zu analysierenden Abschnitt (6) verknüpft ist und über Baustein (8) detektiert und/oder quantifiziert und an eine zweite Festphase gebunden werden kann, wobei
der Baustein (2) ein Syntheseanker ist,
der Baustein (3) eine detektierbare Gruppe ist,
der Baustein (4) eine chemische Struktur mit einer oder mehreren negativen Ladungen ist, welche aus einer oder mehreren Aminosäuren besteht, die Phosphat- oder Sulfatgruppen enthalten, oder aus der Gruppe der Aminodicarbonsäuren oder Aminopolycarbonsäuren ausgewählt sind,
der Baustein (5) ein Abstandhalter ist, der jeweils eine Polyethylenglycolstruktur mit einer Molmasse von 100 bis 5000 g/mol umfasst, wobei die Polyethylenglycolstruktur aus der Gruppe bestehend aus Aminopolyethylenglycoldiglycolsäure mit zwei Ethylenglycoleinheiten (PEG-2, MW 530,6) und Aminopolyethylenglycoldiglycolsäure mit neun Ethylenglycoleinheiten (PEG-9, MW 839,0) ausgewählt ist,
der Baustein (7) ein Abstandhalter ist,
der Baustein (8) eine detektierbare Gruppe ist,
wobei die Bausteine (4) und (5) der Komponenten A und B untereinander jeweils gleich oder verschieden sein können.

**12.** Verbindung nach Anspruch 11, **dadurch gekennzeichnet, dass** der Baustein (2) ein spaltbarer Anker ist, durch dessen selektive Spaltung die Verbindung von der Festphase gelöst werden kann.

**13.** Verbindung nach Anspruch 11 und 12, **dadurch gekennzeichnet, dass** der Baustein (2) aus epsilon-Lysyl-Prolin (Lys-Pro), p-[Amino-(2,4-dimethoxybenzyl)]-phenoxyacetyl (Rink-Linker), p-Benzyloxybenzylalkohol (Wang-Linker) oder 4-Hydroxymethylphenoxyacetyl (HMP) ausgewählt ist.

**14.** Verbindung nach Anspruch 13, **dadurch gekennzeichnet, dass** der Baustein (2) epsilon-Lysyl-Prolin (Lys-Pro) ist, wobei die Verbindung über den Prolin-Rest an die Festphase gebunden werden kann.

**15.** Verbindung nach den Ansprüchen 11 bis 14 **dadurch gekennzeichnet, dass** der Baustein (3) aus der Gruppe der biotinylierten Aminosäuren ausgewählt ist.

**16.** Verbindung nach Anspruch 15, wobei es sich bei der biotinylierten Aminosäure um Biocytin oder N-gamma-(N-biotinyl-3-(2-(2-(3-aminopropyloxy)-ethoxy)-ethoxy)-propyl)-L-Glutamat handelt.

**17.** Verbindung nach den Ansprüchen 11 bis 16, **dadurch gekennzeichnet, dass** der Baustein (4) aus einer oder mehreren Aminosäuren besteht, die aus der Gruppe der Aminodicarbonsäuren oder Aminopolycarbonsäuren ausgewählt sind.

**18.** Verbindung nach Anspruch 17, **dadurch gekennzeichnet, dass** die Aminosäuren des Bausteins (4) aus der Gruppe bestehend aus Glutamat und Carboxyglutamat ausgewählt sind.

**19.** Verbindung nach Anspruch 11 bis 17, **dadurch gekennzeichnet, dass** der Baustein (7) aus der Gruppe der aliphatischen Aminocarbonsäuren ausgewählt ist.

**20.** Verbindung nach Anspruch 11, **dadurch gekennzeichnet, dass** der Baustein (7) Aminoundecansäure oder Aminohexansäure ist.

**21.** Verbindung nach den Ansprüchen 11 bis 20, **dadurch gekennzeichnet, dass** der Baustein (8) aus der Gruppe bestehend aus 2,4-Dichlorphenoxyacetyl- und Dinitrophenylverbindungen ausgewählt ist.

**22.** Verbindung nach den Ansprüchen 11 bis 21, **dadurch gekennzeichnet, dass** die Bausteine (7) und (8) in einem Baustein zusammengefasst sind, der ein 2,4-Dichlophenoxyessigsäurederivat der allgemeinen Formel (I),

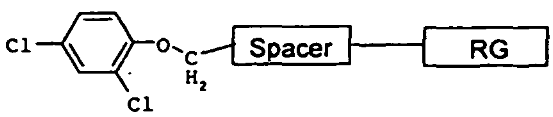

(I)

Umfasst, die in Wasser stabil und löslich ist, wobei der Spacer 1 bis 25 gleiche oder verschiedene geschützte oder ungeschützte Aminosäuren, Nukleotide, Saccharide oder aus der folgenden Gruppe ausgewählte Reste umfasst:

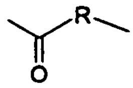 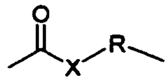 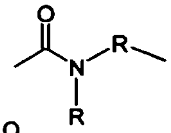

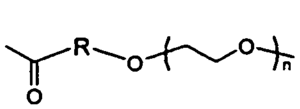 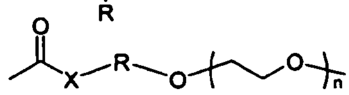 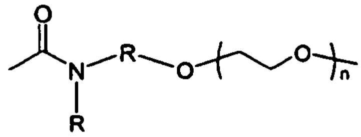

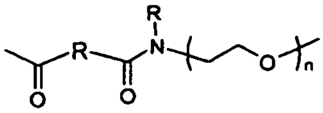 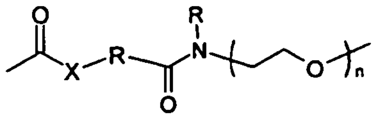 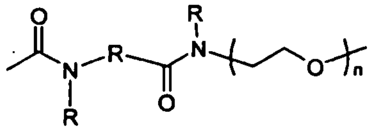

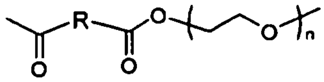 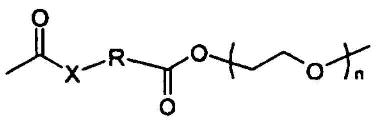 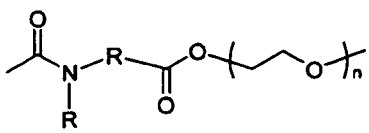

wobei X -O- oder -S- ist und n eine ganze Zahl im Bereich zwischen 1 und 10 ist,
wobei RG aus der folgenden Gruppe ausgewählt ist

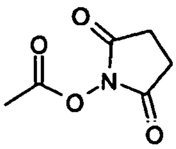 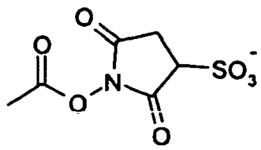 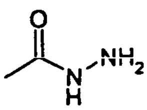 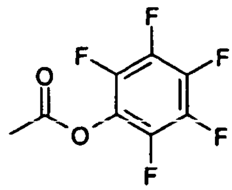 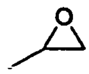 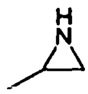

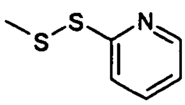 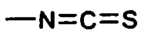 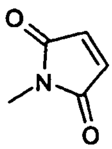 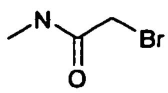 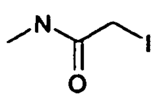 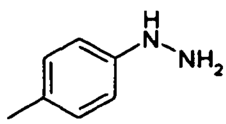

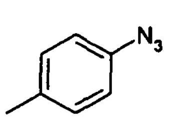 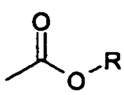 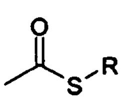 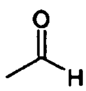 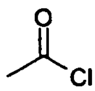 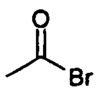 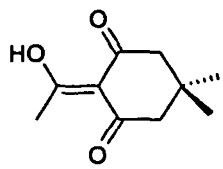

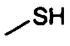 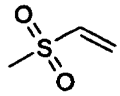 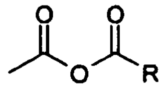 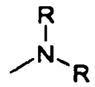 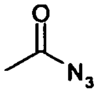 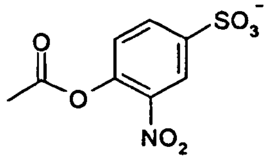

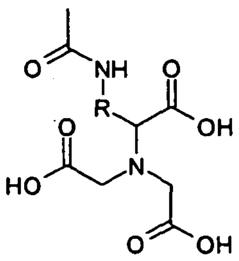 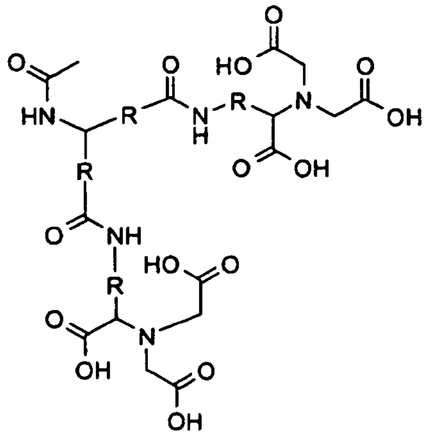

wobei R gleiche oder verschiedene Reste aus der folgenden Gruppe sind:
Wasserstoff, linearer, verzweigter oder cyclischer Alkyl- oder Alkoxyrest mit 1 bis 15 Kohlenstoffatomen, linearer oder verzweigter Alkenylrest mit 2 bis 15 Kohlenstoffatomen, geschütztes oder ungeschütztes Amin ausgenommen die folgenden Verbindungen (n = 1 - 10):

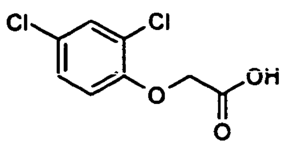 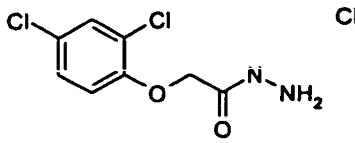 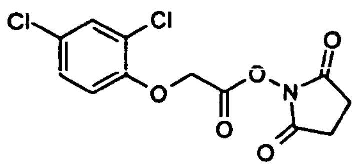

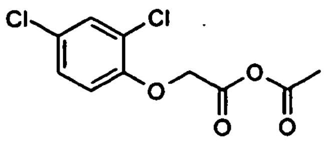 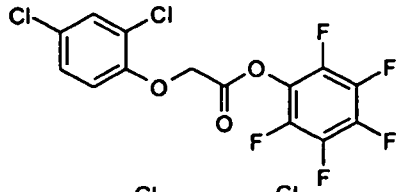 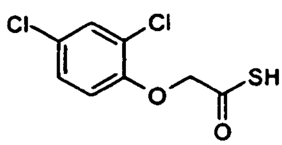

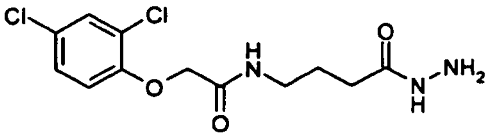 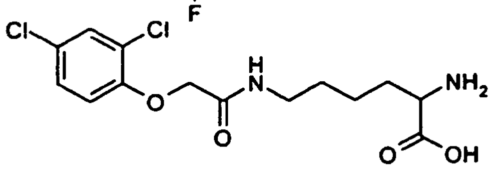

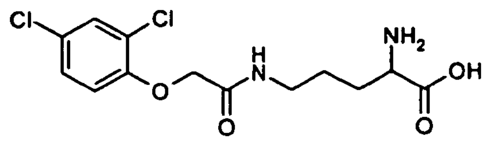 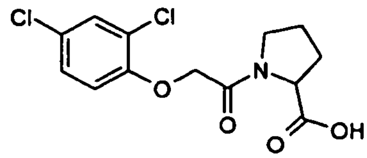

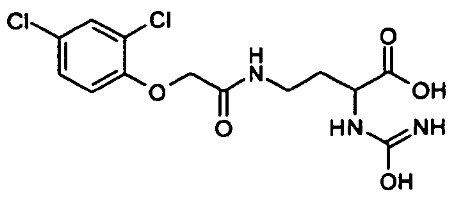 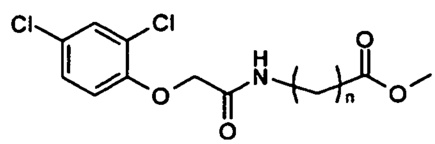

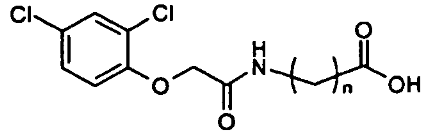 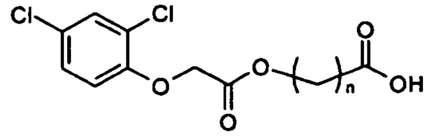

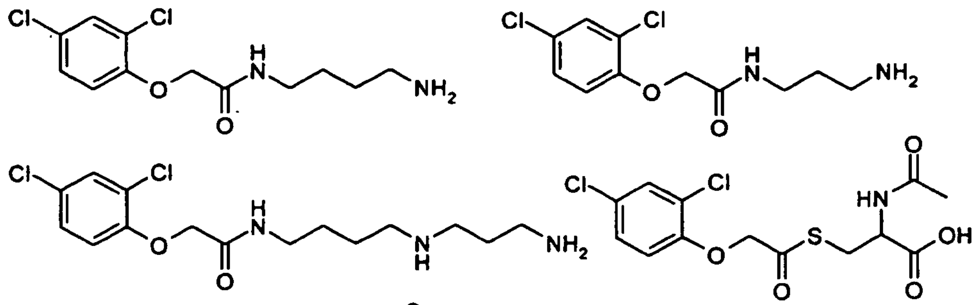

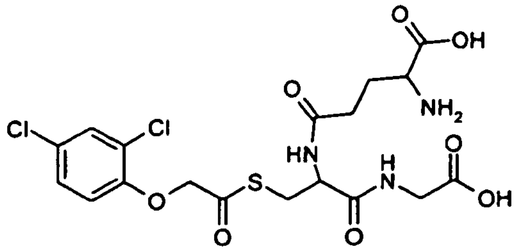

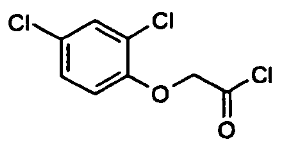

**23.** Verbindung nach den Ansprüchen 11 bis 22, **dadurch gekennzeichnet, dass** die Bausteine (4) und (5) der Komponente A aus der Gruppe bestehend aus PEG-2/D-Glutamat, PEG-2/Carboxyglutamat, PEG-9/D-Glutamat und PEG-9/Carboxyglutamat ausgewählt sind und unabhängig davon die Bausteine (4) und (5) der Komponente B aus der Gruppe bestehend aus PEG-2/D-Glutamat, PEG-2/Carboxyglutamat, PEG-9/D-Glutamat und PEG-9/Carboxyglutamat ausgewählt sind.

**24.** Kit zur Durchführung der Verfahren nach den Ansprüchen 1 bis 10, **dadurch gekennzeichnet, dass** es die Komponenten A und B der Verbindungen nach den Ansprüchen 11 bis 23, Festphasen, Puffer und Lösungen sowie optional verschiedene Enzyme oder auch Inhibitorsubstanzen enthält.

**25.** Kit zur Durchführung der Verfahren nach den Ansprüchen 1 bis 10, **dadurch gekennzeichnet, dass** es die Verbindungen nach den Ansprüchen 11 bis 23, Festphasen, Puffer und Lösungen sowie optional verschiedene Enzyme oder auch Inhibitorsubstanzen enthält.

## Claims

**1.** Method for investigating the enzymatic cleavability of substrates, said method being suitable for high-throughput screening of peptide substrate libraries, comprising:

a) providing compounds which

- are synthesized on a first solid phase;
- have a component 1 which faces the first solid phase and can be quantified;
- have a section selected from the group comprising peptides and polypeptides which is to be analyzed for enzymatic cleavability;
- have a component 2 which faces away from the first solid phase and can bind directly or via a binding partner to a second solid phase;
- wherein chemical structures having one or more negative charges and consisting of one or more amino acids that comprise phosphate or sulphate groups or are selected from the group of amino dicarboxylic acids or amino polycarboxylic acids, are inserted between the section to be cleaved and the two components;
- spacer are inserted between the section to be cleaved and the chemical structures having one or more negative charges, wherein polyethylene glycol substructures are used, and wherein the polyethylene glycol structure is selected from the group consisting of amino polyethylene glycol diglycolic acid with two ethylene glycol units (PEG-2, MW 530.6) and amino polyethylene glycol diglycolic acid with nine ethylene glycol units (PEG-9, MW 839.0);

b) bringing the compounds into contact with an enzyme or enzyme mixture in solution after cleaving from the first solid phase;

c) subsequently binding the cleaved and uncleaved compounds to a second solid phase which may be identical to the first solid phase, wherein the binding takes place via component 2 which binds directly to the second solid phase or to a binding partner provided on the second solid phase;
d) separating the non-immobilized constituents from the second solid phase;
e) detecting the amount of uncleaved compounds by quantifying component 1; and
f) determining the cleavability by comparing the amount of uncleaved compounds before and after the cleavage reaction.

**2.** Method for determining enzyme kinetics, **characterized in that** the method of claim 1 is carried out repeatedly, wherein the contacting in step b) takes place for different time intervals or with different enzyme concentrations, and the half-life of the substrates is determined.

**3.** Method of any of claims 1 or 2, **characterized in that** in step d) the non-immobilized constituents are removed from the second solid phase by a washing step.

**4.** Method of any of claims 1 to 3, **characterized in that** the polymeric section to be analyzed is a peptide.

**5.** Method of any of claims 1 to 4, **characterized in that** component 1 and 2 are different and are each selected from the group comprising ligands, haptens and biotin.

**6.** Method of claim 5, **characterized in that** the hapten is 2,4-dichlorophenoxyacetic acid (2,4-D).

**7.** Method of any of claims 1 to 6, **characterized in that** the first and the second solid phase consist of a material which is selected from the group comprising silicates, ceramic, glass, metals, organic substances.

**8.** Method of any of claims 1 to 7, **characterized in that** substrates with different peptide sections are prepared in parallel from amino acids.

**9.** Method of any of claims 1 to 8, **characterized in that** the enzyme or enzyme mixture is selected from the group of proteases.

**10.** Method of any of claims 1 to 9, **characterized in that** an inhibitor is additionally added in step b) whose influence on the cleavage reaction is to be analyzed.

**11.** Compound of the general structure

(2)-(3)-(4)-(5)-(6)-(5)-(4)-(7)-(8),

which has a component A comprising units (2) and (3) and units (4) and (5), and which can be bound to a first solid phase via unit (2) and which can be detected and/or quantified via unit (3), and which can optionally be bound to a second solid phase, wherein the end of component A that points away from the solid phase is linked via unit (5) to a section (6) which is to be analyzed for enzymatic cleavability and is selected from the group comprising peptides and polypeptides, and which is linked, at its end opposite to component A, to a component B, said component B comprising unit (8) and units (4), (5) and optionally (7), wherein component B is linked via unit (5) to the section (6) which is to be analyzed for enzymatic cleavability and can be detected and/or quantified via unit (8) and can be bound to a second solid phase, wherein
unit (2) is a synthesis anchor,
unit (3) is a detectable group,
unit (4) is a chemical structure with one or more negative charges, which consists of one or more amino acids which comprise phosphate or sulphate groups, or are selected from the group of amino dicarboxylic acids or amino polycarboxylic acids,
unit (5) is a spacer which in each case comprises a polyethylene glycol structure with a molecular mass of from 100 to 5000 g/mol, wherein the polyethylene glycol structure is selected from the group consisting of amino polyethylene glycol diglycolic acid with two ethylene glycol units (PEG-2, MW 530.6) and amino polyethylene glycol diglycolic acid with nine ethylene glycol units (PEG-9, MW 839.0),
unit (7) is a spacer,
unit (8) is a detectable group,
wherein units (4) and (5) of components A and B may each be identical to or different from one another.

**12.** Compound of claim 11, **characterized in that** unit (2) is a cleavable anchor, through selective cleavage of which the compound can be detached from the solid phase.

**13.** Compound of any of claims 11 and 12, **characterized in that** unit (2) is selected from epsilon-lysyl-proline (Lys-Pro), p-[amino(2,4-dimethoxybenzyl)]phenoxyacetyl (Rink linker), p-benzyloxybenzyl alcohol (Wang linker) or 4-hydroxymethylphenoxyacetyl (HMP).

**14.** Compound of claim 13, **characterized in that** unit (2) is epsilon-lysyl-proline (Lys-Pro), wherein the compound can be bound to the solid phase via the proline residue.

**15.** Compound of any of claims 11 to 14, **characterized in that** unit (3) is selected from the group of biotinylated amino acids.

**16.** Compound of claim 15, wherein the biotinylated amino acid is biocytin or N-gamma(N-biotinyl-3-(2-(2-(3-aminopropyloxy)ethoxy)ethoxy)propyl)L-glutamate.

**17.** Compound of any of claims 11 to 16, **characterized in that** unit (4) consists of one or more amino acids which are selected from the group of amino dicarboxylic acids or amino polycarboxylic acids.

**18.** Compound of claim 17, **characterized in that** the amino acids of unit (4) are selected from the group consisting of glutamate and carboxyglutamate.

**19.** Compound of any of claim 11 to 17, **characterized in that** unit (7) is selected from the group of aliphatic amino carboxylic acids.

**20.** Compound of claim 11, **characterized in that** unit (7) is aminoundecanoic acid or aminohexanoic acid.

**21.** Compound of any of claims 11 to 20, **characterized in that** unit (8) is selected from the group consisting of 2,4-dichlorophenoxyacetyl and dinitrophenyl compounds.

**22.** Compound of any of claims 11 to 21, **characterized in that** units (7) and (8) are combined in one unit, which comprises a 2,4-dichlorophenoxyacetic acid derivative of the general formula (I)

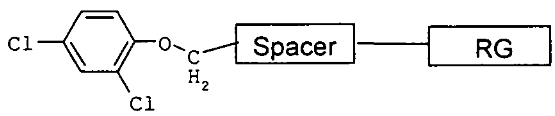

(I)

which compound is stable and soluble in water, wherein the spacer comprises 1-25 identical or different protected or unprotected amino acids, nucleotides, saccharides or residues selected from the following group:

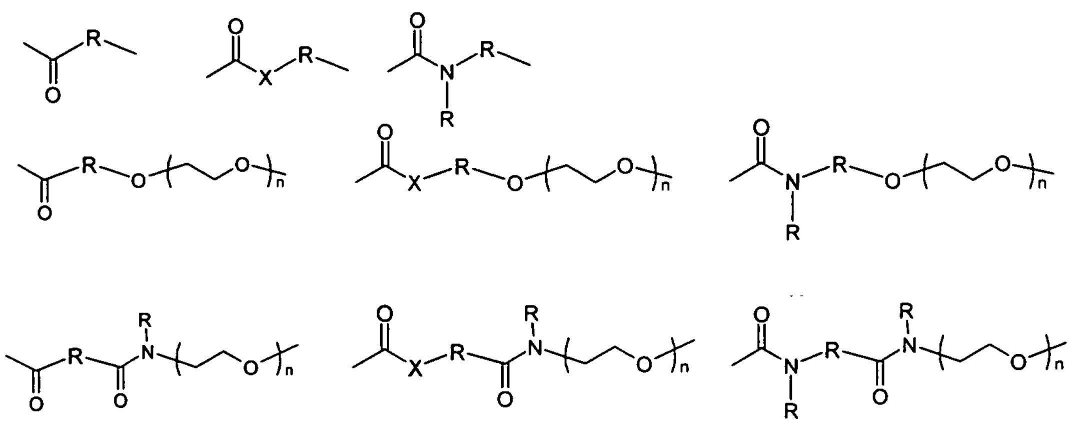

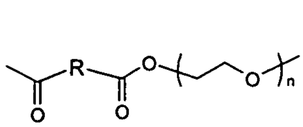 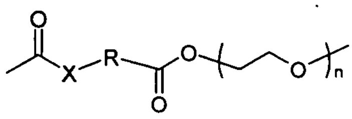 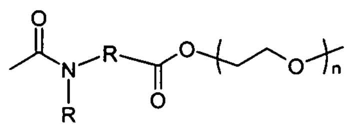

wherein X is -O- or -S- and n is an integer between 1 and 10, wherein RG is selected from the following group:

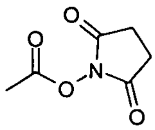 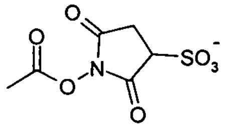 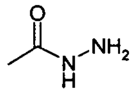 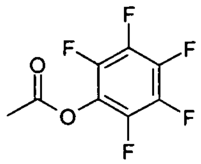 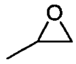 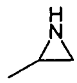

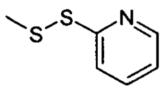 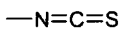 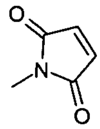 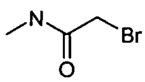 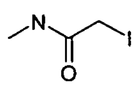 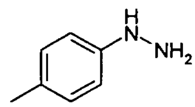

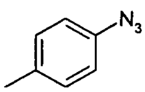 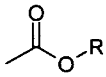 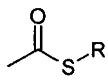 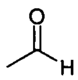 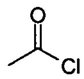 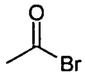 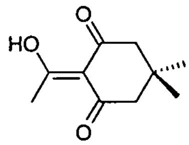

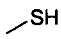 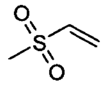 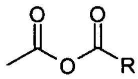 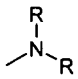 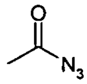 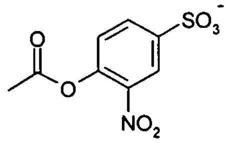

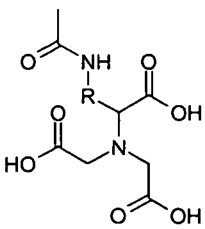 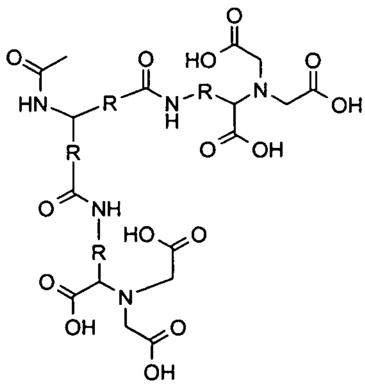

wherein R are identical or different residues from the following group:
hydrogen, linear, branched or cyclic alkyl or alkoxy residue with 1 to 15 carbon atoms, linear or branched alkylene residue with 2 to 15 carbon atoms, protected or unprotected amine
except for the following compounds (n=1-10):

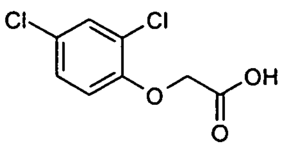
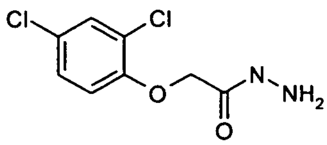
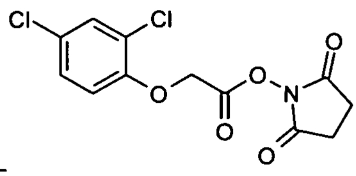
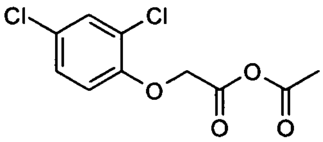
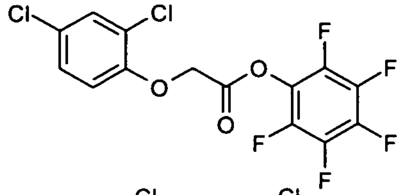
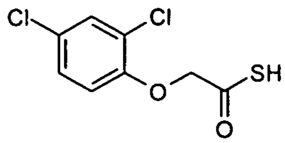
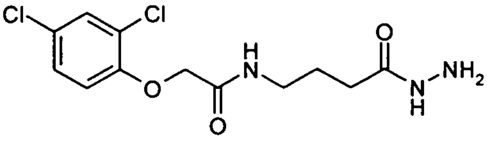
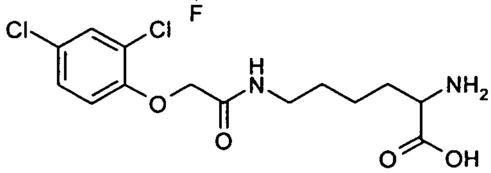
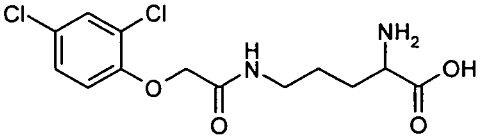
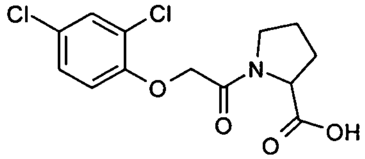
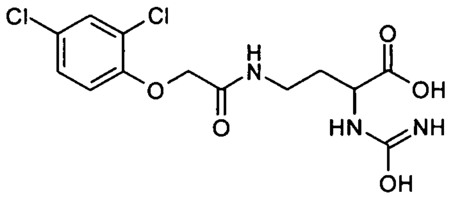
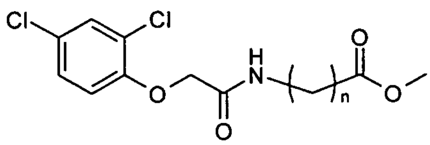
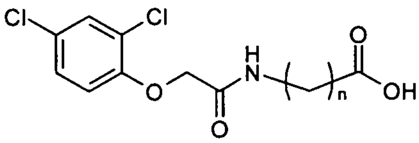
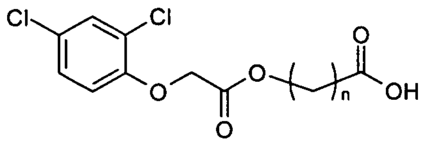
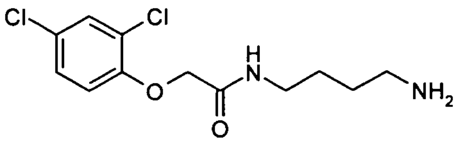
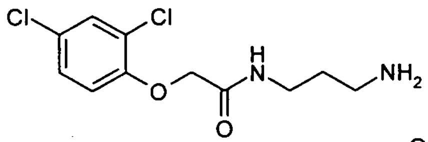
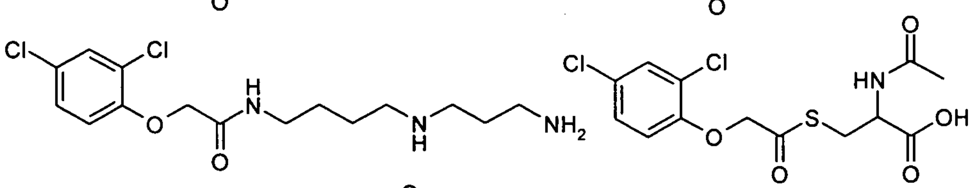
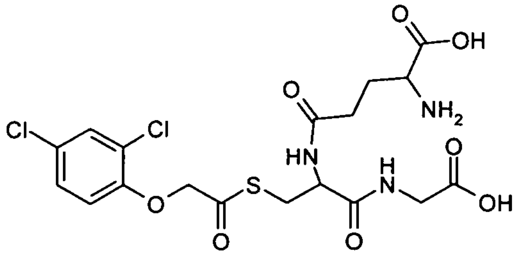
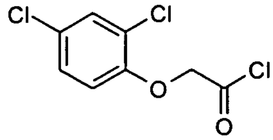

23. Compound of any of claims 11 to 22, **characterized in that** units (4) and (5) of component A are selected from the group consisting of PEG-2/D-glutamate, PEG-2/carboxyglutamate, PEG-9/D-glutamate and PEG-9/carboxyglutamate, and independently thereof units (4) and (5) of component B are selected from the group consisting of PEG-2/D-glutamate, PEG-2/carboxyglutamate, PEG-9/D-glutamate and PEG-9/carboxyglutamate.

**24.** Kit for carrying out the methods of any of claims 1 to 10, **characterized in that** it comprises components A and B of the compounds according to claims 11 to 23, solid phases, buffers and solutions and, optionally, various enzymes or inhibitor substances.

**25.** Kit for carrying out the methods of any of claims 1 to 10, **characterized in that** it comprises the compounds of any of claims 11 to 23, solid phases, buffers and solutions and optionally various enzymes or inhibitor substances.

## Revendications

**1.** Procédé d'analyse de l'aptitude au clivage enzymatique de substrats, qui est approprié pour les analyses à haut débit de bibliothèques de substrats peptidiques, **caractérisé en ce que**

a) des composés sont mis à disposition, qui

- sont synthétisés sur une première phase solide;
- comprennent un composant 1 qui est tourné vers la première phase solide et qui peut être quantifié;
- comprennent une partie à analyser en matière d'aptitude au clivage enzymatique, qui est choisie dans le groupe comprenant les peptides et les polypeptides;
- comprennent un composant 2, qui est détourné de la première phase solide et qui peut être relié directement ou par le biais d'un partenaire de liaison à une seconde phase solide;
- des structures chimiques comprenant une ou plusieurs charges négatives étant insérées entre la partie à cliver et les deux composants, qui sont constituées d'un ou de plusieurs acides aminés contenant des groupes phosphate ou sulfate ou qui sont choisies dans le groupe des acides aminodicarboxyliques ou des acides aminopolycarboxyliques;
- des espaceurs étant insérés à chaque fois entre la partie à cliver et les structures chimiques comprenant une ou plusieurs charges négatives, des sous-structures polyéthylène glycol étant utilisées, la structure polyéthylène glycol étant choisie dans le groupe constitué par l'acide aminopolyéthylène glycol diglycolique contenant deux unités éthylène glycol (PEG-2, MW 530,6) et l'acide aminopolyéthylène glycol diglycolique contenant neuf unités éthylène glycol (PEG-9, MW 839,0);

b) les composés sont mis en contact avec une enzyme ou un mélange d'enzymes en solution après le clivage de la première phase solide;
c) les composés clivés et non clivés sont ensuite reliés à une seconde phase solide, qui peut être identique à la première phase solide, la liaison ayant lieu par le biais du composant 2, qui se relie directement à la seconde phase solide ou à un partenaire de liaison appliqué sur la seconde phase solide;
d) les constituants non immobilisés sont séparés de la seconde phase solide;
e) la quantité des composés non clivés est établie par quantification du composant 1; et
f) l'aptitude au clivage est déterminée par comparaison de la quantité de composés non clivés avant et après la réaction de clivage.

**2.** Procédé de détermination de la cinétique enzymatique, **caractérisé en ce que** le procédé selon la revendication 1 est réalisé de manière répétée, la mise en contact à l'étape b) ayant lieu pour différents intervalles de temps ou avec différentes concentrations d'enzymes, et la demi-vie des substrats étant déterminée.

**3.** Procédé selon la revendication 1 ou 2, **caractérisé en ce que** les constituants non immobilisés sont éliminés de la seconde phase solide par une étape de lavage à l'étape d).

**4.** Procédé selon l'une quelconque des revendications 1 à 3, **caractérisé en ce que** la partie polymère à analyser est un peptide.

**5.** Procédé selon l'une quelconque des revendications 1 à 4, **caractérisé en ce que** le composant 1 et le composant 2 sont différents et sont chacun choisis dans le groupe comprenant les ligands, les haptènes et la biotine.

**6.** Procédé selon la revendication 5, **caractérisé en ce que** l'haptène est l'acide 2,4-dichlorophénoxyacétique (2,4-D).

7. Procédé selon l'une quelconque des revendications 1 à 6, **caractérisé en ce que** la première et la seconde phase solide sont constituées d'un matériau qui est choisi dans le groupe comprenant les silicates, la céramique, le verre, les métaux, les substances organiques.

8. Procédé selon l'une quelconque des revendications 1 à 7, **caractérisé en ce que** des substrats comprenant différentes parties peptidiques sont fabriqués en parallèle à partir d'acides aminés.

9. Procédé selon l'une quelconque des revendications 1 à 8, **caractérisé en ce que** l'enzyme ou le mélange d'enzymes est choisi dans le groupe des protéases.

10. Procédé selon l'une quelconque des revendications 1 à 9, **caractérisé en ce qu'**un inhibiteur dont l'effet sur la réaction de clivage doit être analysé est également ajouté à l'étape b).

11. Composé de structure générale

    (2)-(3)-(4)-(5)-(6)-(5)-(4)-(7)-(8)

    qui comprend un composant A comprenant les constituants (2) et (3) et les constituants (4) et (5), qui est relié à une première phase solide par le biais du constituant (2) et peut être détecté et/ou quantifié par le biais du constituant (3) et peut éventuellement être relié à une seconde phase solide, l'extrémité du composant A distale de la phase solide étant reliée par le biais du constituant (5) avec une partie (6) à analyser en matière d'aptitude au clivage enzymatique, qui est choisie dans le groupe comprenant les peptides et les polypeptides, et qui est reliée à son extrémité opposée au composant A avec un composant B, qui comprend le constituant (8) et les constituants (4) et (5) et éventuellement (7), le composant B étant relié par le biais du constituant (5) avec la partie (6) à analyser en matière d'aptitude au clivage enzymatique et pouvant être détectée et/ou quantifiée et reliée à une seconde phase solide par le biais du constituant (8),
    le constituant (2) étant une ancre de synthèse,
    le constituant (3) étant un groupe détectable,
    le constituant (4) étant une structure chimique comprenant une ou plusieurs charges négatives, qui sont constituées d'un ou de plusieurs acides aminés contenant des groupes phosphate ou sulfate ou qui sont choisies dans le groupe des acides aminodicarboxyliques ou des acides aminopolycarboxyliques,
    le constituant (5) étant un espaceur, qui comprend à chaque fois une structure polyéthylène glycol ayant une masse molaire de 100 à 5 000 g/mol, la structure polyéthylène glycol étant choisie dans le groupe constitué par l'acide aminopolyéthylène glycol diglycolique contenant deux unités éthylène glycol (PEG-2, MW 530,6) et l'acide aminopolyéthylène glycol diglycolique contenant neuf unités éthylène glycol (PEG-9, MW 839,0),
    le constituant (7) étant un espaceur,
    le constituant (8) étant un groupe détectable,
    les constituants (4) et (5) des composants A et B pouvant à chaque fois être identiques ou différents l'un de l'autre.

12. Composé selon la revendication 11, **caractérisé en ce que** le constituant (2) est une ancre clivable par le clivage sélectif de laquelle le composé peut être détaché de la phase solide.

13. Composé selon la revendication 11 ou 12, **caractérisé en ce que** le constituant (2) est choisie parmi l'epsilon-lysylproline (Lys-Pro), le p-[amino-(2,4-diméthoxybenzyl)]-phénoxyacétyle (Rink Linker), l'alcool p-benzyloxybenzylique (Wang Linker) ou le 4-hydroxyméthylphénoxyacétyle (HMP).

14. Composé selon la revendication 13, **caractérisé en ce que** le constituant (2) est l'epsilon-lysyl-proline (Lys-Pro), le composé pouvant être relié à la phase solide par le biais du radical proline.

15. Composé selon les revendications 11 à 14, **caractérisé en ce que** le constituant (3) est choisi dans le groupe des acides aminés biotinylés.

16. Composé selon la revendication 15, **caractérisé en ce que** l'acide aminé biotinylé est la biocytine ou le N-gamma-(N-biotinyl-3-(2-(2-(3-aminopropyloxy)-éthoxy)-éthoxy)-propyl)-L-glutamate.

17. Composé selon les revendications 11 à 16, **caractérisé en ce que** le constituant (4) est constitué d'un ou de plusieurs acides aminés, qui sont choisis dans le groupe des acides aminodicarboxyliques ou des acides aminopolycarboxyliques.

**18.** Procédé selon la revendication 17, **caractérisé en ce que** les acides aminés du constituant (4) sont choisis dans le groupe constitué par le glutamate et le carboxyglutamate.

**19.** Composé selon les revendications 11 à 17, **caractérisé en ce que** le constituant (7) est choisi dans le groupe des acides aminocarboxyliques aliphatiques.

**20.** Composé selon la revendication 11, **caractérisé en ce que** le constituant (7) est l'acide aminoundécanoïque ou l'acide aminohexanoïque.

**21.** Composé selon les revendications 11 à 20, **caractérisé en ce que** le constituant (8) est choisi dans le groupe constitué par les composés 2,4-dichlorophénoxyacétyliques et dinitrophényliques.

**22.** Composé selon les revendications 11 à 21, **caractérisé en ce que** les constituants (7) et (8) sont regroupés en un constituant qui comprend un dérivé d'acide 2,4-dichorophénoxyacétique de formule générale (I)

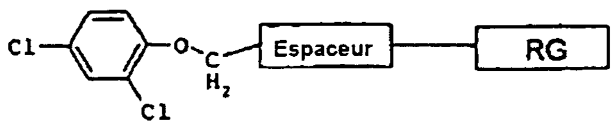

(I)

qui est stable et soluble dans l'eau, l'espaceur comprenant 1 à 25 acides aminés, nucléotides, saccharides ou radicaux choisis dans le groupe suivant, identiques ou différents, protégés ou non protégés:

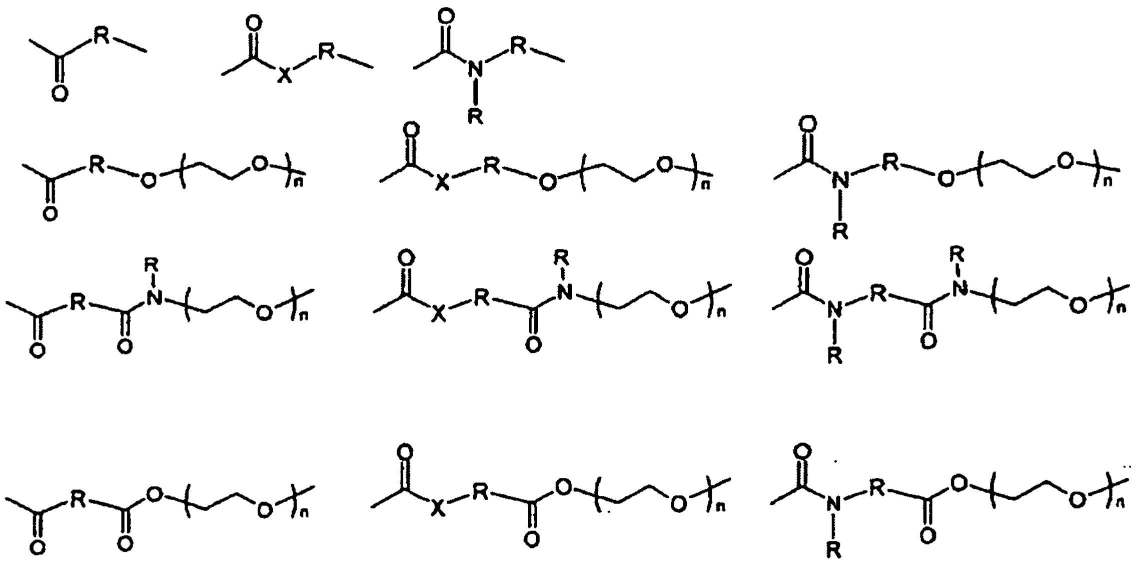

X représentant -O- ou -S- et n étant un nombre entier dans la plage comprise entre 1 et 10,
RG étant choisi dans le groupe suivant:

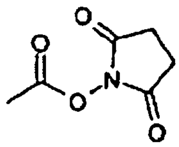 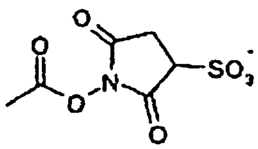 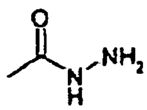 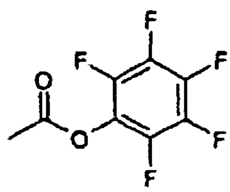 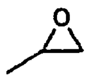 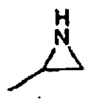

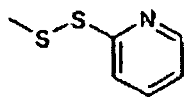 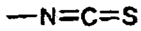 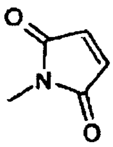 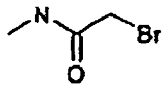 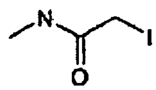 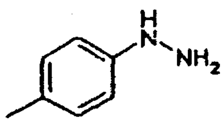

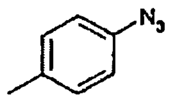 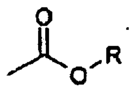 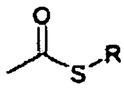 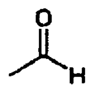 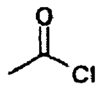 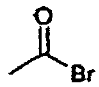 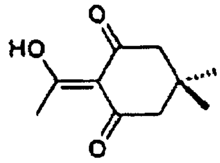

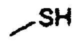 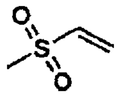 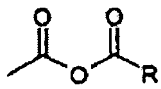 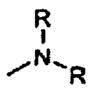 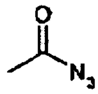 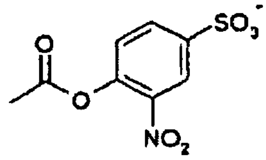

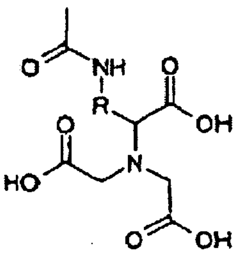 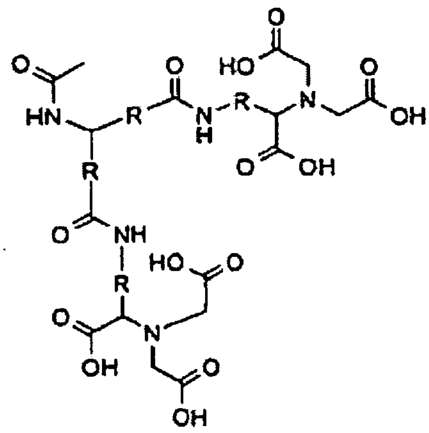

R représentant des radicaux identiques ou différents du groupe suivant:

hydrogène, radical alkyle ou alcoxy linéaire, ramifié ou cyclique de 1 à 15 atomes de carbone, radical alcényle linéaire ou ramifié de 2 à 15 atomes de carbone, amine protégée ou non protégée,
à l'exception des composés suivants (n = 1 à 10):

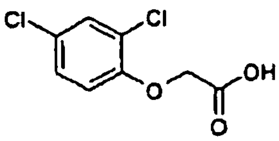
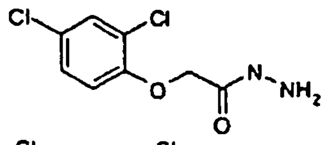
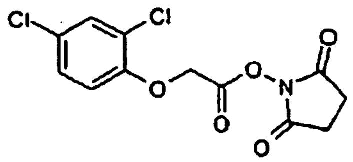
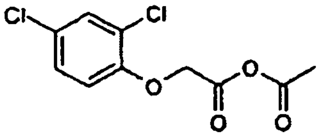
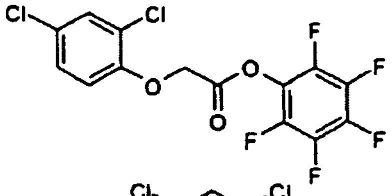
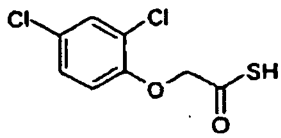
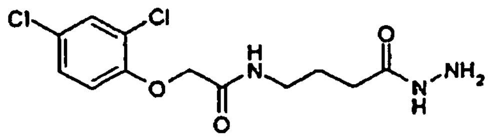
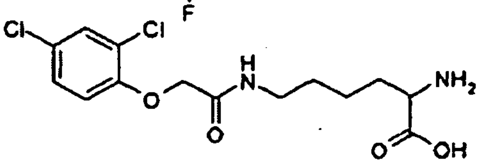
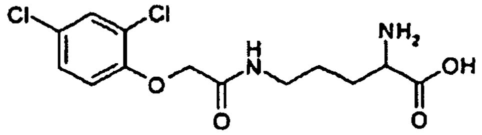
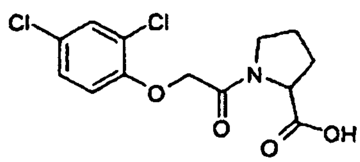
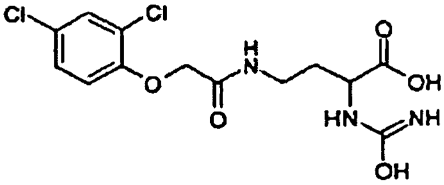
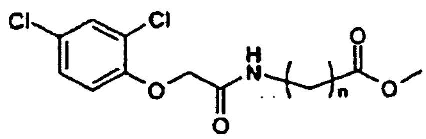
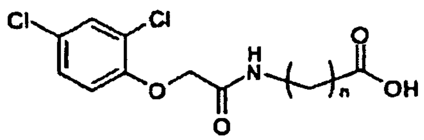
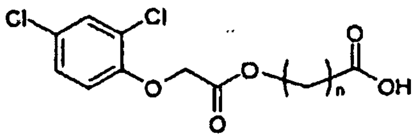
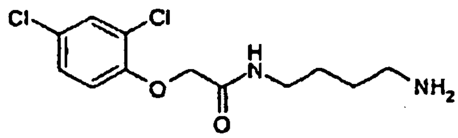
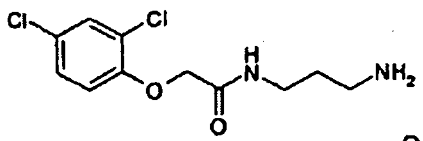
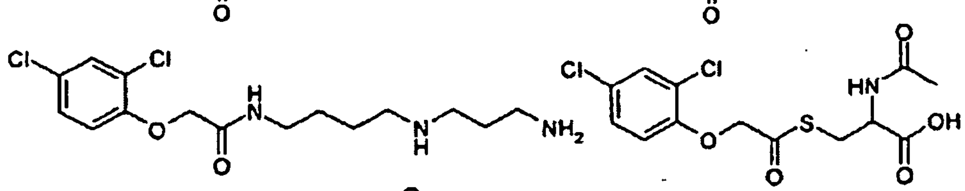
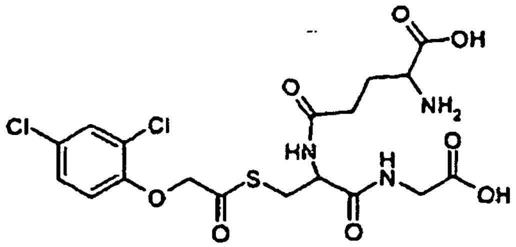
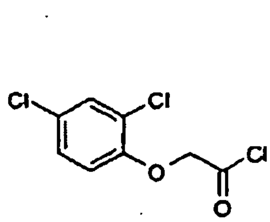

**23.** Composé selon les revendications 11 à 22, **caractérisé en ce que** les constituants (4) et (5) du composant A sont choisis dans le groupe constitué par le PEG-2/D-glutamate, le PEG-2/carboxyglutamate, le PEG-9/D-glutamate et le PEG-9/carboxyglutamate et, indépendamment, les constituants (4) et (5) du composant B sont choisis dans le groupe constitué par le PEG-2/D-glutamate, le PEG-2/carboxyglutamate, le PEG-9/D-glutamate et le PEG-9/carboxyglutamate.

**24.** Kit pour la réalisation du procédé selon les revendications 1 à 10, **caractérisé en ce qu'**il contient les composants A et B des composés selon les revendications 11 à 23, des phases solides, des tampons et des solutions, ainsi qu'éventuellement différentes enzymes ou également des substances inhibitrices.

**25.** Kit pour la réalisation du procédé selon les revendications 1 à 10, **caractérisé en ce qu'**il contient les composés selon les revendications 11 à 23, des phases solides, des tampons et des solutions, ainsi qu'éventuellement différentes enzymes ou également des substances inhibitrices.

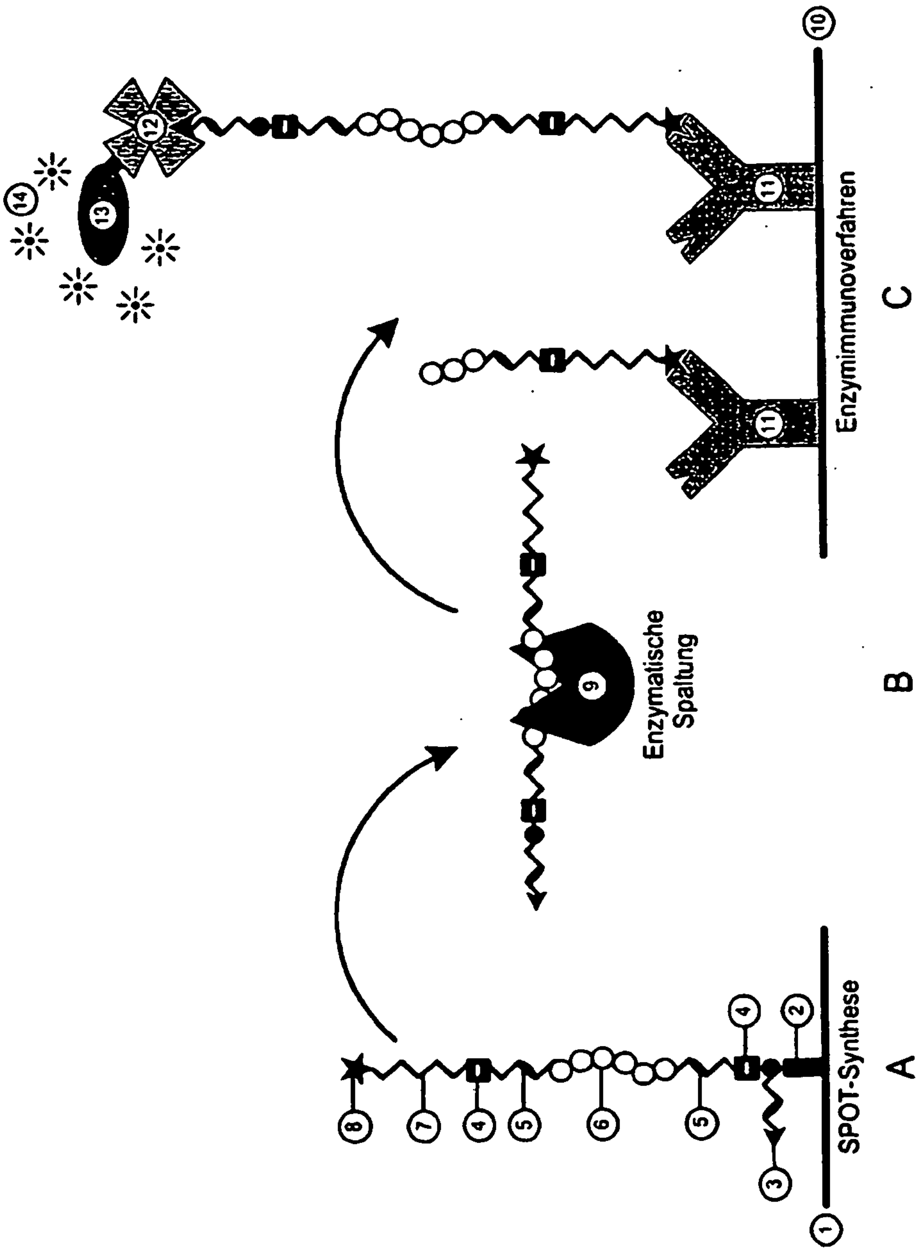
1
2
3
4
5
6
5
4
7
8
SPOT-Synthese
A
9
Enzymatische Spaltung
B
10
11
11
12
13
14
Enzymimmunoverfahren
C

Figur 1

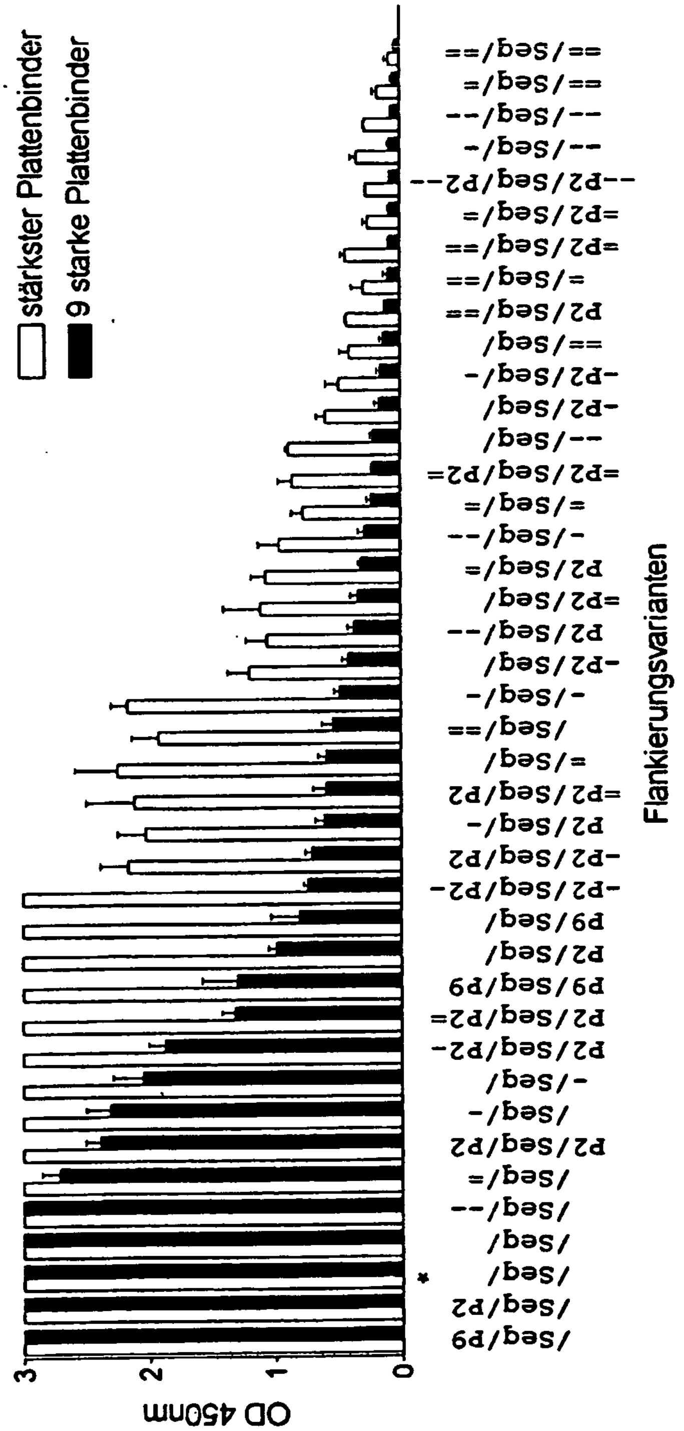
stärkster Plattenbinder
9 starke Plattenbinder
OD 450nm
0
1
2
3
Flankierungsvarianten

Figur 2

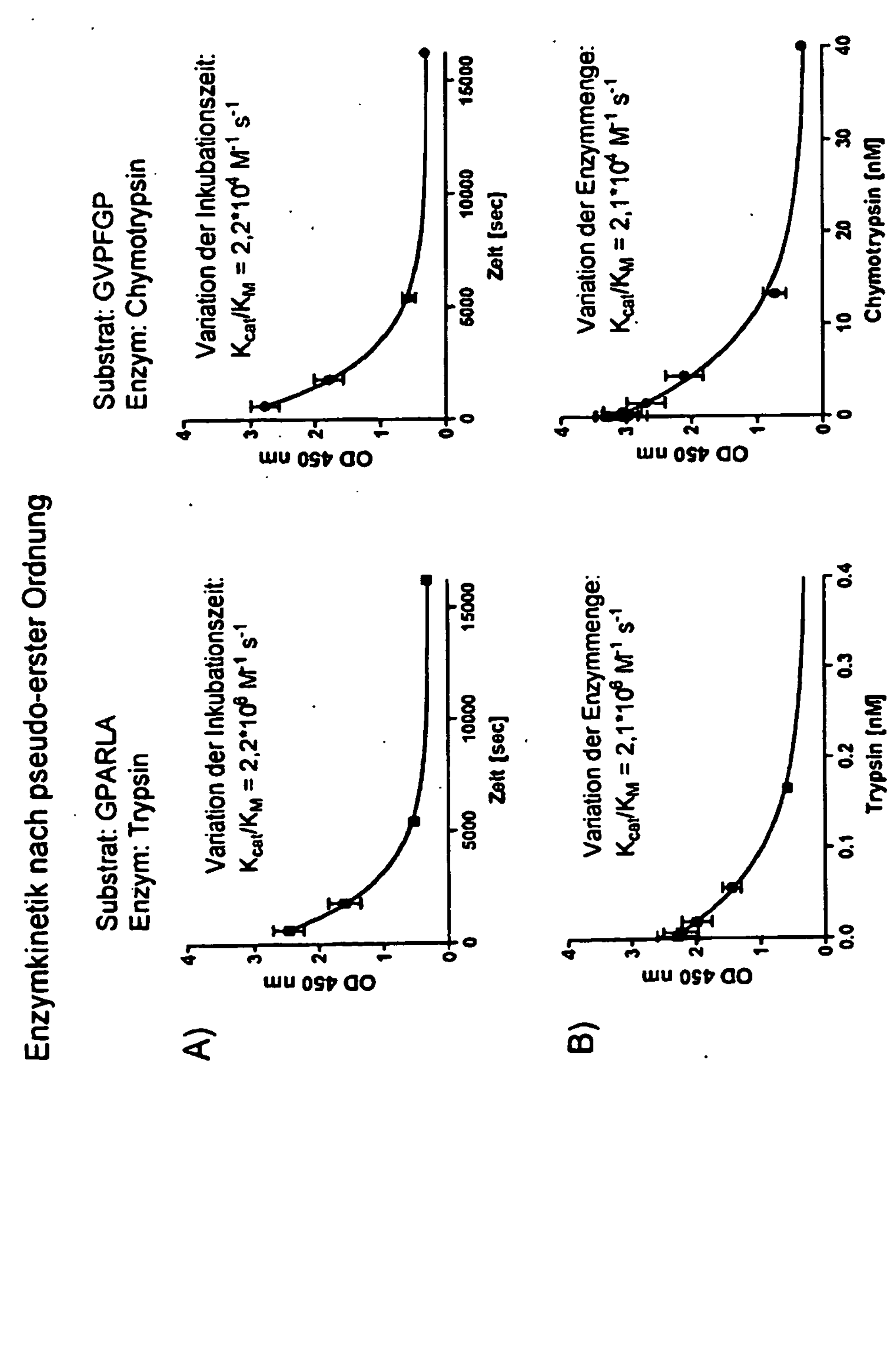
Enzymkinetik nach pseudo-erster Ordnung
A)
Substrat: GPARLA
Enzym: Trypsin
Variation der Inkubationszeit:
$K_{cat}/K_M = 2,2*10^6\ M^{-1}\ s^{-1}$
OD 450 nm
Zeit [sec]
Substrat: GVPFGP
Enzym: Chymotrypsin
Variation der Inkubationszeit:
$K_{cat}/K_M = 2,2*10^4\ M^{-1}\ s^{-1}$
OD 450 nm
Zeit [sec]
B)
Variation der Enzymmenge:
$K_{cat}/K_M = 2,1*10^6\ M^{-1}\ s^{-1}$
OD 450 nm
Trypsin [nM]
Variation der Enzymmenge:
$K_{cat}/K_M = 2,1*10^4\ M^{-1}\ s^{-1}$
OD 450 nm
Chymotrypsin [nM]

Figur 3

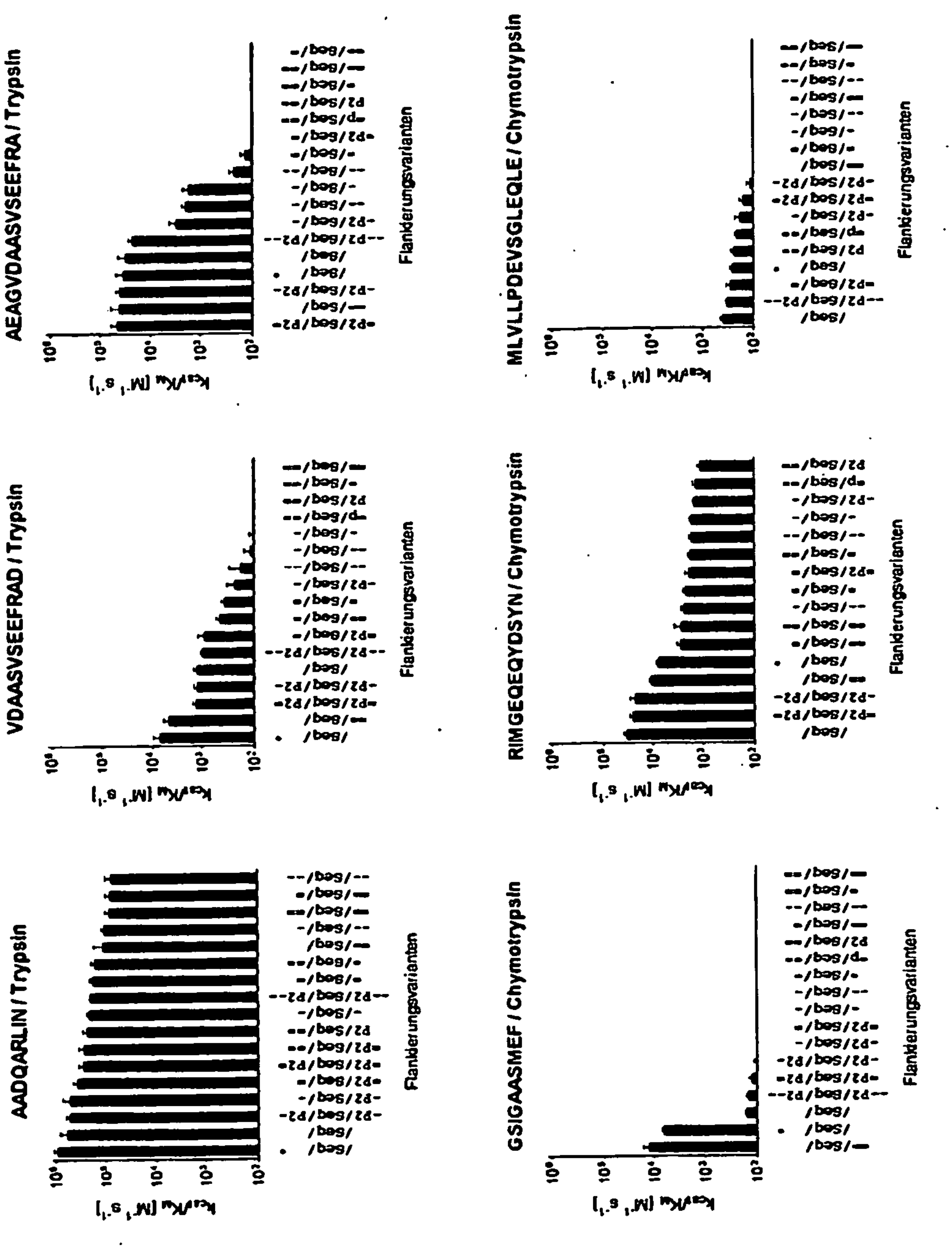
AEAGVDAASVSEEFRA / Trypsin
MLVLLPDEVSGLEQLE / Chymotrypsin
VDAASVSEEFRAD / Trypsin
RIMGEQEQYDSYN / Chymotrypsin
AADQARLIN / Trypsin
GSIGAASMEF / Chymotrypsin
Flankierungsvarianten

Figur 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10118774
- WO 2005007119 A, J. George
- DE 19840545 A1
- WO 199839471 A
- WO 200106306 A
- WO 2007051604 A
- WO 2007051603 A
- DE 102005051977
- DE 102005051976


### In der Beschreibung aufgeführte Nicht-Patentliteratur


- **MANETTA et al.** *Anal. Biochem.,* 1992, vol. 202, 10-15
- **CRAIG et al.** *Anal. Chem.,* 1998, vol. 70, 3824-3827
- **BAECHLE et al.** *J. Pept. Sci.,* 2005, vol. 11, 166-174
- **BENDER M et al.** The Determination of the Concentration of Hydrolytic Enzyme Solutions: α-Chymotrypsin, Trypsin, Papain, Elastase, Subtilisin, and Acetylcholinesterase. *J. Am. Chem. Soc.,* 1996, vol. 88, 5890-5913
- **BIETH J.** Theoretical and Practical Aspects of Proteinase Inhibition Kinetics. 1995. *Methods Enzymol.,* 1995, vol. 248, 59-84
- **BRAY et al.** The simultaneous multiple production of solution phase peptides: assessment of the Geysen method of simultaneous peptide synthesis. *Tetrahedron Letters,* 1990, vol. 31 (40), 5811-5814
- **COOMBS G et al.** Distinct Mechanisms Contribute to Stringent Substrate Specificity of Tissuetype Plasminogen Activator. *J. Biol. Chem.,* 1996, vol. 271, 4461-4467
- **FRANEK M et al.** Monoclonal ELISA for 2,4-Dichlorphenoxyacetic Acid: Characterization of Antibodies and Assay Optimization. *J. Agric. Food Chem.,* 1994, vol. 42, 1369-1374
- **FRANK R.** Spot synthesis: an easy technique for the positionally addressable, parallel chemical synthesis on a membrane support. *Tetrahedron,* 1992, vol. 48, 9217-9232
- **GEORGE J et al.** Evaluation of an Imaging Platform during the Development of a FRET Protease Assay. *J. Biomol. Screen.,* 2003, vol. 8, 72-80
- **GRAHN, S et al.** Design and synthesis of fluorogenic trypsin peptide substrates based on resonance energy transfer. *Anal. Biochem.,* 1998, vol. 265, 225-231
- **HABER E et al.** Application of a radioimmunoassay for angiotensin I to the physiologic measurements of plasma renin activity in normal human subjects. *J. Clin. Endocrin. Metab.,* 1969, vol. 29, 1349-1355
- **LATT S et al.** Fluorescence determination of carboxypeptidase A activity based on electronic energy transfer. *Anal. Biochem.,* 1972, vol. 50, 56-62
- **LOCKWOOD J ; RANDALL HAT.** The place of electrolyte studies in surgical patients. *Bull. N.Y. Acad. Med.,* 1949, vol. 24, 228
- **MORRISON J.** Kinetics of the reversible inhibition of enzyme catalysed reactions of tight-binding inhibitors. *Biochim. Biophys. Acta,* 1969, vol. 185, 269-265
- **NAQVI et al.** ß-Galaktosidase Enzyme Fragment Complementation as a High-Throughput Screening Protease Technology. *J. Biomol. Screen,* 2004, vol. 9, 398-408
- **O. GUTIÉRREZ et al.** An immunoenzymatic solid-phase assay for quantitative determination of HIV-1 protease acitivity. *Anal. Biochem.,* 2002, vol. 307, 18-24
- **WANG et al.** A Continuous Fluorescence Assay of Renin Activity. *Anal. Biochem.,* 1993, vol. 210, 351-359
- **YARON A et al.** Intramolecularly Quenched Fluorogenic Substrates for Hydrolytic Enzymes. *Anal. Biochem.,* 1979, vol. 95, 228-235